# FASCICULE DE BREVET EUROPEEN

(11) **EP 3 455 239 B1**
(45) Date de publication et mention de la délivrance du brevet: **07.04.2021**
(21) Numéro de dépôt: 17730841.8
(22) Date de dépôt: 12.05.2017
(51) Int. Cl.: C07K 14/08, C12N 7/02, C12N 7/04, C12N 15/867, C12N 15/90, C07K 14/005, C12N 7/00, C12N 15/86

(54) **PARTICULE POUR L'ENCAPSIDATION D'UN SYSTÈME D'INGÉNIERIE DU GÉNOME**
TEILCHEN FÜR DIE EINKAPSELUNG EINES GENOMTECHNISCHEN SYSTEMS
PARTICLE FOR THE ENCAPSIDATION OF A GENOME ENGINEERING SYSTEM

(30) Priorité: 13.05.2016 FR 1654332; 24.10.2016 FR 1660309; 31.03.2017 FR 1752818
(43) Date de publication de la demande: 20.03.2019
(73) Titulaire: Flash Therapeutics, 31400 Toulouse (FR)
(72) Inventeur: BOUILLE, Pascale, 94300 Vincennes (FR); GAYON, Régis, 31520 Ramonville Saint-Agne (FR); LAMOUROUX, Lucille, 31120 Pinsaguel (FR); ICHE, Alexandra, 31450 Corronsac (FR)
(74) Mandataire: Santarelli
(86) Numéro de dépôt international: PCT/FR2017/051164
(87) Numéro de publication internationale: WO 2017/194902

(56) Documents cités:
- ANNE PREL ET AL: "Highly efficient in vitro and in vivo delivery of functional RNAs using new versatile MS2-chimeric retrovirus-like particles", MOLECULAR THERAPY - METHODS & CLINICAL DEVELOPMENT, vol. 2, 21 octobre 2015 (2015-10-21), page 15039, XP055254802, DOI: 10.1038/mtm.2015.39 -& Prel A et al: "Highly efficient in vitro and in vivo delivery of functional RNAs using new versatile MS2-chimeric retrovirus-like particles: Supplementary Figures S2", www.sciencedirect.com Molecular Therapy - Methods & Clinical Development, vol. 2 21 octobre 2015 (2015-10-21), XP002768084, Extrait de l'Internet: URL:http://www.sciencedirect.com/science/a rticle/pii/S2329050116300511 [extrait le 2017-03-08]
- MICHAEL R. WILLIAMS ET AL: "A Retroviral CRISPR-Cas9 System for Cellular Autism-Associated Phenotype Discovery in Developing Neurons", SCIENTIFIC REPORTS, vol. 6, 10 mai 2016 (2016-05-10), page 25611, XP055352201, DOI: 10.1038/srep25611
- SHEFAH QAZI ET AL: "Programmed Self-Assembly of an Active P22-Cas9 Nanocarrier System", MOLECULAR PHARMACEUTICS, vol. 13, no. 3, 7 mars 2016 (2016-03-07), pages 1191-1196, XP055268807, US ISSN: 1543-8384, DOI: 10.1021/acs.molpharmaceut.5b00822

## Description

La présente invention concerne un système rétroviral de transfert d'ARN non viraux dans des cellules cibles et plus particulièrement une particule rétrovirale capable de délivrer de multiples ARN. L'invention concerne également des compositions comportant ces particules rétrovirales, des kits de production de celles-ci, les procédés de fabrication afférents ainsi que les utilisations des particules et des compositions.

Introduire des ARN multiples dans une cellule cible est un enjeu majeur en recherche et développement comme en thérapie génique.

L'utilisation de vecteurs dérivés de virus est devenue une méthode cruciale de transfert de gènes. Les vecteurs viraux se répartissent aujourd'hui en deux catégories principales:
- les vecteurs intégratifs, qui s'intègrent dans le génome receveur, et
- les vecteurs non-intégratifs, qui forment habituellement un élément génétique extra-chromosomique.

Les vecteurs intégratifs tels que les vecteurs gamma-rétroviraux (RV) et vecteurs lentiviraux (LV) sont intégrés de façon stable. Les vecteurs non-intégratifs, tels que les vecteurs adénoviraux (ADV) et les vecteurs adéno-associés (AAV) sont rapidement éliminés des cellules qui se divisent rapidement. Certains facteurs influençant le choix d'un vecteur particulier, comprennent sa capacité d'encapsidation, sa gamme de cellules cibles ou hôtes, son profil d'expression génique, son efficacité de transduction et sa capacité à induire une réponse immunitaire, ce qui est particulièrement problématique si des transductions répétées sont nécessaires.

Certaines applications nécessitent l'utilisation de vecteurs non-intégratifs comme en thérapie génique ou dans de nombreuses applications *in vitro, ex vivo* et *in vivo.* A titre d'exemples, on peut citer :
- l'induction de la reprogrammation de cellules spécialisées en cellules pluripotentes, ainsi que l'induction de la différentiation de cellules souches ou pluripotentes en cellules spécialisées,
- l'expression d'antigènes ou de protéines (toxiques ou non) simultanément dans une cellule cible,
- l'expression de systèmes d'ingénierie du génome comme par exemple la protéine CRE, les systèmes TALEN, Zn Finger Nuclease ou CRISPR, ou de toute autre système nécessitant une expression de protéine ou d'ARN.

Un moyen d'introduire des ARN dans une cellule cible met en œuvre des vecteurs viraux basé sur des virus appartenant à la famille des *Retroviridae* (également désignée sous l'appellation famille des Rétrovirus ou virus à ARN). En effet, durant son cycle de réplication, un virus de la famille des *Retroviridae* possède la capacité de convertir son génome, constitué d'ARN, en ADN double brin qui sera intégré dans le génome de la cellule cible. Des exemples particuliers de cette famille de Rétrovirus sont les gamma-rétrovirus et les lentivirus.

Le cycle réplicatif d'un rétrovirus comporte une première phase de reconnaissance de la cellule cible (ou hôte) via la fixation à un récepteur membranaire. Cette phase de reconnaissance aboutit, après fusion membranaire, à l'entrée du rétrovirus dans la cellule hôte. L'ARN rétroviral est alors copié en ADN double brin par la transcriptase inverse, codée par le rétrovirus, puis intégré au génome de la cellule hôte. Ce génome viral est alors transcrit et traduit par la cellule hôte comme tous les autres gènes de la cellule. Ce génome code l'ensemble des protéines et des séquences permettant la fabrication d'autres virus.

Plus particulièrement, trois gènes sont communs à l'ensemble des rétrovirus : *gag, pol* et *env.*

*Gag* est un gène codant pour une polyprotéine, les protéines issues de cette polyprotéine par clivage, étant des protéines de structure impliquées dans l'assemblage des virus lors de la réplication. Ces protéines de structure sont plus spécifiquement la protéine de matrice (MA), la protéine de capside (CA) et la protéine de nucléocapside (NC).

*Pol* est un gène codant pour les enzymes intégrase, transcriptase inverse et protéase.

*Env* est un gène codant pour des glycoprotéines d'enveloppe.

Ces trois gènes permettent donc de copier l'ARN rétroviral en ADN double brin, d'intégrer cet ADN au génome de la cellule hôte puis de générer la structure des rétrovirus néo-synthétisés : protéines d'enveloppe, de capside et de nucléocapside. Toutefois, afin que les rétrovirus néo-synthétisés soient complets, il est nécessaire d'encapsider dans chacune de ces structures deux copies de l'ARN rétroviral. Cette encapsidation de deux copies de l'ARN rétroviral dans la structure s'effectue par la reconnaissance par la protéine de nucléocapside, d'une séquence d'encapsidation appelée Psi (pour « Packaging Signal ») portée par la copie de l'ARN rétroviral.

Lorsqu'un vecteur viral dérivé d'un rétrovirus est utilisé à des fins de thérapie génique, au moins une partie des régions codantes de *gag, pol* et/ou *env* est dissociée entre le système d'encapsidation et le système d'expression de la séquence d'intérêt. Les séquences codantes *gag* et *pol,* par exemple, sont portées par le plasmide d'encapsidation apportant en trans les protéines nécessaires à la production de vecteurs viraux. La séquence d'encapsidation comme la séquence d'intérêt sont portée par des systèmes indépendants, afin de rendre le rétrovirus non réplicatif.

Toutefois, dans certains cas, l'intégration de la séquence d'ARN d'intérêt dans le génome de la cellule hôte de façon aléatoire peut interrompre une phase ouverte de lecture et bloquer l'expression de gènes importants. De plus, pour certaines applications, telles que la reprogrammation de cellules ou la différentiation de cellules souches, il est recommandé que l'expression d'une séquence d'intérêt soit réalisée de manière transitoire.

La demande WO2007/072056 décrit un système de vectorisation viral comprenant *env, gag,* optionnellement *gag*/*pol* ainsi qu'une séquence d'ARN contenant un signal d'encapsidation hétérologue qui est reconnu par un domaine de liaison à ARN correspondant associé à *gag* ou à *gag*/*pol.* Ce système est décrit dans la demande comme étant non intégratif et permettant une expression transitoire.

Toutefois, l'efficacité de tels systèmes reste limitée. En particulier, pour qu'une cellule cible exprime un ARN d'intérêt véhiculé par ces systèmes, il est généralement nécessaire d'introduire dans la cellule plusieurs copies de cet ARN d'intérêt et par conséquent, d'utiliser de fortes multiplicités d'infection (« multiplicity of infection » ou MOI). La MOI correspond au ratio du nombre de systèmes de vectorisation introduit sur le nombre de cellules à infecter. Une forte MOI permet en effet d'introduire dans les cellules plusieurs copies de l'ARN d'intérêt en permettant qu'une même cellule subisse plusieurs infections. Or, si elle permet d'améliorer le niveau d'expression de l'ARN d'intérêt véhiculé, l'utilisation de fortes MOI, du fait des multiples infections subies par la cellule, engendre également une certaine toxicité.

Comme mentionné précédemment, ce type de système présente également un intérêt pour l'ingénierie du génome, et notamment pour des applications en thérapie génique.

A l'heure actuelle, l'introduction de systèmes d'ingénierie du génome dans des cellules cibles reste complexe et constitue un enjeu majeur pour la mise en œuvre avec succès des systèmes d'ingénierie du génome mentionnés ci-avant.

En effet, pour qu'un système d'ingénierie du génome puisse fonctionner, il convient d'introduire *a minima* dans la cellule cible une enzyme capable de cliver l'ADN ou nucléase. Les applications en thérapie génique requièrent, en outre, une spécificité de la coupure à l'ADN afin de cibler précisément un gène donné, sans générer de coupure non souhaitée. Aussi, pour l'utilisation des systèmes TALEN, ZnFinger et CRISPR, il est généralement nécessaire d'introduire un élément de reconnaissance à l'ADN. Par conséquent, de tels systèmes nécessitent l'introduction dans les cellules transduites d'au moins une nucléase, et préférentiellement de deux éléments constitutifs du système d'ingénierie concerné.

En outre, ces système d'ingénierie du génome doivent tenir compte de paramètres tels que la durée et l'intensité de l'expression du matériel génétique introduit dans la cellule cible, pour permettre une efficacité satisfaisante du système sans induire de toxicité.

Les méthodes existantes à l'heure actuelle de délivrance de ces systèmes d'ingénierie du génome sont principalement :
- la transfection d'ADN,
- l'électroporation d'ARN, et
- l'utilisation de vecteurs viraux.

Toutefois, chacune de ces méthodes présentent de sérieux inconvénients.

En effet, la transfection d'ADN est un procédé qui a une très faible efficacité sur des cellules primaires ou sensibles, du fait d'une toxicité élevée liée au transfert d'ADN, à la présence de séquences bactériennes dans les plasmides et/ou de l'intégration aléatoire des plasmides dans le génome des cellules transfectées.

L'électroporation d'ARN messager quant à elle est une méthode utilisée préférentiellement *ex vivo,* dans des cellules primaires ou sensibles, qui ne permet pas non plus d'obtenir de bons résultats en termes d'efficacité à cause d'une toxicité élevée résultant de la déstabilisation des membranes des cellules cibles.

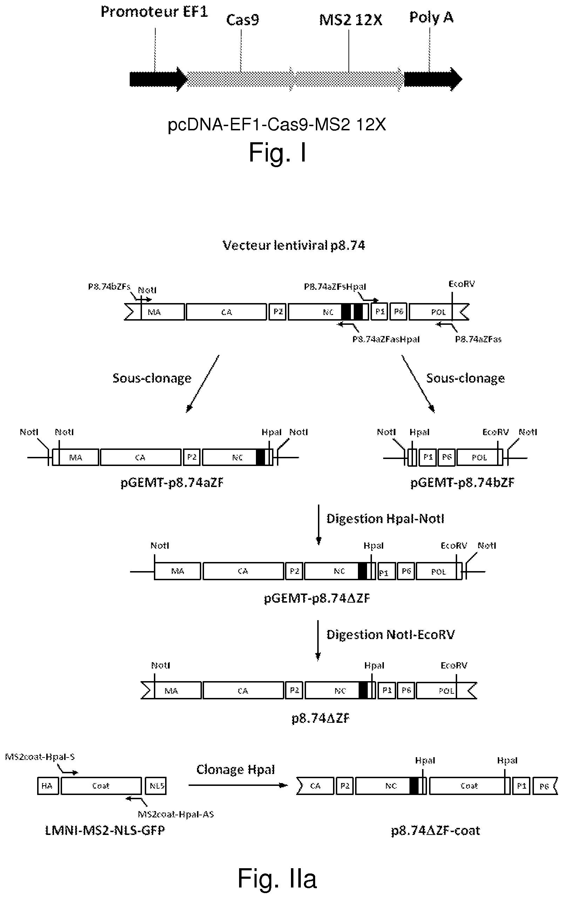

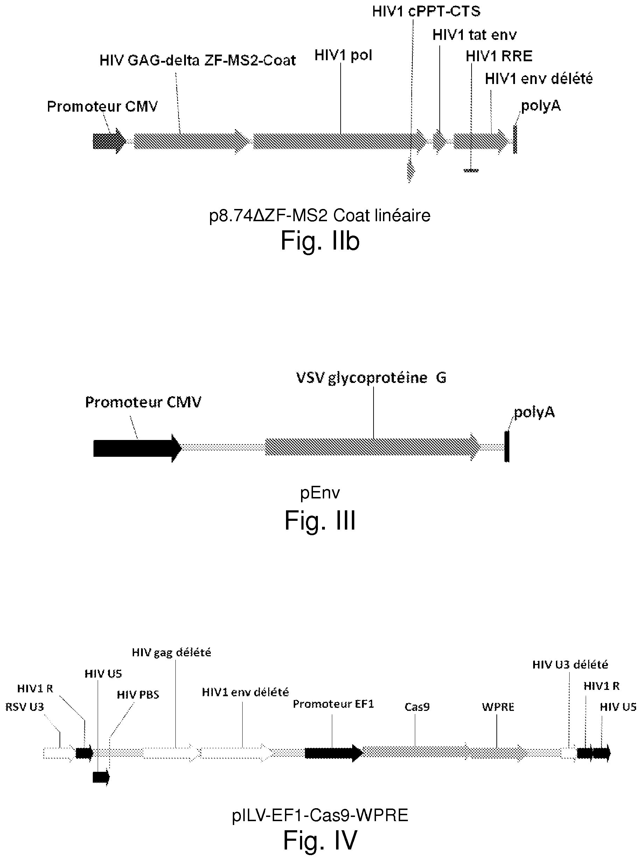

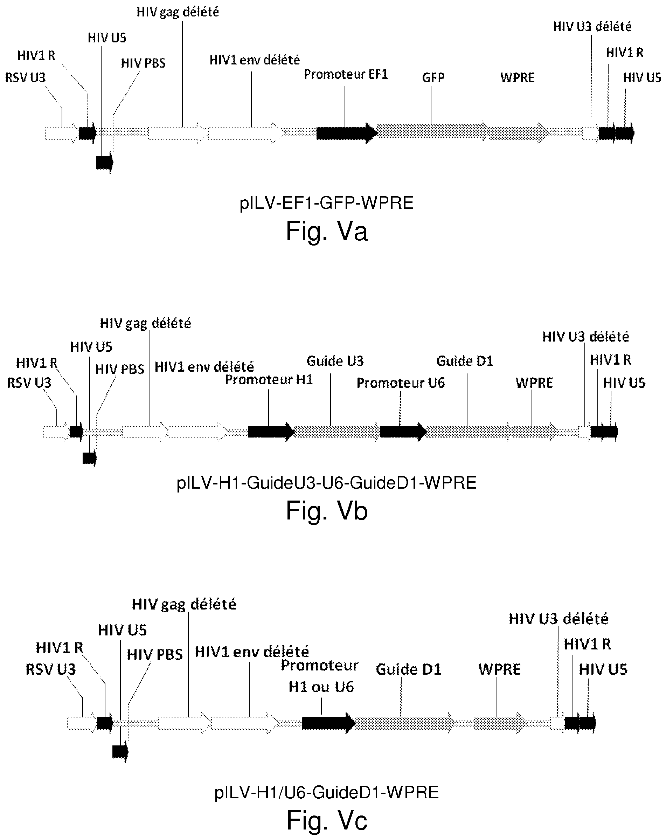

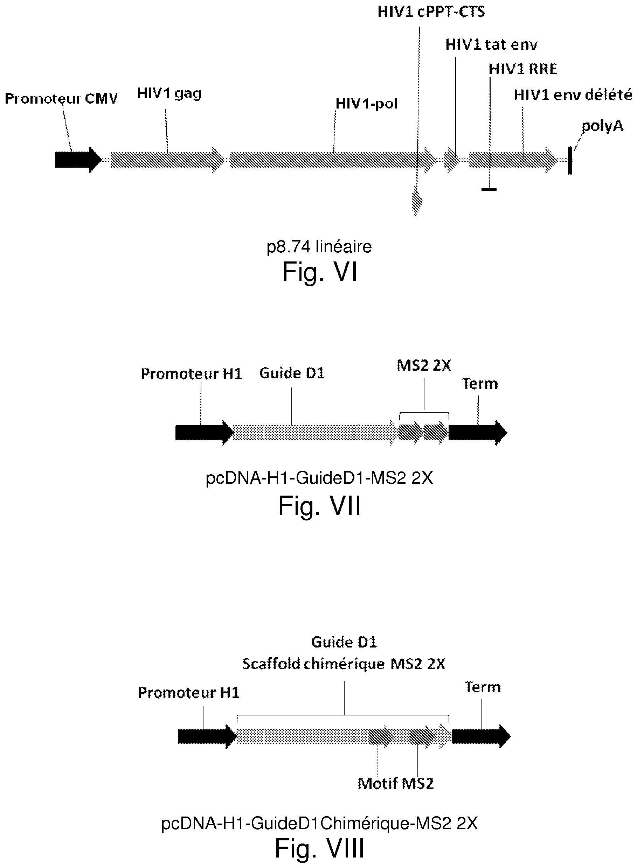

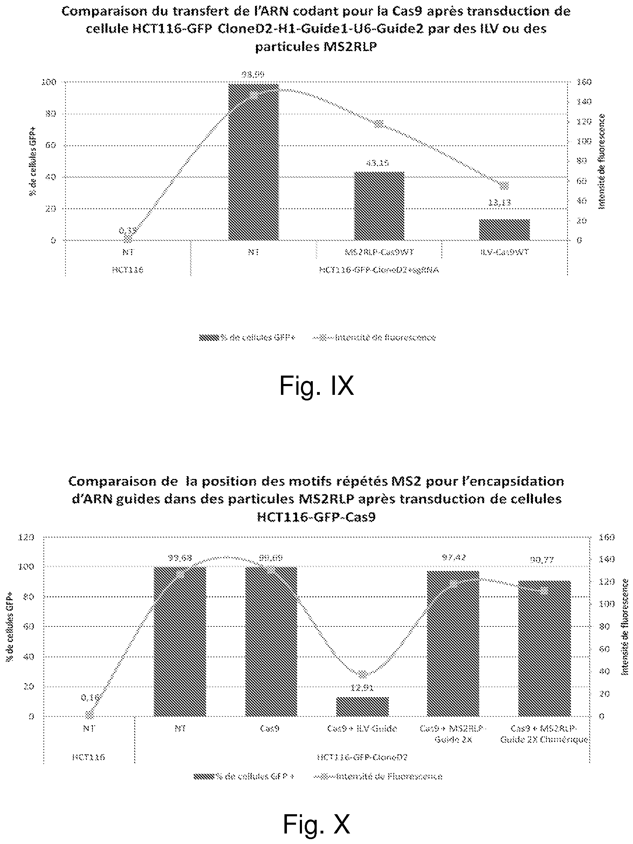

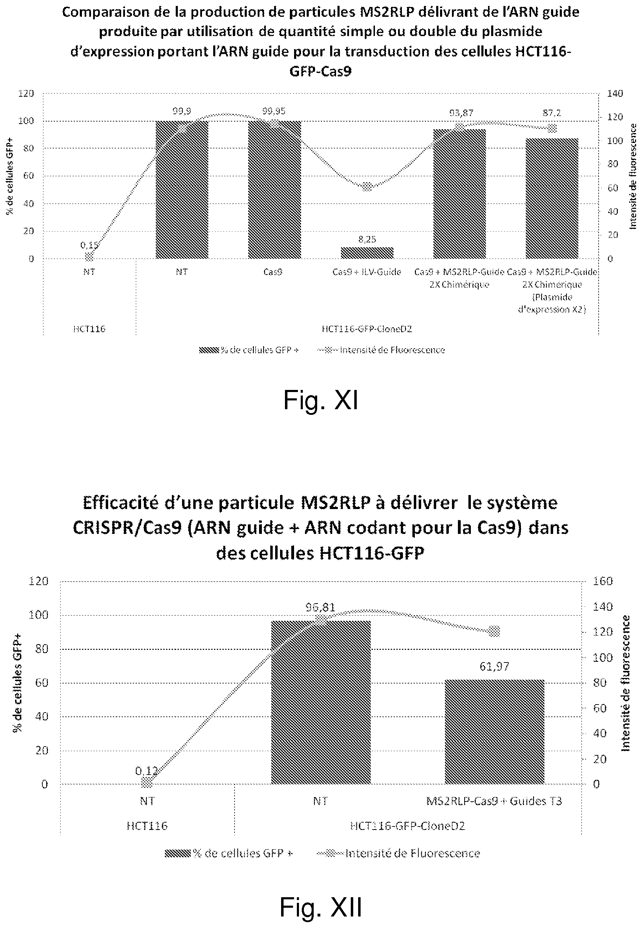

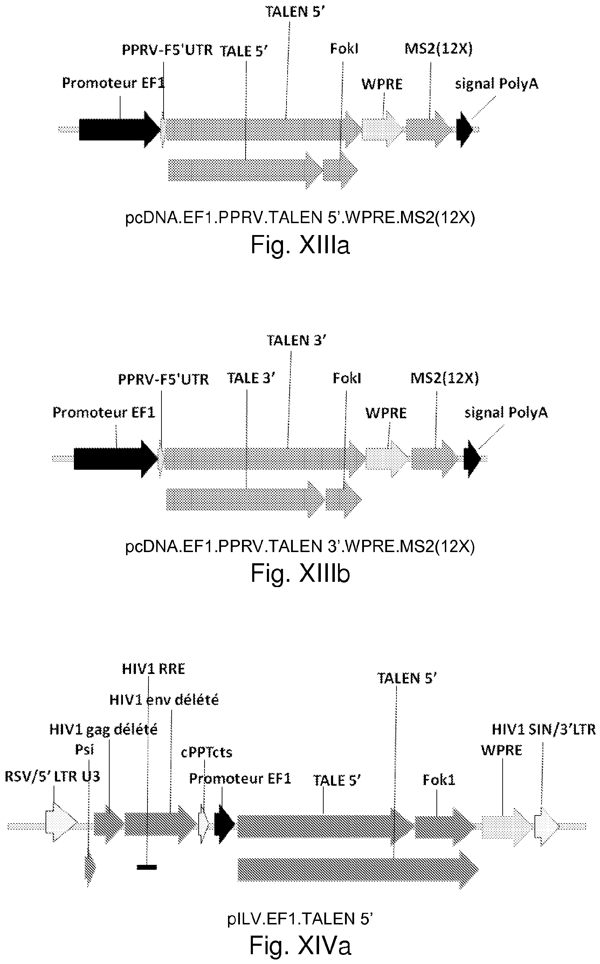

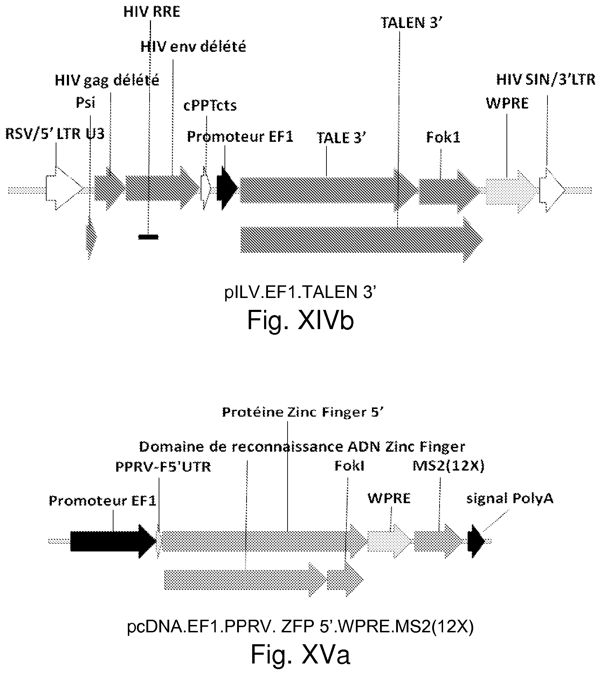

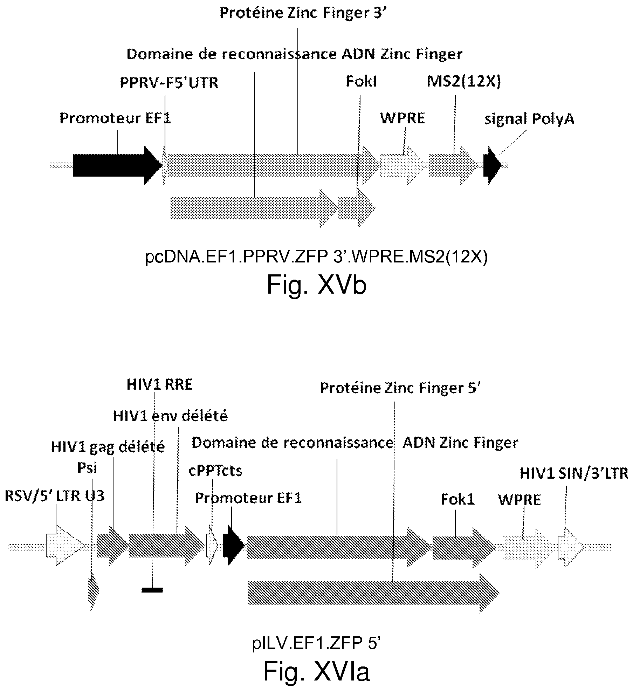

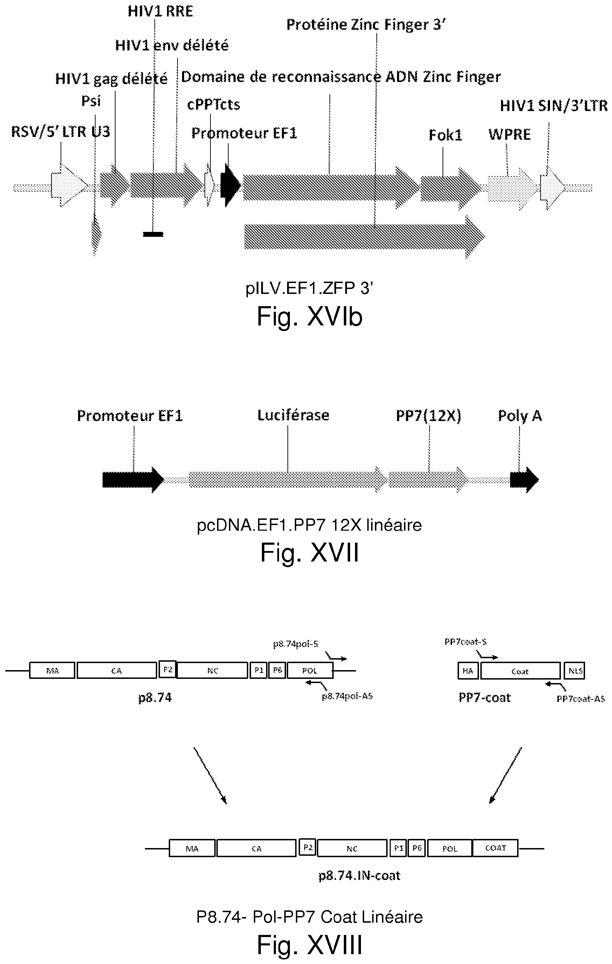

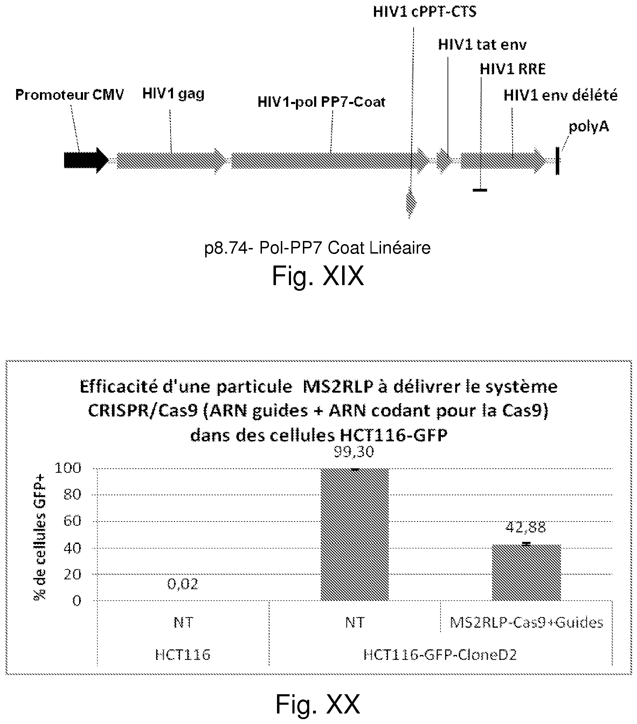

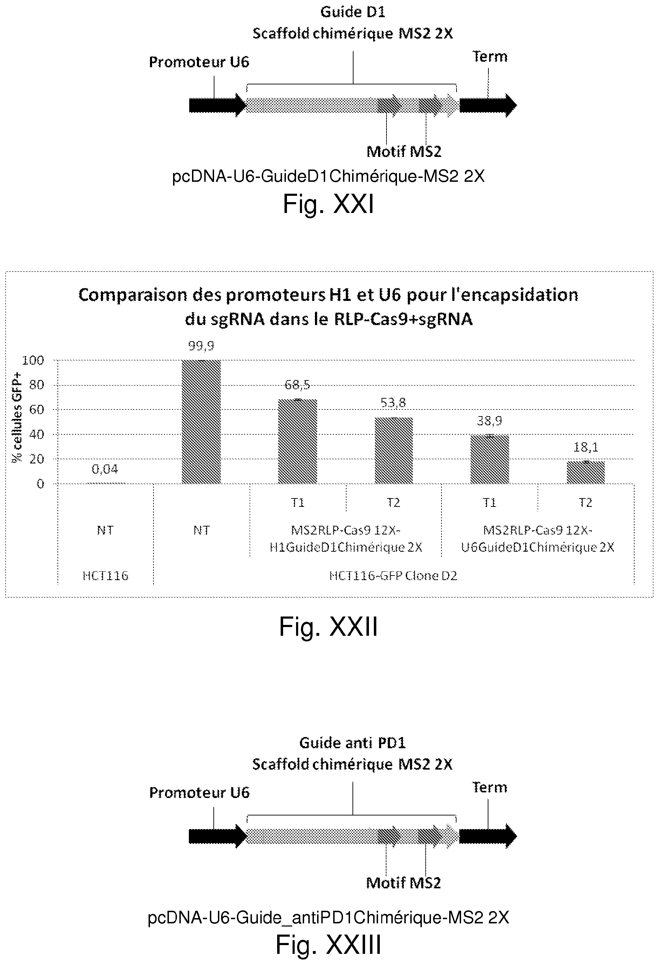

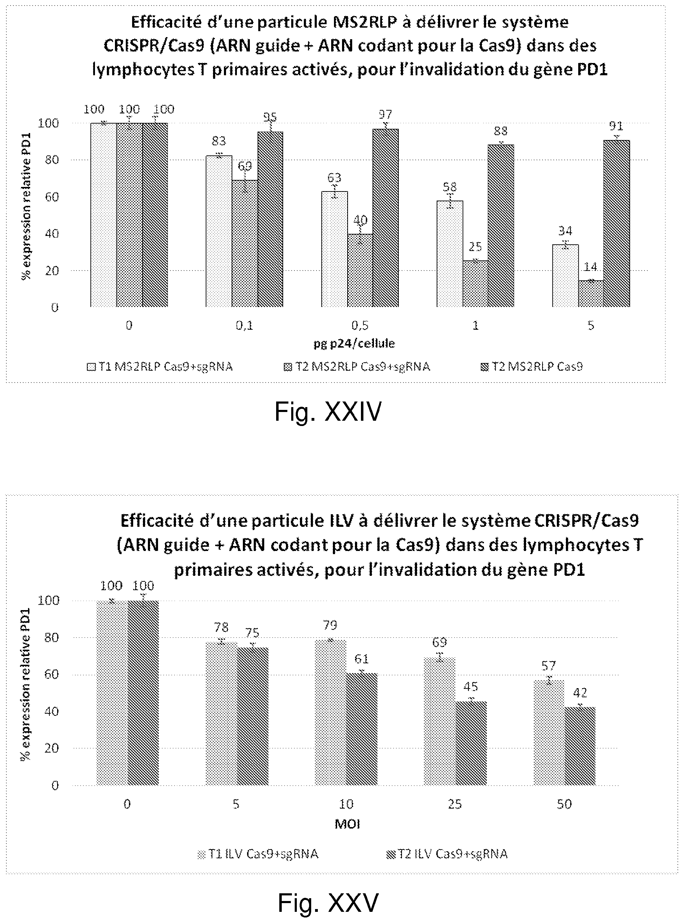

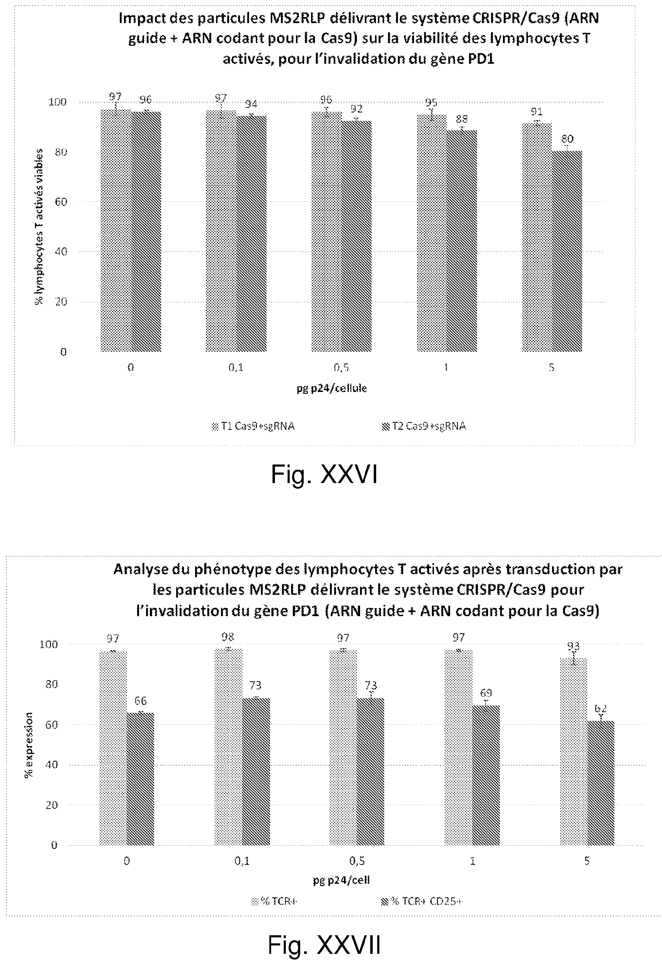

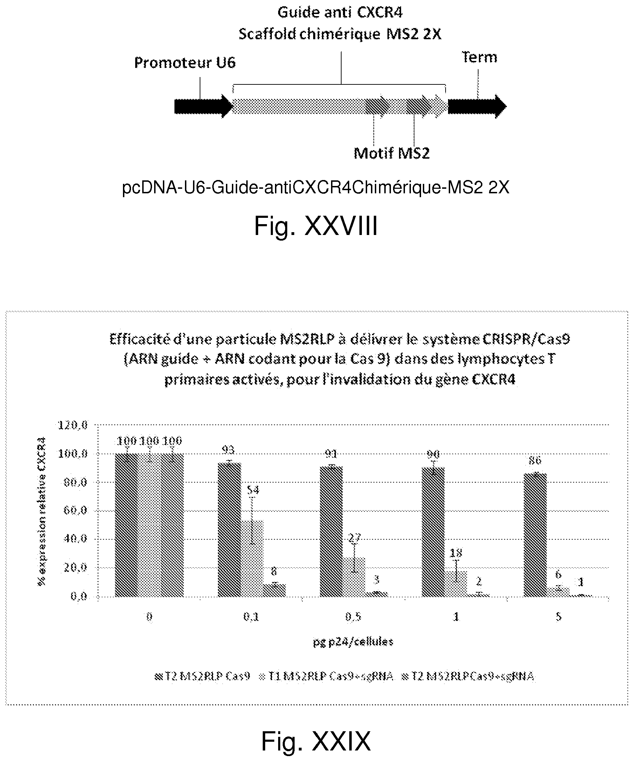

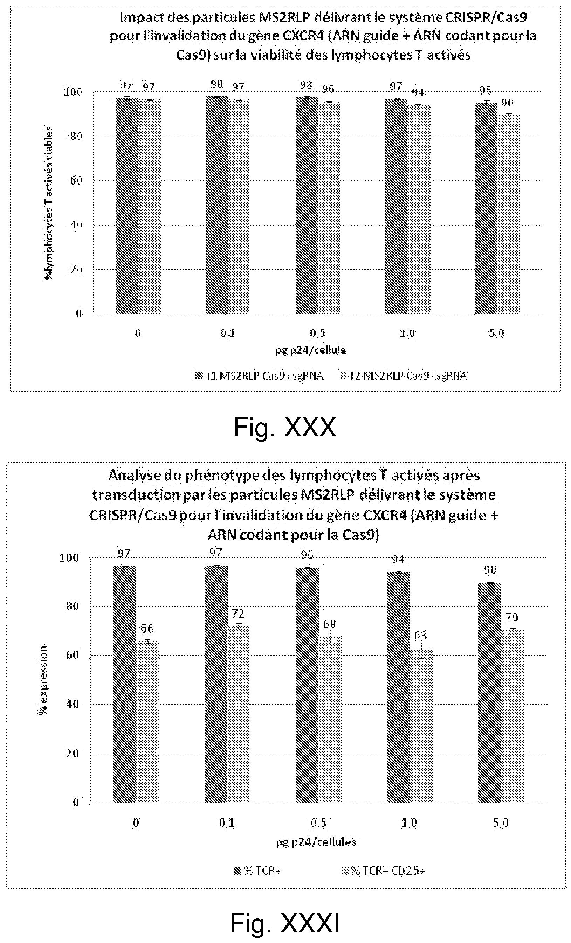

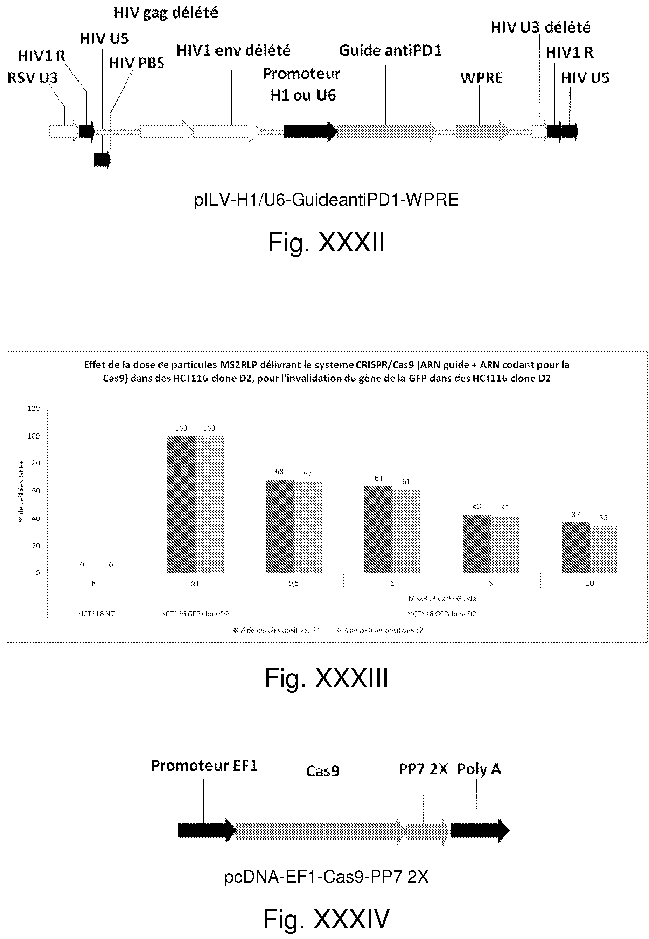

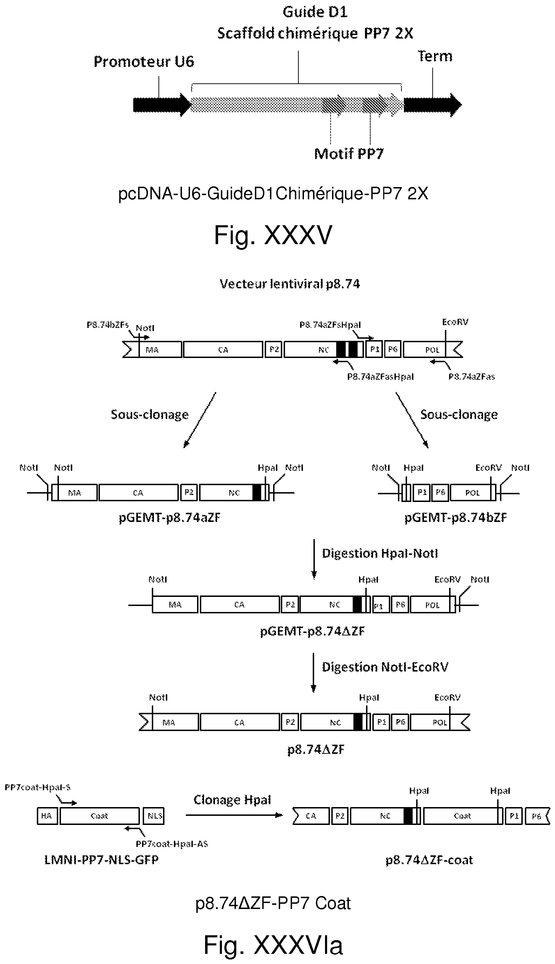

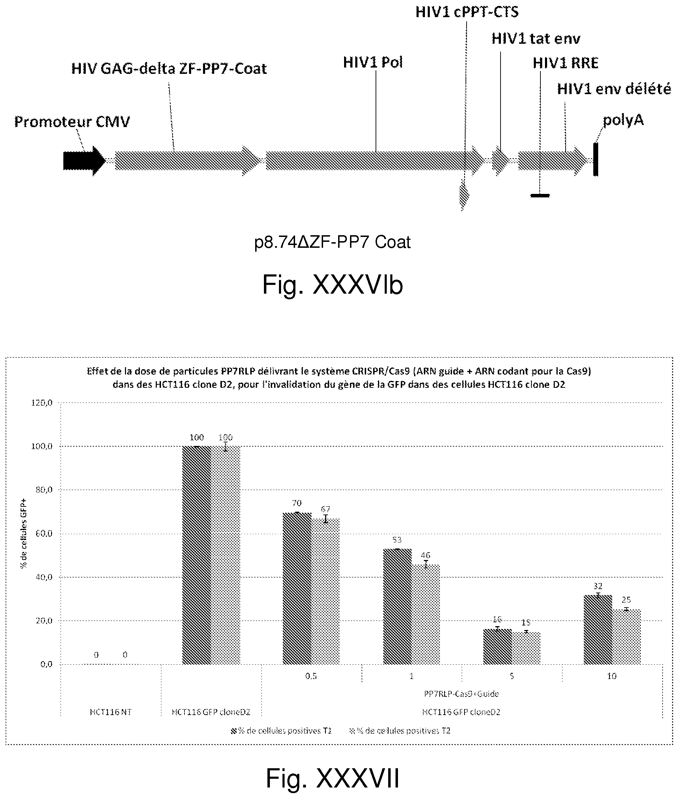

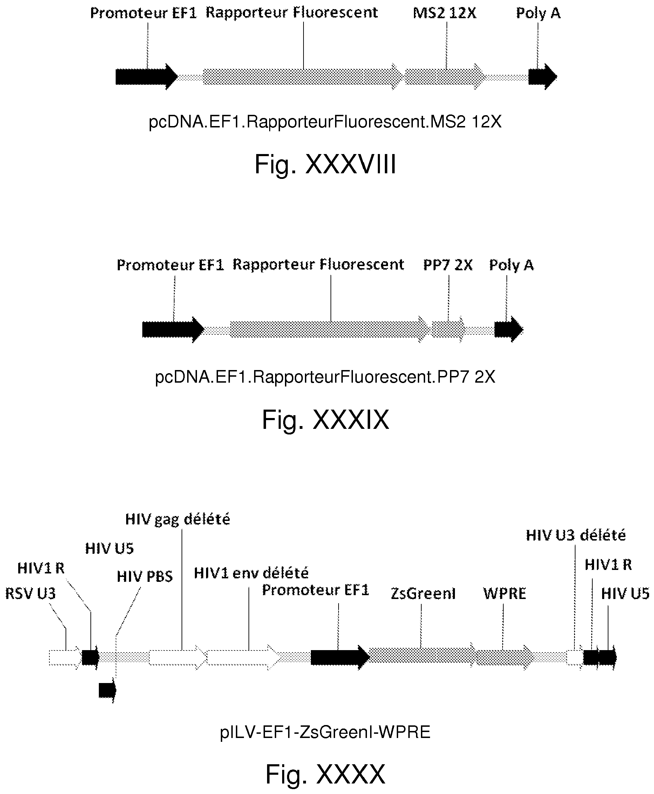

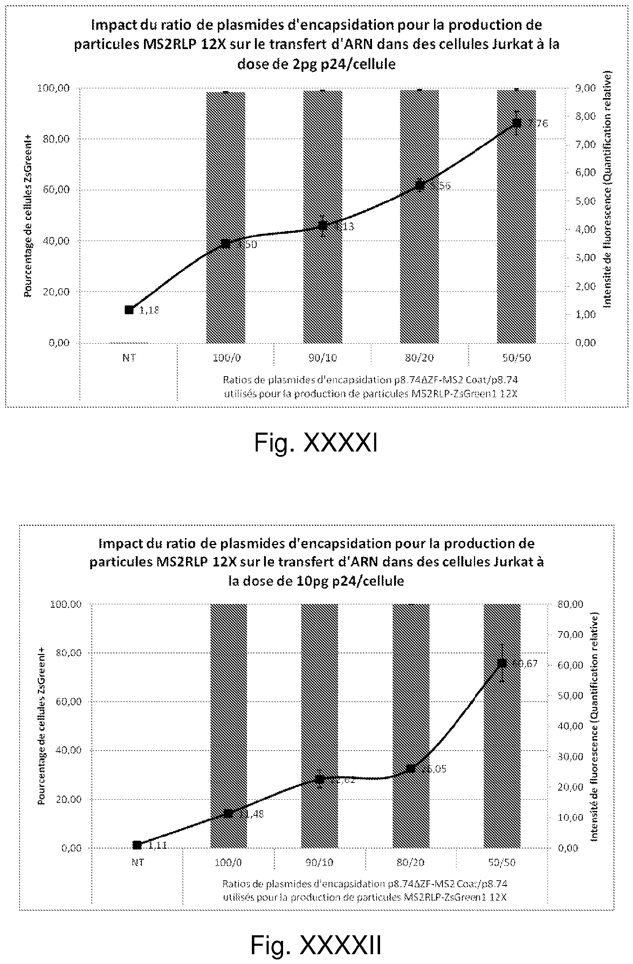

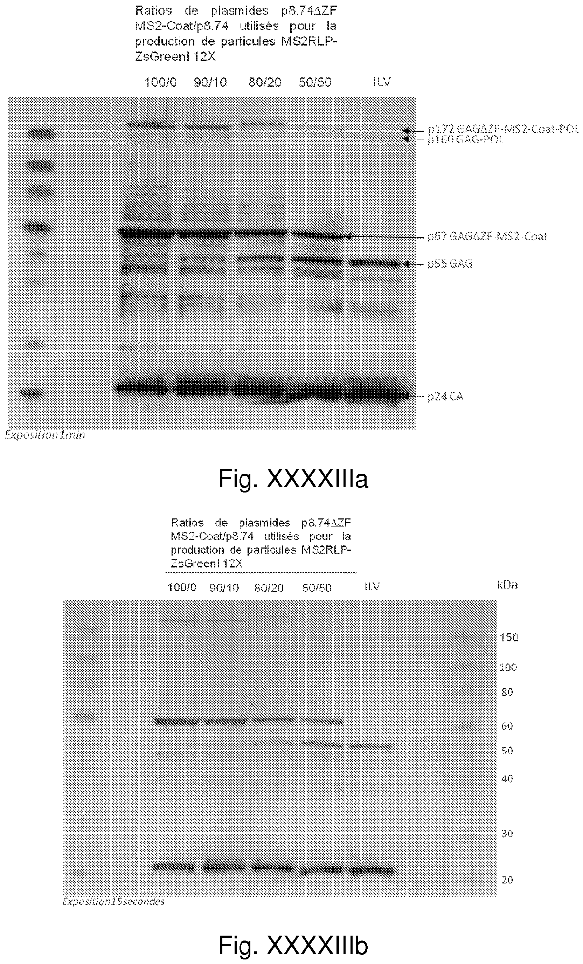

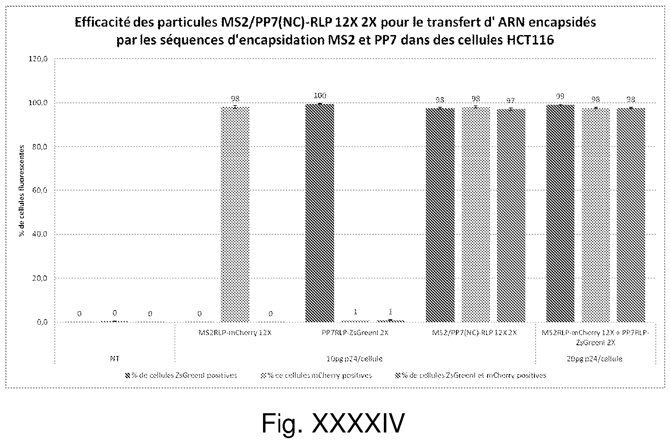

L'utilisation de vecteurs viraux semble être la méthode la plus prometteuse pour augmenter l'efficacité de la délivrance, tout en minimisant la cytotoxicité. Le document par Williams M.R. et al (2016) décrit le transfert du système CRISPR par des vecteurs viraux dérivés du MoMuLV ou de HIV. Les éléments du système CRISPR sont portés par le plasmide d'expression (figure 1b).

Toutefois, si les vecteurs lentiviraux sont des outils efficaces, ils s'intègrent dans le génome des cellules cibles pour une expression stable du transgène qui peut conduire à des réactions incontrôlées. En outre, les vecteurs lentiviraux déficients pour l'intégration (IDLV) peuvent être utilisés afin de limiter la durée d'expression de la nucléase, néanmoins ils ne garantissent pas l'absence totale d'intégration dans le génome des cellules cibles.

L'autre alternative est l'utilisation de systèmes adénoviraux d'AAV pour des applications *in vivo,* mais ce type d'outil implique l'utilisation d'une nucléase de taille réduite à cause d'une capacité d'encapsidation des systèmes adénoviraux limitée à une taille inférieure à 4,8kb, incompatibles avec les systèmes CRISPR ou TALEN (Chen, 2015). Le document par Prel. A. et al (2015) décrit l'introduction d'ARNs fonctionnels utilisant des particules de type rétrovirus-MS2 chimériques.

Il existe donc toujours un besoin pour des systèmes de vectorisation viraux plus efficaces et moins toxiques.

Le travail des inventeurs a permis de réaliser un système de vectorisation capable de délivrer plusieurs ARN d'intérêt dans une même cellule en une seule infection.

La présente invention se rapporte donc à une particule rétrovirale comportant une protéine issue de la polyprotéine Gag, une protéine d'enveloppe, optionnellement une intégrase et au moins deux ARN non viraux encapsidés, les ARN non viraux encapsidés comportant chacun une séquence d'ARN d'intérêt liée à une séquence d'encapsidation, chaque séquence d'encapsidation étant reconnue par un domaine de liaison hétérologue introduit dans la protéine issue de la polyprotéine Gag et/ou dans l'intégrase, dans laquelle les au moins deux ARN non viraux encapsidés se distinguent par leur séquence d'intérêt, l'une au moins desdites séquences d'intérêt des ARN non viraux encapsidés comportant une partie codant pour une nucléase choisie parmi le groupe constitué par les nucléases associées au système CRISPR, et la séquence d'intérêt de l'autre ARN non viral encapsidé correspondant à au moins un élément de reconnaissance d'un guide d'ARN.

La particule rétrovirale selon l'invention permet d'introduire au moins deux ARN non viraux, préférentiellement 3, dans une cellule par une seule infection. L'introduction de telles particules dans les cellules peut être effectuée par un procédé *in vivo, in vitro* ou *ex vivo.*

Par « protéine issue de la polyprotéine Gag », on entend toute protéine résultant du clivage de la polyprotéine Gag. Plus particulièrement, il s'agit d'une protéine de nucléocapside, d'une protéine de matrice (par exemple, dans des particules rétrovirales dérivées du virus murins de type MoMuLV) ou de la protéine p12, spécifique aux gammaretrovirus.

Par « protéine d'enveloppe », on entend toute protéine d'enveloppe, y compris une protéine d'enveloppe de pseudotypage. A titre d'exemple, on peut citer la protéine d'enveloppe Ampho, la protéine d'enveloppe écotrope, la protéine d'enveloppe du virus Moloney de la leucémie murine (MoMuLV), la protéine d'enveloppe du virus de l'Immunodéficience Féline (FIV), la protéine d'enveloppe du virus du sarcome murin d'Harvey (HaMuSV), la protéine d'enveloppe du virus de la tumeur mammaire murine (MuMTV), la protéine d'enveloppe du virus du Sarcome de Rous (RSV), la protéine d'enveloppe du virus de la rougeole (aussi MV pour *measles* virus), la protéine d'enveloppe du virus de la leucémie du Gibbon (GaIV), la protéine du virus endogène félin (RD114) ou la protéine d'enveloppe du virus de la stomatite vésiculaire (VSV-G). Plus particulièrement, la protéine d'enveloppe est la protéine d'enveloppe Ampho, la protéine d'enveloppe écotrope, la protéine d'enveloppe du virus Moloney de la leucémie murine (MoMuLV), la protéine d'enveloppe du virus de l'Immunodéficience Féline (FIV), la protéine d'enveloppe du virus du sarcome murin d'Harvey (HaMuSV), la protéine d'enveloppe du virus de la tumeur mammaire murine (MuMTV), la protéine d'enveloppe du virus du Sarcome de Rous (RSV), la protéine d'enveloppe du virus de la rougeole (aussi MV pour *measles* virus), la protéine d'enveloppe du virus de la leucémie du Gibbon (GalV) ou la protéine d'enveloppe du virus de la stomatite vésiculaire (VSV-G). La protéine d'enveloppe peut ainsi être modifiée pour cibler certains types cellulaires ou certaines applications (utilisation de récepteurs de surface comme protéine d'enveloppe).

Il est également possible de modifier la protéine d'enveloppe avec un anticorps, un glycolipide et/ou un ligand particulier afin de cibler un récepteur et/ou un type cellulaire particulier.

Préférentiellement, la protéine d'enveloppe est la protéine VSV-G.

Par « intégrase », on entend la protéine enzymatique codée par le gène *pol,* qui permet l'intégration de l'ADN rétroviral dans l'ADN de la cellule infectée par le rétrovirus lors de la réplication dudit rétrovirus.

Par « séquence d'encapsidation », on désigne un motif (séquence et structure tridimensionnelle) ARN reconnu spécifiquement par un domaine de liaison. Préférentiellement, la séquence d'encapsidation est un motif tige-boucle. Plus préférentiellement encore, la séquence d'encapsidation de la particule rétrovirale est le motif tige-boucle de l'ARN du bactériophage MS2 ou du phage PP7 tel que par exemple, celui résultant de la séquence ctagaaaacatgaggatcacccatgtctgcag (SEQ ID N°1) ou (ctagaaaggagcagacgatatggcgtcgctccctgcag SEQ ID N°2) respectivement. Le motif tige-boucle et plus particulièrement le motif tige-boucle de l'ARN du bactériophage MS2 ou celui de l'ARN du phage PP7, peut être utilisé seul ou répété plusieurs fois, de préférence de 2 à 25 fois, plus préférentiellement de 2 à 18 fois, par exemple de 6 à 18 fois.

Par « domaine de liaison », on désigne tout ou partie d'une protéine se liant spécifiquement à la séquence d'encapsidation liée à la séquence d'ARN d'intérêt. Plus particulièrement, il s'agit d'une protéine mutante ou non, définie par une structure tridimensionnelle, se liant spécifiquement à la séquence d'encapsidation. Préférentiellement, le domaine de liaison est un domaine hétérologue. Plus préférentiellement, le domaine de liaison est la protéine Coat du bactériophage MS2, du phage PP7 ou du phage Qβ, la protéine nun du prophage HK022, la protéine U1A ou encore la protéine hPum.

Plus préférentiellement, le domaine de liaison est la protéine Coat du bactériophage MS2 ou du phage PP7.

Plus préférentiellement encore, lorsque le domaine de liaison est la protéine Coat du phage PP7, la séquence de celle-ci est déficiente pour l'auto-assemblage, grâce à une délétion des acides aminés 67 à 75 (PCPΔFG) (Chao et al. 2008). Préférentiellement, la séquence de la protéine Coat du phage PP7 est codon-optimisée pour les cellules humaines, c'est-à-dire que les bases de l'ADN sont choisies pour coder pour des acides aminés préférentiellement présents dans l'espèce humaine.

Par « chaque séquence », on entend que les séquences d'encapsidation peuvent être identiques ou différentes, selon que ces séquences d'encapsidation sont reconnues par un domaine de liaison identique ou différent. En effet, la particule rétrovirale selon l'invention peut comporter un ou plusieurs domaines de liaison. Lorsque plusieurs domaines de liaison sont introduits, ceux-ci peuvent l'être dans la polyprotéine Gag et/ou dans l'intégrase.

Le domaine de liaison permet non seulement la reconnaissance de la séquence d'encapsidation mais également l'encapsidation des ARN portant la séquence d'encapsidation dans la particule (ou dans le cas présent, d'un ARN non viral lié à une séquence d'encapsidation).

Par « encapsidation », on entend l'empaquetage d'un ARN dans la capside virale d'une particule virale. On notera que dans la présente invention, l'encapsidation des ARN non viraux s'effectue par la reconnaissance d'un signal d'encapsidation non viral, en particulier autre que Psi.

Par « séquence d'intérêt », on vise une séquence codant pour ou ayant une fonction présentant un intérêt pour l'utilisateur. Plus particulièrement, la séquence d'intérêt portée par chacun des deux ARN non viraux encapsidés peut être identique ou différente.

A l'aide des particules selon l'invention, il est possible de transférer dans des cellules cibles des ARN non viraux différents.

Les ARN encapsidés étant non viraux, ces ARN ne présentent pas les sites de reconnaissance des protéines codées par le gène *pol.*

En effet, ces ARN non viraux n'entrent pas dans les étapes précoces du cycle réplicatif des rétrovirus, à savoir :
- La copie de l'ARN viral simple brin en ADN double brin par la transcriptase inverse ;
- La maturation de l'ADN viral double brin par reconnaissance des extrémités LTR par l'intégrase et maturation du complexe de pré-intégration cytoplasmique en complexe de pré-intégration nucléaire.

Ces protéines virales (transcriptase inverse, intégrase), codées par le gène pol sont donc optionnelles dans la particule et le gène *pol* peut donc être soit présent, soit délété partiellement ou totalement. Préférentiellement, le gène *pol* est soit présent, soit délété partiellement.

On notera que les particules rétrovirales selon l'invention comportent un matériel génétique à la fois viral et non viral :
- le gène *gag,* qui peut être viral ou chimérique. Plus particulièrement, le gène *gag* est chimérique lorsque le ou les domaine(s) de liaison est/sont introduit(s) dans celui-ci.
- Optionnellement, le gène *pol,* qui peut être viral ou chimérique. Le gène *pol* codant pour plusieurs enzymes, les séquences afférentes à ces enzymes peuvent être délétées totalement ou partiellement, présentes et non fonctionnelles, ou présentes et fonctionnelles. Plus particulièrement, le gène *pol* est chimérique lorsque le ou les domaine(s) de liaison est/sont introduit(s) dans l'intégrase.
- Au moins deux ARN non viraux, chaque ARN non viral étant porteur d'une séquence d'intérêt et d'une séquence d'encapsidation. Plus particulièrement, ces ARN non viraux sont dépourvus de toute séquence virale.

Plus spécifiquement, les particules rétrovirales selon la divulgation permettent d'introduire dans des cellules cibles des ARN capables d'induire :
- Le transfert d'une ou plusieurs séquences d'intérêt codantes endogènes ou exogènes de la cellule cible,
- Le transfert d'un ou plusieurs ARN non codants comme des ARN capables d'induire un effet sur l'expression génétique, par exemple par le biais de shRNA, miRNA, sgRNA, LncRNA ou circRNA.
- Le transfert d'ARN cellulaires, de type ARN messagers ou autres (miRNA...), des réplicons sous génomique de virus à ARN (VHC, etc...) ou de génomes complets de virus à ARN,
- l'expression simultanée de séquences codantes ou non codantes endogènes, exogènes de la cellule cible,
- participer à la modification du génome de la cellule cible par des systèmes d'ingénierie du génome, par exemple, selon l'invention, le système CRISPR.

Par « nucléase », on entend une enzyme ayant la capacité de cliver un ou deux brins d'ADN, une nucléase ayant la capacité d'initier le clivage d'une liaison phosphodiester des acides nucléiques de deux nucléotides.

Avantageusement, la nucléase est une endonucléase, c'est-à-dire qu'elle coupe le brin d'ADN à l'intérieur de celui-ci.

La nucléase peut par ailleurs générer des extrémités cohésives ou des extrémités non cohésives. Elle génère avantageusement des extrémités cohésives.

La nucléase peut être sous sa forme native (WT), sous une forme mutée, sous une forme optimisée, sous une forme tronquée ou une forme invalidée. Il est en effet possible de muter la nucléase native pour invalider ses capacités de clivage de la liaison phosphodiester des acides nucléiques.

L'une au moins des séquences d'intérêt des ARN non viraux encapsidés comporte une partie codant pour une nucléase, c'est-à-dire que cette séquence d'ARN peut comprendre uniquement une séquence d'ARN codant pour une nucléase ou alternativement une séquence d'ARN codant pour une nucléase et une ou plusieurs autre(s) séquence(s) d'ARN, ces autres séquences d'ARN pouvant par exemple coder pour au moins une protéine, identique ou différente, ou une séquence d'ARN non codante.

Plus particulièrement, la nucléase est choisie parmi le groupe constitué par les nucléases associées au système CRISPR.

Le système d'ingénierie du génome CRISPR/Cas9 est devenu un outil de génie génétique à fort potentiel. Il fait intervenir deux types de composants :
- Les CRISPR (Clustered Regularly Interspaced Short Palindromic Repeats) caractérisées par des séries de répétitions directes courtes (de 21 à 37 paires de bases) et régulièrement espacées par des séquences appelées « spacer », généralement uniques, de 20 à 40 paires de bases ;
- Une des nucléases associées au système CRISPR, préférentiellement la nucléase Cas9 (CRISPR associated protein 9), c'est-à-dire une enzyme spécialisée pour couper l'ADN avec deux zones de coupe actives, une pour chaque brin de la double hélice. Par exemple, S. pyogenes utilise l'outil Cas9 pour détecter et défaire l'ADN étranger, tels que l'invasion de l'ADN de bactériophages ou d'un ADN plasmidique. Cas9 effectue cette détection par déroulement de l'ADN étranger et la vérification de complémentarité avec la région longue d'espacement d'une vingtaine de paires de base de l'ARN guide. Si une séquence d'ADN est apparentée à l'ARN guide, Cas9 découpe l'ADN invasif.

Ce système est utilisé pour modifier facilement et rapidement le génome des cellules animales et végétales. Grâce à la spécificité de la cible de Cas9 qui provient de l'ARN guide et de la complémentarité de l'ADN et non pas des modifications de la protéine elle-même (comme TALEN et les nucléases à doigt de zinc), l'outil Cas9 peut cibler un nouvel ADN très facilement.

Le système d'ingénierie du génome TALEN utilise une nucléase artificielle générée par la fusion d'un domaine de liaison à l'ADN, appelé TALE, avec un domaine ayant la capacité de cliver l'ADN, appelée TALENs. La relation simple entre la séquence en acides aminés et la séquence d'ADN reconnue par le domaine de liaison à l'ADN permet de synthétiser des protéines personnalisées. Ces systèmes peuvent être conçus pour se fixer à quasiment n'importe quelle séquence spécifique d'ADN cible en combinant un domaine de reconnaissance de l'ADN cible TALE avec un domaine de clivage de l'ADN.

Les TAL effector (TALE) sont des protéines naturellement sécrétées par la bactérie Xanthomonas comprenant un domaine de liaison à l'ADN constitué de répétitions de 33 ou 34 acides aminés identiques à l'exception des acides aminés 12 et 13 très variables qui confère la reconnaissance d'un nucléotide spécifique. Cette relation simple entre la séquence en acides aminés et la reconnaissance de l'ADN permet de créer des domaines de liaison à l'ADN spécifiques d'une séquence en sélectionnant un assemblage de segments répétés contenant les résidus variables appropriés. Ces domaines de liaison à l'ADN spécifiques d'une séquence peuvent être rapidement conçus pour se fixer à quasiment n'importe quelle séquence d'ADN voulue.

Le domaine de clivage de l'ADN non spécifique de l'endonucléase Fok1 peut être utilisé pour construire des nucléases hybrides qui ont prouvé leur efficacité dans des essais chez la levure, dans les cellules végétales et animales. Les premières études sur les TALEN ont été réalisées avec le domaine original de Fok1, ou des variants de Fok1 présentant une plus grande spécificité ou efficacité du clivage de l'ADN.

Les domaines Fok1 fonctionnent sous forme de dimère, ce qui nécessite deux constructions avec des domaines de liaison à l'ADN dirigés contre des sites du génome présentant des orientations tête-bêche et un espacement correct.

En combinant un domaine TALE avec un domaine de clivage de l'ADN (qui va couper le brin d'ADN), on peut ainsi créer des enzymes de restriction spécifiques pour n'importe quelle séquence d'ADN.

Le système Zn Finger utilise la nucléase à doigts de zinc, et est constitué d'une association de deux protéines, comportant chacune trois doigts de zinc de reconnaissance de l'ADN spécifiquement choisis et d'un domaine catalytique de l'endonucléase Fok I.

La nature des acides aminés qui composent un doigt de zinc va conduire à la reconnaissance d'une séquence spécifique d'ADN de trois nucléotides. Ainsi en utilisant deux protéines comportant chacune trois doigts de zinc spécifiques, la reconnaissance s'effectue sur une séquence spécifique de dix-huit nucléotides et conférera une spécificité de reconnaissance sur le génome.

L'endonucléase utilisée est le domaine endonucléasique de l'enzyme Fok I et agit sous forme dimérique.

Les domaines Fok1 fonctionnent sous forme de dimère, ce qui nécessite deux constructions avec des domaines de liaison à l'ADN dirigés contre des sites du génome présentant des orientations tête-bêche et un espacement correct.

Avantageusement, deux protéines comportant chacune trois structures en doigt de zinc reconnaissant des séquences éloignées de quelques nucléotides sont utilisées. La liaison des deux protéines en doigts de zinc sur leurs séquences respectives rapproche les deux endonucléases qui leur sont associées. Ce rapprochement permet la dimérisation des endonucléases et par la suite la coupure du double brin d'ADN.

Préférentiellement, la nucléase est Cas9.

Par « nucléases associées au système CRISPR », on peut citer à titre non limitatif la nucléase Cas9 dans sa version sauvage (Cas9 WT), mutée pour un de ses deux sites actifs (Cas9N ou Cas9 Nickase) ou mutée pour ses deux sites actifs (dCas9), ainsi que la nucléase Cpf1.

La nucléase Cas9 (CRISPR associated protein 9), est une enzyme spécialisée pour couper l'ADN avec deux zones de coupe actives, une pour chaque brin de la double hélice. Par exemple, S. *pyogenes* utilise l'outil Cas9 pour détecter et défaire l'ADN étranger, tels que l'invasion de l'ADN de bactériophages ou d'un ADN plasmidique.

La nucléase TALEN (Transcription activator-like effector nucleases), est générée par la fusion d'un domaine de liaison à l'ADN, appelé TALE, et d'un domaine enzymatique ayant la capacité de cliver l'ADN, tel que par exemple le domaine catalytique de Fok1, ou des variants de Fok1.

Par « variant de Fok1 », on désigne tout domaine protéique permettant de couper la séquence d'ADN 5'-GGATG(N)₉-3'.

La nucléase du système Zn Finger (également désignée nucléase du système à doigt de zinc) est une enzyme artificielle formée par la fusion d'un domaine de liaison à l'ADN, de type doigt de zinc, et d'un domaine catalytique de coupure à l'ADN, par exemple le domaine catalytique de Fok1, ou des variants de Fok1.

Selon un premier type de particule rétrovirale selon la divulgation, la séquence d'intérêt d'au moins deux ARNs non viraux encapsidés peut comporter une partie codant pour une nucléase.

Ces particules ont un intérêt particulier pour les systèmes d'ingénierie du génome TALEN et ZnFinger dont les nucléases agissent sous forme dimérique. Aussi préférentiellement, la séquence d'intérêt comporte une partie codant pour la nucléase TALEN ou la nucléase à doigt de zinc, encore préférentiellement pour le domaine catalytique de Fok1, ou des variants de Fok1.

Selon l'invention, les au moins deux ARN non viraux encapsidés se distinguent par leur séquence d'intérêt, l'une au moins desdites séquences d'intérêt des ARN non viraux encapsidés comportant une partie codant pour une nucléase choisie parmi le groupe constitué par les nucléases associées au système CRISPR, et la séquence d'intérêt de l'autre ARN non viral encapsidé correspondant à au moins un élément de reconnaissance d'un guide d'ARN.

Plus particulièrement, dans un second type de particule rétrovirale selon la divulgation :
- la séquence d'intérêt d'au moins un ARN comporte une partie codant pour une nucléase et une partie codant pour un système de reconnaissance à l'ADN en 3', et
- la séquence d'intérêt d'au moins un ARN comporte une partie codant pour une nucléase et une partie codant pour un système de reconnaissance à l'ADN en 5'.

La partie codant pour un système de reconnaissance à l'ADN de la séquence d'intérêt correspond avantageusement à un ARN codant pour une protéine capable de reconnaitre une séquence spécifique de l'ADN.

Avantageusement, dans une particule rétrovirale selon ce second type, les séquences d'intérêt comportent une partie codant pour le domaine catalytique de la nucléase Fokl, ou des variants de celui-ci.

Ce domaine catalytique de la nucléase Fokl est avantageusement utilisé pour la construction des nucléase recombinantes TALEN et de la nucléase à doigts de zinc, respectivement pour la mise en œuvre des systèmes d'ingénierie du génome TALEN ou ZnFinger.

A titre d'exemple, la particule rétrovirale selon la divulgation peut comporter deux ARN non viraux encapsidés de séquences d'intérêt différentes :
- une première séquence d'intérêt comportant une partie codant pour la nucléase Fokl et une partie codant pour un TALE 3', et
- une deuxième séquence d'intérêt comportant une partie codant pour la nucléase Fokl et une partie codant pour un TALE 5' ;
ou, la particule rétrovirale selon la divulgation peut comporter deux ARN non viraux encapsidés de séquences d'intérêt différentes :
- une première séquence d'intérêt comportant une partie codant pour la nucléase Fokl et une partie codant pour trois doigts de zinc de reconnaissance d'une séquence d'ADN en 3', et
- une deuxième séquence d'intérêt comportant une partie codant pour la nucléase Fokl et une partie codant pour trois doigts de zinc de reconnaissance d'une séquence d'ADN en 5'.

Selon un mode de réalisation spécifique, dans la particule rétrovirale selon l'invention, la séquence d'intérêt d'au moins un des ARN non viraux encapsidés code pour une nucléase associée au système CRISPR.

Avantageusement, dans ce mode de réalisation spécifique, la nucléase est Cas9.

Plus particulièrement, dans la particule rétrovirale selon ce mode de réalisation spécifique, les au moins deux ARN non viraux encapsidés se distinguent par leur séquence d'intérêt et la séquence d'intérêt de l'autre ARN non viral encapsidé correspond à au moins un élément de reconnaissance d'un guide d'ARN.

Un guide d'ARN correspond à une séquence d'ARN comportant généralement deux éléments de reconnaissance :
- un ARN non codant allant de 3 à 40 bases, capable de s'hybrider par complémentarité de bases à une séquence spécifique de l'ADN et
- un ARN non codant dit « transactivateur » (« scaffold »), qui permet de fixer la nucléase sur l'ADN.

La séquence d'ARN permet de cibler spécifiquement un site spécifique de coupure de l'ADN.

La séquence d'intérêt peut comprendre une ou plusieurs partie(s) correspondant à un ou plusieurs guide(s) d'ARN, identique(s) ou différent(s).

Avantageusement, la séquence d'intérêt du second ARN non viral encapsidé correspond à un guide d'ARN. Le guide d'ARN inclue à la fois la partie de reconnaissance à l'ADN et la partie permettant la fixation d'une nucléase.

Préférentiellement, le guide d'ARN est un sgRNA. Plus préférentiellement encore, le guide d'ARN est un sgRNA chimérique comportant deux répétitions de la séquence d'encapsidation.

Préférentiellement, la particule comporte deux ARN de séquences d'intérêt différentes :
- une première séquence d'intérêt codant pour la nucléase Cas9, et
- une deuxième séquence d'intérêt correspondant à un guide d'ARN.

Avantageusement, la particule rétrovirale selon ce mode de réalisation spécifique comporte en outre au moins un troisième ARN non viral encapsidé ayant une séquence d'intérêt correspondant à un second élément de reconnaissance d'un guide d'ARN ou à un guide d'ARN supplémentaire.

Avantageusement, la particule comporte trois ARN de séquences d'intérêt différentes :
- une première séquence d'intérêt codant pour la nucléase Cas9,
- une deuxième séquence d'intérêt correspondant à un guide d'ARN, et
- une troisième séquence d'intérêt correspondant à un guide d'ARN identique ou différent de la deuxième séquence d'intérêt.

Avantageusement, la particule rétrovirale selon l'invention comporte une protéine de nucléocapside issue de la polyprotéine Gag, une protéine d'enveloppe, optionnellement une intégrase et au moins deux ARN non viraux encapsidés, les ARN non viraux encapsidés comportant chacun une séquence d'ARN d'intérêt liée à une séquence d'encapsidation, chaque séquence d'encapsidation étant reconnue par un domaine de liaison hétérologue introduit dans la protéine de nucléocapside et/ou dans l'intégrase, dans laquelle les au moins deux ARN non viraux encapsidés se distinguent par leur séquence d'intérêt:
- l'une au moins desdites séquences d'intérêt des ARN non viraux encapsidés comportant une partie codant pour une nucléase choisie parmi le groupe constitué par les nucléases associées au système CRISPR, et
- la séquence d'intérêt de l'autre ARN non viral encapsidé correspondant à au moins un élément de reconnaissance d'un guide d'ARN.

Par « protéine de nucléocapside », on entend la protéine de structure NC codée par le gène *gag.* Lorsque le domaine de liaison est introduit dans la protéine de nucléocapside, alors cette protéine est une protéine chimérique, issue d'un gène *gag* chimérique.

Lorsque le domaine de liaison est introduit dans l'intégrase, alors l'intégrase est une protéine chimérique, issue d'un gène *pol* chimérique.

Par « protéine chimérique », on désigne une protéine recombinante comprenant plusieurs séquences protéiques différentes fusionnées.

Selon un premier mode de réalisation, dans la particule rétrovirale selon l'invention, le domaine de liaison est introduit dans la protéine de nucléocapside et les au moins deux ARN non viraux encapsidés se distinguent par leur séquence d'ARN.

Ce premier mode de réalisation permet une expression précoce et transitoire des ARN d'intérêt, sans événement d'intégration associé dans le génome des cellules cibles.

En effet, lors de la mise en contact d'une particule selon ce premier mode de réalisation avec une cellule cible, la membrane de la particule rétrovirale et celle de la cellule cible vont fusionner et permettre la libération du contenu de la particule dans la cellule cible. Les ARN sont alors libérés dans le cytoplasme et la machinerie cellulaire permet de traduire directement ces ARN en protéine(s), c'est-à-dire sans étapes supplémentaires comme la transcription inverse, la translocation dans le noyau ou l'intégration dans le génome de la cellule cible.

Plus particulièrement, les au moins deux ARN non viraux présentent la même séquence d'encapsidation. Dans ce cas, les au moins deux ARN non viraux encapsidés se distinguent par leur séquence d'ARN d'intérêt, c'est-à-dire que les séquences d'ARN d'intérêt comprises dans les deux ARN non viraux encapsidés sont différentes. Par « différentes », on vise des séquences d'intérêt non identiques ou présentant une différence ne résultant pas d'une mutation spontanée ou non intentionnellement choisie par le manipulateur.

Alternativement, les au moins deux ARN non viraux présentent deux séquences d'encapsidation différentes. Ces au moins deux séquences d'encapsidation sont alors reconnues par au moins deux domaines de liaison différents, au moins un de ces domaines étant introduit dans la protéine de nucléocapside. Dans ce cas, selon la divulgation, les au moins deux ARN non viraux encapsidés peuvent comporter des séquences d'intérêt identiques ou différentes.

Il est possible d'encapsider au moins deux ARN non viraux, préférentiellement trois ARN non viraux.

Selon un mode de réalisation particulier du premier mode de réalisation, un second domaine de liaison est introduit dans la protéine de nucléocapside de la particule rétrovirale selon l'invention.

A titre d'exemple, le second domaine de liaison peut être la protéine « Coat » du bactériophage MS2 lorsque le premier domaine de liaison est la protéine « Coat » du phage PP7 ou le second domaine de liaison peut être la protéine « Coat » du phage PP7 lorsque le premier domaine de liaison est la protéine « Coat » du bactériophage MS2.

Dans ce cas, au moins deux ARN non viraux encapsidés portent des séquences d'encapsidation différentes, chaque séquence d'encapsidation correspondant respectivement au premier et second domaine de liaison introduit dans la protéine de nucléocapside.

Plus particulièrement, lorsque trois ARN non viraux sont encapsidés :
- au moins deux des ARN non viraux encapsidés présentent la même séquence d'encapsidation correspondant au premier domaine de liaison et se distinguent uniquement par leur séquence d'ARN d'intérêt,
- le troisième ARN non viral encapsidé peut porter une séquence d'encapsidation identique ou différente. Lorsque la séquence d'encapsidation est différente, celle-ci peut correspondre à un second domaine de liaison introduit dans la protéine de nucléocapside.

D'autres domaines de liaisons peuvent également être introduits dans la protéine de nucléocapside.

Outre le ou les domaine(s) de liaison introduit(s) dans la protéine de nucléocapside, il est également possible d'introduire un domaine de liaison dans l'intégrase.

L'intégrase est alors une protéine chimérique, issue d'un gène *pol* chimérique.

Préférentiellement, lorsque le domaine de liaison est introduit dans l'intégrase, la séquence de l'intégrase est mutée au niveau du domaine C-terminal afin d'insérer la séquence du domaine de liaison. Plus préférentiellement encore, la séquence de l'intégrase est mutée au niveau du domaine C-terminal de manière à introduire celle de la protéine « Coat » du bactériophage MS2 ou du phage PP7.

Dans ce cas, les au moins deux ARN non viraux encapsidés peuvent porter des séquences d'encapsidation différentes, chaque séquence d'encapsidation correspondant aux domaines de liaison introduits dans la protéine de nucléocapside et dans l'intégrase respectivement.

Plus particulièrement, lorsque trois ARN non viraux sont encapsidés :
- au moins deux des ARN non viraux encapsidés présentent la même séquence d'encapsidation correspondant au premier domaine de liaison et se distinguent uniquement par leur séquence d'ARN d'intérêt,
- le troisième ARN non viral encapsidé portant une séquence d'encapsidation différente, correspondant à un second domaine de liaison introduit dans l'intégrase.

D'autres domaines de liaisons peuvent également être introduits dans l'intégrase.

Avantageusement, la particule rétrovirale selon l'invention est une particule lentivirale.

Préférentiellement, dans une telle particule lentivirale :
- le domaine de liaison est la protéine « Coat » du bactériophage MS2,
- la séquence d'encapsidation des ARN non viraux est une séquence tige-boucle de MS2,
- la protéine de nucléocapside est la protéine de nucléocapside (NC) de HIV appartenant à la polyprotéine Gag, chimérique, la séquence de NC étant mutée au niveau du second doigt de zinc afin d'insérer la séquence de la protéine « Coat » du bactériophage MS2.

### Avantageusement :

- La protéine d'enveloppe est la protéine VSV-G codant pour la protéine d'enveloppe du Virus de la stomatite vésiculaire.
- La séquence d'encapsidation comporte de 2 à 25 répétitions de la séquence tige-boucle de MS2, préférentiellement, de 6 à 18 répétitions de la séquence tige-boucle, plus préférentiellement encore de 10 à 14, par exemple 12 répétitions. De préférence, la séquence tige-boucle est la suivante : ctagaaaacatgaggatcacccatgtctgcag (SEQ ID N°1).

Ou, préférentiellement, dans une telle particule lentivirale :
- le domaine de liaison est la protéine « Coat » du phage PP7,
- la séquence d'encapsidation des ARN non viraux est une séquence tige-boucle de PP7,
- la protéine de nucléocapside est la protéine de nucléocapside (NC) de HIV appartenant à la polyprotéine Gag, chimérique, la séquence de NC étant mutée au niveau du second doigt de zinc afin d'insérer la séquence de la protéine « Coat » du phage PP7.

### Avantageusement :

- La protéine d'enveloppe est la protéine VSV-G codant pour la protéine d'enveloppe du Virus de la stomatite vésiculaire.
- La séquence d'encapsidation comporte de 2 à 25 répétitions de la séquence tige-boucle de PP7, préférentiellement, de 2 à 18 répétitions de la séquence tige-boucle, plus préférentiellement encore de 2 à 12, par exemple 6 répétitions. De préférence, la séquence tige-boucle est la suivante : ctagaaaggagcagacgatatggcgtcgctccctgcag (SEQ ID N°2).

Avantageusement, la SEQ ID N°2 peut être optimisée pour favoriser le repliement de la tige boucle. Notamment, il a été trouvé qu'il pouvait être intéressant d'insérer successivement la SEQ ID N°2 et SEQ ID N°4. Cette alternance de la SEQ ID N°2 et de la SEQ ID N°4 (ctagaaaccagcagagcatatgggctcgctggctgcag) pour favoriser le repliement est particulièrement intéressante dans le cas d'un ARN codant. En revanche, pour un ARN non codant, chaque motif tige boucle de PP7 est avantageusement obtenu en insérant successivement la séquence tige-boucle SEQ ID N°5 (ggagcagacgatatggcgtcgctcc) et la séquence tige-boucle SEQ ID N°6 (ccagcagagcatatgggctcgctgg).

Optionnellement, le second domaine de liaison introduit dans l'intégrase peut être la protéine Coat du phage PP7 si le premier domaine de liaison est la protéine Coat du bactériophage MS2, ou le second domaine de liaison introduit dans l'intégrase peut être la protéine Coat du bactériophage MS2 si le premier domaine de liaison est la protéine Coat du phage PP7.

Selon un second mode de réalisation, l'invention concerne une particule rétrovirale comportant une protéine issue de la polyprotéine Gag, préférentiellement une protéine de nucléocapside, une protéine d'enveloppe, une intégrase et au moins deux ARN non viraux encapsidés, les ARN non viraux encapsidés comportant chacun une séquence d'ARN d'intérêt liée à une séquence d'encapsidation, chaque séquence d'encapsidation étant reconnue par un domaine de liaison hétérologue introduit dans l'intégrase et, optionnellement par un domaine de liaison hétérologue introduit dans une protéine issue de la polyprotéine Gag, préférentiellement une protéine de nucléocapside.

Ce second mode de réalisation permet une expression transitoire des ARN d'intérêt, sans événement d'intégration associé dans le génome des cellules cibles.

Préférentiellement, dans ce second mode de réalisation, la séquence de l'intégrase est mutée au niveau du domaine C-terminal afin d'insérer la séquence du domaine de liaison.

Le domaine de liaison est un domaine hétérologue. Plus particulièrement, le domaine de liaison est la protéine Coat du bactériophage MS2, du phage PP7 ou du phage Qβ, la protéine nun du prophage HK022, la protéine U1A ou encore la protéine hPum.

De préférence, la séquence de l'intégrase est mutée au niveau du domaine C-terminal de manière à introduire celle de la protéine « Coat » du bactériophage MS2 ou du phage PP7.

Les au moins deux ARN non viraux peuvent présenter ou non la même séquence d'encapsidation.

De même, selon la divulgation, les au moins deux ARN non viraux encapsidés peuvent présenter ou non la même séquence d'ARN d'intérêt.

Préférentiellement, les au moins deux ARN non viraux encapsidés se distinguent par leur séquence d'ARN d'intérêt c'est-à-dire que les séquences d'ARN d'intérêt comprises dans les deux ARN non viraux encapsidés sont différentes.

Plus particulièrement, les au moins deux ARN non viraux présentent la même séquence d'encapsidation, celle-ci étant reconnue par le domaine de liaison introduit dans l'intégrase.

Il est possible d'encapsider au moins deux ARN non viraux, préférentiellement trois ARN non viraux.

Selon un mode de réalisation particulier du second mode de réalisation, un second domaine de liaison est introduit dans l'intégrase de la particule rétrovirale selon l'invention.

A titre d'exemple, le second domaine de liaison peut être la protéine « Coat » du bactériophage MS2 lorsque le premier domaine de liaison est la protéine « Coat » du phage PP7 ou le second domaine de liaison peut être la protéine « Coat » du phage PP7 lorsque le premier domaine de liaison est la protéine « Coat » du bactériophage MS2.

Dans ce cas, au moins deux ARN non viraux encapsidés portent des séquences d'encapsidations différentes, chaque séquence d'encapsidation correspondant respectivement au premier et second domaine de liaison introduit dans l'intégrase.

Plus particulièrement, lorsque trois ARN non viraux sont encapsidés :
- au moins deux des ARN non viraux encapsidés présentent la même séquence d'encapsidation correspondant au premier domaine de liaison, ces deux ARN non viraux pouvant éventuellement se distinguer par leur séquence d'ARN d'intérêt,
- le troisième ARN non viral encapsidé peut porter une séquence d'encapsidation identique ou différente. Lorsque la séquence d'encapsidation est différente, celle-ci peut correspondre à un second domaine de liaison introduit dans l'intégrase.

D'autres domaines de liaisons peuvent également être introduits dans l'intégrase.

Outre le ou les domaine(s) de liaison introduit(s) dans l'intégrase, il est également possible d'introduire un domaine de liaison dans la protéine de nucléocapside.

La protéine de nucléocapside est alors une protéine chimérique, issue d'un gène *gag* chimérique.

Dans ce cas, les au moins deux ARN non viraux encapsidés peuvent porter des séquences d'encapsidation différentes, chaque séquence d'encapsidation correspondant aux domaines de liaison introduits dans l'intégrase et dans la protéine de nucléocapside respectivement.

Plus particulièrement, lorsque trois ARN non viraux sont encapsidés :
- au moins deux des ARN non viraux encapsidés présentent la même séquence d'encapsidation correspondant au premier domaine de liaison introduit dans l'intégrase, ces ARN non viraux pouvant éventuellement se distinguer par leur séquence d'ARN d'intérêt,
- le troisième ARN non viral encapsidé porte une séquence d'encapsidation différente, correspondant à un second domaine de liaison introduit dans la protéine de nucléocapside.

D'autres domaines de liaisons peuvent également être introduits dans la protéine de nucléocapside.

Avantageusement, dans le cas où la particule selon l'invention comporte une protéine de nucléocapside, le domaine de liaison peut être introduit dans la protéine de nucléocapside, un second domaine de liaison pouvant être introduit dans la nucléocapside et/ou dans l'intégrase.

De préférence, la particule rétrovirale selon l'invention comporte alors une protéine de nucléocapside, une protéine d'enveloppe, optionnellement une intégrase et au moins deux ARN non viraux encapsidés, les ARN non viraux encapsidés comportant chacun une séquence d'ARN d'intérêt liée à une séquence d'encapsidation, au moins une séquence d'encapsidation étant le motif tige-boucle (ou séquence tige-boucle) du bactériophage MS2 répété 12 fois, ce motif étant reconnu par la protéine Coat du bactériophage MS2 introduit dans la protéine de nucléocapside, et dans laquelle les au moins deux ARN non viraux encapsidés se distinguent par leur séquence d'intérêt: l'une au moins desdites séquences d'intérêt des ARN non viraux encapsidés comportant une partie codant pour une nucléase choisie parmi le groupe constitué par les nucléases associées au système CRISPR, et la séquence d'intérêt de l'autre ARN non viral encapsidé correspondant à au moins un élément de reconnaissance d'un guide d'ARN.

Le motif tige-boucle est avantageusement la séquence SEQ ID N°1.

A titre d'exemple, une telle particule selon l'invention est la particule MS2 (NC)-RLP 12X, décrite ci-après dans les Exemples.

Dans cette particule rétrovirale, l'ARN non viral encapsidé comportant comme séquence d'encapsidation le motif tige boucle du bactériophage MS2 répété 12 fois est avantageusement un ARN codant pour Cas 9, et un deuxième ARN non viral encapsidé est un ARN correspondant à au moins un élément de reconnaissance d'un guide d'ARN ou codant pour un guide, ledit ARN non viral encapsidé comportant comme séquence d'encapsidation le motif tige boucle du bactériophage MS2 répété 2 fois.

A titre d'exemple, une telle particule selon l'invention est la particule MS2 (NC)-RLP 12X 2X, décrite ci-après dans les Exemples.

Alternativement ou concomitamment, la particule rétrovirale selon l'invention comporte une protéine de nucléocapside, une protéine d'enveloppe, optionnellement une intégrase et au moins deux ARN non viraux encapsidés, les ARN non viraux encapsidés comportant chacun une séquence d'ARN d'intérêt liée à une séquence d'encapsidation, au moins une séquence d'encapsidation étant le motif tige boucle du bactériophage PP7 répété 2 fois, ce motif étant reconnue par la protéine Coat du bactériophage PP7 introduit dans la protéine de nucléocapside, et dans laquelle les au moins deux ARN non viraux encapsidés se distinguent par leur séquence d'intérêt: l'une au moins desdites séquences d'intérêt des ARN non viraux encapsidés comportant une partie codant pour une nucléase choisie parmi le groupe constitué par les nucléases associées au système CRISPR, et la séquence d'intérêt de l'autre ARN non viral encapsidé correspondant à au moins un élément de reconnaissance d'un guide d'ARN.

Avantageusement, le motif tige-boucle est la séquence SEQ ID N°2. Alternativement, le motif tige-boucle est une alternance de la SEQ ID N°2 et SEQ ID N°4, en particulier pour un ARN codant, ou de la SEQ ID N°5 et de la SEQ ID N°6, en particulier pour un ARN non codant.

A titre d'exemple, lorsque la particule selon l'invention comporte uniquement comme séquence d'encapsidation le motif tige boucle du bactériophage PP7 répété 2 fois, une telle particule selon l'invention est la particule PP7 (NC)-RLP 2X, décrite ci-après dans les Exemples.

A titre d'exemple, lorsque la particule selon l'invention comporte concomitamment comme séquences d'encapsidation le motif tige boucle du bactériophage MS2 répété 12 fois et le motif tige boucle du bactériophage PP7 répété 2 fois, une telle particule selon l'invention est la particule MS2/PP7 (NC)-RLP 12X 2X, décrite ci-après dans les Exemples.

Alternativement, dans la particule rétrovirale selon l'invention, le domaine de liaison peut être introduit dans l'intégrase, un second domaine de liaison pouvant être introduit dans la nucléocapside et/ou dans l'intégrase.

Avantageusement, la particule rétrovirale selon l'invention est une particule lentivirale.

Lorsque la particule rétrovirale est une particule lentivirale, il est possible d'exprimer transitoirement des ARN d'intérêt, sans événement d'intégration associé dans le génome des cellules cibles, en particulier des cellules quiescentes.

En effet, en dehors de son rôle dans la réaction d'intégration elle-même, l'intégrase (IN) participe à différentes étapes du cycle réplicatif des rétrovirus comme la morphogénèse de la particule virale, la transcription inverse et l'import nucléaire du complexe de préintégration (PIC).

Plus particulièrement, chez les lentivirus, l'intégrase contient des séquences de localisation nucléaire (NLS) permettant sa localisation dans le noyau grâce au PIC. Par conséquent, lorsque l'encapsidation des ARN non viraux est effectuée par un domaine de liaison porté par une intégrase d'un lentivirus, les ARN non viraux encapsidés seront transportées dans le noyau de la cellule cible. En effet, lors de la mise en contact d'une particule lentivirale selon ce second mode de réalisation avec une cellule cible, la membrane de la particule et celle de la cellule cible vont fusionner et permettre la libération du contenu de la capside dans la cellule cible. Les ARN sont alors pris en charge par l'intégrase qui, via les PIC, va permettre l'import des ARN dans le noyau. Cette prise en charge est particulièrement intéressante pour certaines applications, telles que l'expression dans des cellules quiescentes. Dans le cas de particules rétrovirales, autres que les lentivirus, l'intégrase ne contient pas ces NLS et se trouve donc localisée dans le cytoplasme. Il est toutefois possible de rajouter dans ce type d'intégrase, les séquences NLS afin d'induire une localisation nucléaire de l'intégrase, et donc des ARN pris en charge par cette intégrase.

Cette prise en charge est également particulièrement utile pour un système CRISPR, qui fait appel à des guides ARN qui viennent s'hybrider spécifiquement au génome de la cellule cible. Une fois hybridés, ces guides ARN guident une endonucléase (Cas9), qui va permettre la modification d'un locus spécifique du génome de la cellule cible. Plus particulièrement, dans une telle particule lentivirale :
- le domaine de liaison est la protéine « Coat » du bactériophage MS2,
- la séquence d'encapsidation des ARN non viraux est une séquence tige-boucle de MS2,
- l'intégrase est une protéine enzymatique chimérique dont la séquence est mutée au niveau du domaine C-terminal afin d'insérer la séquence de la protéine « Coat » du bactériophage MS2.

Ou, plus particulièrement, dans une telle particule lentivirale :
- le domaine de liaison est la protéine « Coat » du phage PP7,
- la séquence d'encapsidation des ARN non viraux est une séquence tige-boucle de PP7,
- l'intégrase est une protéine enzymatique chimérique dont la séquence est mutée au niveau du domaine C-terminal afin d'insérer la séquence de la protéine « Coat » du phage PP7.

Optionnellement, le second domaine de liaison introduit dans la nucléocapside peut être la protéine Coat du phage PP7 si le premier domaine de liaison est la protéine Coat du bactériophage MS2 ou le second domaine de liaison introduit dans l'intégrase peut être la protéine Coat du bactériophage MS2 si le premier domaine de liaison est la protéine Coat du phage PP7.

En effet, l'intégrase (IN) est composée de 3 domaines fonctionnels distincts, chacun indispensable pour assurer une réaction d'intégration complète. Le domaine N-terminal contient un motif de type doigt de zinc qui stabilise la structure repliée de l'IN et augmente l'activité catalytique de l'enzyme. Le domaine central de l'IN contient le motif d'acides aminés DDE auquel est attribuée l'activité catalytique de l'enzyme. Ce domaine central est aussi impliqué dans la reconnaissance de la séquence nucléotidique conservée à chaque extrémité de l'ADN rétroviral. Le domaine C-terminal est le moins conservé parmi la famille des rétrovirus. Il possède l'activité de liaison à l'ADN et est indispensable pour les réactions de maturation des extrémités 3' de transfert de brin. En dehors de son rôle dans la réaction d'intégration elle-même, IN participe à différentes étapes du cycle réplicatif des rétrovirus comme la morphogénèse de la particule virale, la transcription inverse et l'import nucléaire du complexe de préintégration.

Comme décrit par Petit et al. (1999; J. Virol. P5079-5088), l'insertion d'une séquence exogène en C-terminal de l'IN ne perturbe pas les étapes de production et de transduction de cellules cibles alors qu'une même insertion en N-terminal ne permet pas la détection d'un événement de transduction.

La particule rétrovirale a donc été modifiée pour contenir la protéine « Coat » du bacteriophage MS2 en fusion avec la protéine de l'intégrase (voir Figure I) ou la protéine « Coat » du phage PP7 (voir Figure XXXVII). Le plasmide d'encapsidation p8.74, porteur du gène *pol* codant pour la protéine de l'intégrase, est modifié afin d'insérer la séquence codant la protéine Coat en C-terminal de l'intégrase par PCR d'assemblage. Le plasmide p8.74 est linéarisé par PCR puis la séquence Coat, préalablement amplifiée par PCR, est clonée au niveau C-terminal de l'intégrase, soit directement bout-à-bout soit avec l'ajout un linker.

### Avantageusement :

- La protéine d'enveloppe est la protéine VSV-G codant pour la protéine d'enveloppe du Virus de la stomatite vésiculaire.
- La séquence d'encapsidation comporte de 2 à 25 répétitions de la séquence tige-boucle de MS2 et/ou de PP7, selon le domaine de liaison introduit, préférentiellement, de 2 à 18 répétitions, plus préférentiellement de 2 à 18 répétitions, tel que de 6 à 18 répétitions de la séquence tige-boucle, plus préférentiellement encore pour la séquence tige-boucle de MS2, de 10 à 14, par exemple 12 répétitions.
- Préférentiellement, la séquence tige-boucle est la suivante : ctagaaaacatgaggatcacccatgtctgcag (SEQ ID N°1) lorsque le domaine de liaison est la protéine Coat de MS2 et/ou la séquence tige-boucle est la suivante : ctagaaaggagcagacgatatggcgtcgctccctgcag (SEQ ID N°2) lorsque le domaine de liaison est la protéine Coat de PP7.

Plusieurs exemples de particule lentivirale selon l'invention sont décrits ci-après et pour certaines de manière plus détaillée dans les exemples qui suivent :
- une particule MS2RLP ou MS2 (NC)-RLP 12X est une particule lentivirale formée par encapsidation d'ARNs portant le motif tige boucle du bactériophage MS2, répété 12 fois, par l'insertion de la protéine Coat du bactériophage MS2 dans la nucléocapside,
- une particule MS2 (NC)-RLP 2X est une particule lentivirale formée par encapsidation d'ARNs portant le motif tige boucle du bactériophage MS2, répété 2 fois, par l'insertion de la protéine Coat du bactériophage MS2 dans la nucléocapside,
- une particule MS2RLP 12X 2X ou MS2 (NC)-RLP 12X 2X est une particule lentivirale formée par encapsidation d'au moins un premier ARN portant le motif tige boucle du bactériophage MS2, répété 12 fois et d'au moins un second ARN portant le motif tige boucle du bactériophage MS2, répété 2 fois, par l'insertion de la protéine Coat du bactériophage MS2 dans la nucléocapside,
- une particule MS2 (NC)-RLP 6X est une particule lentivirale formée par encapsidation d'ARNs portant le motif tige boucle du bactériophage MS2, répété 6 fois, par l'insertion de la protéine Coat du bactériophage MS2 dans la nucléocapside,
- une particule MS2 (IN)-RLP 2X est une particule lentivirale formée par encapsidation d'ARNs portant le motif tige boucle du bactériophage MS2, répété 2 fois, par l'insertion de la protéine Coat du bactériophage MS2 dans l'intégrase,
- une particule MS2 (IN)-RLP 6X est une particule lentivirale formée par encapsidation d'ARNs portant le motif tige boucle du bactériophage MS2, répété 6 fois, par l'insertion de la protéine Coat du bactériophage MS2 dans l'intégrase,
- une particule MS2 (IN)-RLP 12X est une particule lentivirale formée par encapsidation d'ARNs portant le motif tige boucle du bactériophage MS2, répété 12 fois, par l'insertion de la protéine Coat du bactériophage MS2 dans l'intégrase,
- une particule PP7RLP ou PP7 (NC)-RLP 2X est une particule lentivirale formée par encapsidation d'ARNs portant le motif tige boucle du phage PP7, répété 2 fois, par l'insertion de la protéine Coat du phage PP7 dans la nucléocapside,
- une particule PP7 (NC)-RLP 6X est une particule lentivirale formée par encapsidation d'ARNs portant le motif tige boucle du phage PP7, répété 6 fois, par l'insertion de la protéine Coat du phage PP7 dans la nucléocapside,
- une particule PP7RLP ou PP7 (NC)-RLP 12X est une particule lentivirale formée par encapsidation d'ARNs portant le motif tige boucle du phage PP7, répété 12 fois, par l'insertion de la protéine Coat du phage PP7 dans la nucléocapside,
- une particule PP7 (IN)-RLP 2X est une particule lentivirale formée par encapsidation d'ARNs portant le motif tige boucle du phage PP7, répété 2 fois, par l'insertion de la protéine Coat du phage PP7 dans l'intégrase,
- une particule PP7 (IN)-RLP 6X est une particule lentivirale formée par encapsidation d'ARNs portant le motif tige boucle du phage PP7, répété 6 fois, par l'insertion de la protéine Coat du phage PP7 dans l'intégrase,
- une particule PP7 (IN)-RLP 12X est une particule lentivirale formée par encapsidation d'ARNs portant le motif tige boucle du phage PP7, répété 12 fois, par l'insertion de la protéine Coat du phage PP7 dans l'intégrase.
- une particule MS2/PP7RLP ou MS2/PP7(NC)-RLP 12X 2X est une particule lentivirale formée par encapsidation d'ARNs portant le motif tige boucle du bactériophage MS2, répété 12 fois, par l'insertion de la protéine Coat du bactériophage MS2 dans la nucléocapside, et portant le motif tige boucle du phage PP7, répété 2 fois, par l'insertion de la protéine Coat du phage PP7 dans la nucléocapside.

Préférentiellement, la particule lentivirale formée par encapsidation d'ARNs porte un motif tige boucle du bactériophage MS2 ou du phage PP7, répété de 2 à 25 fois, préférentiellement encore 2, 6 ou 12 fois.

Plus préférentiellement encore, la particule selon l'invention est choisie parmi MS2 (NC)-RLP 12X, MS2 (NC)-RLP 12X 2X, PP7 (NC)-RLP 6X, PP7 (NC)-RLP 2X, MS2 (IN)-RLP 12X, PP7 (IN)-RLP 6X et PP7 (IN)-RLP 2X, MS2/PP7 (NC)-RLP 12X 2X.

L'invention concerne également des compositions comportant des particules selon l'invention.

Plus particulièrement, les compositions selon l'invention sont des compositions concentrées. Avantageusement, les compositions sont également purifiées. Ces compositions peuvent être concentrées et purifiées selon le procédé décrit dans la demande WO2013/014537.

Typiquement, les compositions selon l'invention comportent moins de 30 % de contaminants ADN et moins de 45% de contaminants protéiques par rapport au surnageant brut. Plus particulièrement, les compositions selon l'invention comportent moins de 30 % de contaminants ADN et moins de 2% de contaminants protéiques par rapport au surnageant brut.

Par « surnageant brut », on vise le surnageant de culture(s) cellulaire(s), comportant des particules rétrovirales selon l'invention, après clarification. Une telle étape de clarification est plus particulièrement décrite ci-après dans les procédés de fabrication des particules selon l'invention. Lorsque la récupération du surnageant est effectué en plusieurs fois, le surnageant brut correspond alors à l'ensemble des surnageants collectés, réunis (ou « poolés ») puis clarifié.

Optionnellement, les compositions selon l'invention comportent moins de 1 % de contaminants ADN et moins de 1% de contaminants protéiques par rapport au surnageant brut.

L'invention se rapporte aussi à des kits de production de particules selon l'invention et aux procédés de fabrication de ces kits.

Plus particulièrement, la divulgation concerne un kit de production de particules selon l'invention, comportant :
(i) un plasmide d'expression comportant au moins une séquence d'intérêt, pour laquelle en amont, en aval ou au sein de cette séquence est insérée une séquence d'encapsidation,
(ii) un plasmide d'encapsidation codant pour une protéine issue de la polyprotéine Gag et/ou une intégrase, chimérique, comportant un domaine de liaison permettant de reconnaître une séquence d'encapsidation, et,
(iii) un plasmide d'enveloppe codant pour une protéine d'enveloppe.

Plus particulièrement, l'invention concerne un kit de production de particules selon l'invention, comportant :
(i) un plasmide d'expression comportant au moins deux séquences d'ARN non virales différentes, chaque séquence d'ARN comportant une séquence d'intérêt pour laquelle en amont, en aval ou au sein de cette séquence est insérée une séquence d'encapsidation, ou alternativement, un premier et un second plasmide d'expression comportant chacun une séquence d'intérêt en amont ou en aval de laquelle est insérée une séquence d'encapsidation, au moins une des séquences d'intérêt codant pour une nucléase choisie parmi le groupe constitué par les nucléases associées au système CRISPR, l'autre séquence d'intérêt correspondant à au moins un élément de reconnaissance d'un guide d'ARN,
(ii) un plasmide d'encapsidation codant pour une protéine issue de la polyprotéine Gag et/ou une intégrase, chimérique, comportant un domaine de liaison hétérologue permettant de reconnaître une séquence d'encapsidation, et,
(iii) un plasmide d'enveloppe codant pour une protéine d'enveloppe.

Un tel kit peut également comporter une notice d'utilisation des plasmides contenus dans le kit.

Plus spécifiquement, lorsque le kit vise à produire des particules selon le premier mode de réalisation, ce kit comporte :
(i) un plasmide d'expression comportant au moins deux séquences d'ARN non virales différentes, chaque séquence d'ARN comportant une séquence d'intérêt pour laquelle en amont, en aval ou au sein de cette séquence d'intérêt est insérée une séquence d'encapsidation, ou alternativement un premier et un second plasmide d'expression comportant chacun une séquence d'intérêt en amont ou en aval de laquelle est insérée une séquence d'encapsidation, au moins une des séquences d'intérêt codant pour une nucléase choisie parmi le groupe constitué par les nucléases associées au système CRISPR, l'autre séquence d'intérêt correspondant à au moins un élément de reconnaissance d'un guide d'ARN,
(ii) un plasmide d'encapsidation codant pour une protéine de nucléocapside chimérique comportant un domaine de liaison hétérologue permettant de reconnaître la séquence d'encapsidation, et,
(iii) un plasmide d'enveloppe codant pour une protéine d'enveloppe.

Les au moins deux séquences d'ARN non virales sont différentes parce que les séquences d'intérêt sont différentes et optionnellement les séquences d'encapsidation sont différentes.

Un plasmide étant une structure d'ADN, il est bien entendu que par « plasmide d'expression comportant au moins deux séquences d'ARN non virales », on vise un plasmide d'expression codant pour au moins deux séquences d'ARN non virales.

En effet, les plasmides d'expression des kits contiennent toutes les séquences d'ADN nécessaires à l'obtention d'au moins un ARN non viral par transcription. Le plasmide d'expression contient au moins un promoteur, suivi d'une séquence ADN d'intérêt (ADNc ou ADN qui sera transcrit en un ARN non viral) et une séquence d'encapsidation (un ADN codant par exemple pour des motifs répétés MS2 ou PP7) et optionnellement une séquence stabilisatrice de l'ARN.

Préférentiellement, le kit produisant des particules selon le premier mode de réalisation comporte :
(i) un plasmide d'expression comportant au moins deux séquences d'ARN non virales différentes, chaque séquence d'ARN comportant une séquence d'intérêt, pour laquelle en amont, en aval ou au sein de chacune de cette séquence est insérée une séquence d'encapsidation, ou alternativement un premier et un second plasmide d'expression comportant chacun une séquence d'intérêt en amont ou en aval de laquelle est insérée une séquence d'encapsidation, au moins une des séquences d'intérêt codant pour une nucléase choisie parmi le groupe constitué par les nucléases associées au système CRISPR, l'autre séquence d'intérêt correspondant à au moins un élément de reconnaissance d'un guide d'ARN,
   les séquences d'intérêt étant différentes et les séquences d'encapsidation étant identiques,
(ii) un plasmide d'encapsidation codant pour une protéine de nucléocapside chimérique comportant un domaine de liaison hétérologue permettant de reconnaître la séquence d'encapsidation, et,
(iii) un plasmide d'enveloppe codant pour une protéine d'enveloppe. Avantageusement, le kit produit des particules lentivirales. Préférentiellement :
   - Dans le plasmide d'expression, la séquence d'encapsidation comporte de 2 à 25 répétitions de la séquence tige-boucle de MS2, préférentiellement de 6 à 18 répétitions de la séquence tige-boucle, plus préférentiellement encore de 10 à 14, par exemple 12 répétitions. Avantageusement, la séquence tige-boucle est la suivante : ctagaaaacatgaggatcacccatgtctgcag (SEQ ID N°1).
   - Dans le plasmide d'encapsidation, la protéine de nucléocapside est la protéine de nucléocapside (NC) de HIV appartenant à la polyprotéine Gag, laquelle NC est mutée au niveau du second doigt de zinc afin d'insérer la séquence de la protéine « Coat » du bactériophage MS2.
   - Dans le plasmide d'enveloppe, la protéine d'enveloppe est la protéine VSV-G codant pour la protéine d'enveloppe du Virus de la stomatite vésiculaire.

Ou, préférentiellement :
- Dans le plasmide d'expression, la séquence d'encapsidation comporte de 2 à 25 répétitions de la séquence tige-boucle de PP7, préférentiellement, de 2 à 18 répétitions de la séquence tige-boucle, plus préférentiellement encore de 2 à 12, par exemple 6 répétitions. Avantageusement, la séquence tige-boucle est la suivante : ctagaaaggagcagacgatatggcgtcgctccctgcag (SEQ ID N°2). Alternativement, la séquence tige-boucle est une alternance de la SEQ ID N°2 et SEQ ID N°4, en particulier pour un ARN codant, ou de la SEQ ID N°5 et de la SEQ ID N°6, en particulier pour un ARN non codant.
- Dans le plasmide d'encapsidation, la protéine de nucléocapside est la protéine de nucléocapside (NC) de HIV appartenant à la polyprotéine Gag, laquelle NC est mutée au niveau du second doigt de zinc afin d'insérer la séquence de la protéine « Coat » du phage PP7.
- Dans le plasmide d'enveloppe, la protéine d'enveloppe est la protéine VSV-G codant pour la protéine d'enveloppe du Virus de la stomatite vésiculaire.

Optionnellement, le kit comporte un second plasmide d'encapsidation codant pour une intégrase chimérique comportant un domaine de liaison permettant de reconnaître une séquence d'encapsidation. Le second domaine de liaison peut être identique ou différent du domaine de liaison de la protéine de nucléocapside chimérique.

A titre d'exemple de domaines de liaison différents :
- le domaine de liaison introduit dans la nucléocapside peut être la protéine Coat de MS2 et le domaine de liaison introduit dans l'intégrase peut être la protéine Coat de PP7, ou
- le domaine de liaison introduit dans la nucléocapside peut être la protéine Coat de PP7 et le domaine de liaison introduit dans l'intégrase peut être la protéine Coat de MS2.

Plusieurs domaines de liaison peuvent être introduits dans chacune des protéines chimériques.

Alternativement, lorsque le kit vise à produire des particules selon le second mode de réalisation, ce kit comporte :
(i) un plasmide d'expression comportant au moins deux séquences d'ARN non virales différentes, chaque séquence d'ARN comportant une séquence d'intérêt pour laquelle en amont, en aval ou au sein de cette séquence est insérée une séquence d'encapsidation, ou alternativement, un premier et un second plasmide d'expression comportant chacun une séquence d'intérêt en amont ou en aval de laquelle est insérée une séquence d'encapsidation, au moins une des séquences d'intérêt codant pour une nucléase choisie parmi le groupe constitué par les nucléases associées au système CRISPR, l'autre séquence d'intérêt correspondant à au moins un élément de reconnaissance d'un guide d'ARN,
(ii) un plasmide d'encapsidation codant pour une intégrase chimérique comportant un domaine de liaison hétérologue permettant de reconnaître la séquence d'encapsidation, et,
(iii) un plasmide d'enveloppe codant pour une protéine d'enveloppe.

Avantageusement, les promoteurs mis en œuvre dans les plasmides d'expression des kits selon l'invention sont EF1, notamment lorsque la séquence d'intérêt est une nucléase, telle que Cas 9, et U6, notamment lorsque la séquence d'intérêt est au moins un élément de reconnaissance d'un guide d'ARN ou un guide d'ARN.

Avantageusement, les kits de production selon l'invention, peuvent comporter en outre un second plasmide d'encapsidation codant pour :
- une protéine issue de la polyprotéine Gag sauvage, lorsque le premier plasmide d'encapsidation code pour une protéine issue de la polyprotéine Gag chimérique, et/ou
- une intégrase sauvage, lorsque le premier plasmide d'encapsidation code pour une intégrase chimérique.

Des procédés de fabrication des kits selon l'invention sont également proposés. Typiquement, un procédé de fabrication d'un kit selon l'invention comporte :
(i) la préparation d'un plasmide d'expression comportant au moins deux séquences d'ARN non virales différentes, chaque séquence d'ARN comportant une séquence d'intérêt pour laquelle en amont, en aval ou au sein de cette séquence est insérée une séquence d'encapsidation, ou alternativement, un premier et un second plasmide d'expression comportant chacun une séquence d'intérêt en amont ou en aval de laquelle est insérée une séquence d'encapsidation, au moins une des séquences d'intérêt codant pour une nucléase choisie parmi le groupe constitué par les nucléases associées au système CRISPR, l'autre séquence d'intérêt correspondant à au moins un élément de reconnaissance d'un guide d'ARN,
(ii) la préparation d'un plasmide d'encapsidation codant pour une protéine issue de la polyprotéine Gag et/ou une intégrase, chimérique, comportant un domaine de liaison hétérologue permettant de reconnaître une séquence d'encapsidation, et
(iii) la préparation d'un plasmide d'enveloppe codant pour une protéine d'enveloppe.

Plus spécifiquement, lorsque le procédé concerne un kit qui vise à produire des particules selon le premier mode de réalisation, il comporte :
(i) la préparation d'un plasmide d'expression comportant au moins deux séquences d'ARN non virales différentes, chaque séquence d'ARN comportant une séquence d'intérêt pour laquelle en amont, en aval ou au sein de cette séquence d'intérêt est insérée une séquence d'encapsidation, ou alternativement un premier et un second plasmide d'expression comportant chacun une séquence d'intérêt en amont ou en aval de laquelle est insérée une séquence d'encapsidation, au moins une des séquences d'intérêt codant pour une nucléase choisie parmi le groupe constitué par les nucléases associées au système CRISPR, l'autre séquence d'intérêt correspondant à au moins un élément de reconnaissance d'un guide d'ARN,
(ii) la préparation d'un plasmide d'encapsidation codant pour une protéine de nucléocapside chimérique comportant un domaine de liaison hétérologue permettant de reconnaître la séquence d'encapsidation, et,
(iii) la préparation d'un plasmide d'enveloppe codant pour une protéine d'enveloppe.

Les au moins deux séquences d'ARN non virales sont différentes parce que les séquences d'intérêt sont différentes et optionnellement les séquences d'encapsidation sont différentes.

Préférentiellement, ce procédé comporte :
(i) la préparation d'un plasmide d'expression comportant au moins deux séquences d'intérêt, pour lesquelles en amont, en aval ou au sein de chacune de ces séquences est insérée une séquence d'encapsidation, ou alternativement d'un premier et d'un second plasmide d'expression comportant chacun une séquence d'intérêt en amont ou en aval de laquelle est insérée une séquence d'encapsidation, au moins une des séquences d'intérêt codant pour une nucléase choisie parmi le groupe constitué par les nucléases associées au système CRISPR, l'autre séquence d'intérêt correspondant à au moins un élément de reconnaissance d'un guide d'ARN,
   les séquences d'intérêt étant différentes et les séquences d'encapsidation étant identiques,
(ii) la préparation d'un plasmide d'encapsidation codant pour une protéine de nucléocapside chimérique comportant un domaine de liaison hétérologue permettant de reconnaître la séquence d'encapsidation, et
(iii) la préparation d'un plasmide d'enveloppe codant pour une protéine d'enveloppe.

Alternativement, lorsque le procédé concerne un kit qui vise à produire des particules selon le second mode de réalisation, ce procédé comporte :
(i) la préparation d'un plasmide d'expression comportant au moins deux séquences d'ARN non virales différentes, chaque séquence d'ARN comportant une séquence d'intérêt pour laquelle en amont, en aval ou au sein de cette séquence est insérée une séquence d'encapsidation, ou alternativement, un premier et un second plasmide d'expression comportant chacun une séquence d'intérêt en amont ou en aval de laquelle est insérée une séquence d'encapsidation, au moins une des séquences d'intérêt codant pour une nucléase choisie parmi le groupe constitué par les nucléases associées au système CRISPR, l'autre séquence d'intérêt correspondant à au moins un élément de reconnaissance d'un guide d'ARN,
(ii) la préparation d'un plasmide d'encapsidation codant pour une intégrase chimérique comportant un domaine de liaison hétérologue permettant de reconnaître la séquence d'encapsidation, et,
(iii) la préparation d'un plasmide d'enveloppe codant pour une protéine d'enveloppe.

L'invention se rapporte également à des procédés de fabrication des particules selon l'invention.

Un tel procédé comporte une étape de co-transfection de cellules avec :
(i) un plasmide d'expression comportant au moins deux séquences d'ARN non virales différentes, chaque séquence d'ARN comportant une séquence d'intérêt pour laquelle en amont, en aval ou au sein de cette séquence est insérée une séquence d'encapsidation, ou alternativement, un premier et un second plasmide d'expression comportant chacun une séquence d'intérêt en amont ou en aval de laquelle est insérée une séquence d'encapsidation, au moins une des séquences d'intérêt codant pour une nucléase choisie parmi le groupe constitué par les nucléases associées au système CRISPR, l'autre séquence d'intérêt correspondant à au moins un élément de reconnaissance d'un guide d'ARN,
(ii) un plasmide d'encapsidation codant pour une protéine issue de la polyprotéine Gag et/ou une intégrase chimérique comportant un domaine de liaison hétérologue permettant de reconnaître une séquence d'encapsidation, et,
(iii) un plasmide d'enveloppe codant pour une protéine d'enveloppe. et la récupération du surnageant des cellules transfectées comportant les particules.

Les cellules mises en œuvre dans les procédés de fabrication de particules selon l'invention sont des cellules productrices, c'est-à-dire des cellules, qui une fois transfectées avec les plasmides portant le matériel génétique nécessaire à la formation des particules rétrovirales, permettent la formation desdites particules. A titre d'exemple de cellules productrices, on peut citer HEK293T.

Par « étape de co-transfection », on entend une étape de transfection lors de laquelle la transfection est effectuée par mise en contact des cellules productrices avec l'ensemble des plasmides du procédé de fabrication des particules.

Plus spécifiquement, lorsque le procédé de fabrication vise à produire des particules selon le premier mode de réalisation, il comporte une étape de co-transfection de cellules avec :
(i) un plasmide d'expression comportant au moins deux séquences d'ARN non virales différentes, chaque séquence d'ARN comportant une séquence d'intérêt pour laquelle en amont, en aval ou au sein de cette séquence d'intérêt est insérée une séquence d'encapsidation, ou alternativement un premier et un second plasmide d'expression comportant chacun une séquence d'intérêt en amont ou en aval de laquelle est insérée une séquence d'encapsidation, au moins une des séquences d'intérêt codant pour une nucléase choisie parmi le groupe constitué par les nucléases associées au système CRISPR, l'autre séquence d'intérêt correspondant à au moins un élément de reconnaissance d'un guide d'ARN,
(ii) un plasmide d'encapsidation codant pour une protéine de nucléocapside chimérique comportant un domaine de liaison hétérologue permettant de reconnaître la séquence d'encapsidation, et,
(iii) un plasmide d'enveloppe codant pour une protéine d'enveloppe, et la récupération du surnageant des cellules transfectées comportant les particules.

Les au moins deux séquences d'ARN non virales sont différentes parce que les séquences d'intérêt sont différentes et optionnellemnt les séquences d'encapsidation sont différentes.

Préférentiellement, ce procédé de fabrication de particules comporte une étape de co-transfection de cellules avec :
(i) un plasmide d'expression comportant au moins deux séquences d'intérêt, pour lesquelles en amont, en aval ou au sein de chacune de ces séquences est insérée une séquence d'encapsidation, ou alternativement un premier et un second plasmide d'expression comportant chacun une séquence d'intérêt en amont ou en aval de laquelle est insérée une séquence d'encapsidation, au moins une des séquences d'intérêt codant pour une nucléase choisie parmi le groupe constitué par les nucléases associées au système CRISPR, l'autre séquence d'intérêt correspondant à au moins un élément de reconnaissance d'un guide d'ARN,
   les séquences d'intérêt étant différentes et les séquences d'encapsidation étant identiques,
(ii) un plasmide d'encapsidation codant pour une protéine de nucléocapside chimérique comportant un domaine de liaison hétérologue permettant de reconnaître la séquence d'encapsidation, et,
(iii) un plasmide d'enveloppe codant pour une protéine d'enveloppe, et la récupération du surnageant des cellules transfectées comportant les particules.

Alternativement, lorsque le procédé de fabrication vise à produire des particules selon le second mode de réalisation, ce procédé comporte une étape de co-transfection de cellules avec :
(i) un plasmide d'expression comportant au moins deux séquences d'ARN non virales différentes, chaque séquence d'ARN comportant une séquence d'intérêt pour laquelle en amont, en aval ou au sein de cette séquence est insérée une séquence d'encapsidation, ou alternativement, un premier et un second plasmide d'expression comportant chacun une séquence d'intérêt en amont ou en aval de laquelle est insérée une séquence d'encapsidation, au moins une des séquences d'intérêt codant pour une nucléase choisie parmi le groupe constitué par les nucléases associées au système CRISPR, l'autre séquence d'intérêt correspondant à au moins un élément de reconnaissance d'un guide d'ARN,
(ii) un plasmide d'encapsidation codant pour une intégrase chimérique comportant un domaine de liaison hétérologue permettant de reconnaître la séquence d'encapsidation, et,
(iii) un plasmide d'enveloppe codant pour une protéine d'enveloppe, et la récupération du surnageant des cellules transfectées comportant les particules.

Avantageusement, dans le procédé de fabrication selon l'invention, les plasmides d'expression représentent au moins 50% en nombre de la totalité des plasmides mis en œuvre dans la co-transfection.

Dans le procédé de fabrication selon l'invention, l'étape de co-transfection peut en outre être réalisée avec un second plasmide d'encapsidation codant pour :
- une protéine issue de la polyprotéine Gag sauvage, lorsque le premier plasmide d'encapsidation code pour une protéine issue de la polyprotéine Gag chimérique, et/ou
- une intégrase sauvage, lorsque le premier plasmide d'encapsidation code pour une intégrase chimérique.

Avantageusement, le ratio du second plasmide d'encapsidation sur le premier plasmide d'encapsidation est dans la gamme allant de [10:90] à [60 :40], préférentiellement, dans la gamme allant de [20 :80] à [50 :50].

Des avantages liés à l'utilisation d'un second plasmide d'encapsidation et plus particulièrement aux ratios définis ci-avant sont plus amplement décrits à l'Exemple 13.

Préférentiellement, tous les procédés de fabrication de particules selon l'invention sont réalisés conformément aux procédés décrits dans la demande WO2013/014537.

Plus particulièrement, ces procédés de fabrication de particules sont réalisés sur des cellules productrices cultivées en milieu sans sérum et aucune induction au sodium butyrate n'est effectuée.

Avantageusement, le surnageant est collecté en plusieurs fois, par exemple entre 3 et 6 fois, à des intervalles de temps spécifiques, tels qu'à des intervalles de temps de l'ordre de la demi vie des particules rétrovirales. Typiquement, la récupération du surnageant est effectuée en 4 à 5 fois, à des intervalles de temps de l'ordre de 6 à 18h, préférentiellement de 8 à 16h, tels que 8h, 12h et/ou 16h. Préférentiellement, cette collecte est effectuée après changement du milieu de culture des cellules, ce changement étant effectué préférentiellement à 24h post-transfection.

Les procédés de fabrication de particules selon l'invention comportent également une étape dans laquelle le surnageant est clarifié.

Préférentiellement, la clarification du surnageant est effectuée par centrifugation.

Plus préférentiellement encore, ces procédés de fabrication de particules selon l'invention comportent en outre une étape dans laquelle le surnageant est concentré et/ou purifié.

De préférence, la concentration et purification est effectuée par ultrafiltration frontale sur des unités de centrifugation.

Avantageusement, le surnageant subit une ou plusieurs étapes supplémentaires de purification. Ces étapes de purification sont effectuées de préférence par ultrafiltration tangentielle et/ou diafiltration. Plus préférentiellement encore, le surnageant subit une étape d'ultrafiltration tangentielle suivie d'une étape de diafiltration.

L'ultrafiltration tangentielle est avantageusement effectuée sur des cartouches de fibres creuses en polysulfone.

Optionnellement, la composition peut ensuite subir une étape de chromatographie par échange d'ions, en particulier une chromatographie par échange d'anions. L'éluât issu de cette étape de chromatographie est ensuite récupéré puis concentré à nouveau par ultrafiltration frontale sur des unités centrales de centrifugation. Une composition résultant d'un tel procédé comporte moins de 1 % de contaminants ADN et moins de 1% de contaminants protéiques par rapport au surnageant brut.

La divulgation concerne également des compositions susceptibles d'être obtenues par l'un quelconque des procédés de fabrication de particules selon l'invention.

Typiquement, ces compositions comportent moins de 30 % de contaminants ADN et moins de 45% de contaminants protéiques par rapport au surnageant brut. Plus particulièrement, les compositions selon l'invention comportent moins de 30 % de contaminants ADN et moins de 2% de contaminants protéiques par rapport au surnageant brut.

Optionnellement, les compositions selon l'invention comportent moins de 1 % de contaminants ADN et moins de 1% de contaminants protéiques par rapport au surnageant brut.

Enfin, l'invention concerne l'utilisation d'une particule selon l'invention, ou d'une composition selon l'invention pour transduire des cellules.

L'utilisation des particules et des compositions selon l'invention est particulièrement avantageuse pour transduire des cellules primaires et des lignées immortalisées, afin de les modifier de façon transitoire. Les cellules peuvent être des cellules de mammifères ou d'autres eucaryotes. En particulier, la transduction de ces cellules peut être effectuée *in vivo, in vitro* ou *ex vivo.*

Les cellules peuvent recevoir une à deux transductions successives avec les particules selon l'invention.

L'utilisation des particules selon l'invention pour transduire des cellules permet d'introduire une mutation dans le génome grâce à leur capacité à induire des cassures du double brin d'ADN, auxquelles les cellules répondent par des mécanismes de réparation de l'ADN. Par exemple, la réparation peut être effectuée par insertion d'extrémités non-homologues permettant de reconnecter deux extrémités d'ADN résultant d'une cassure double brin, d'ARN introduits à cet effet ou disponible dans la cellule.

Ces systèmes d'ingénierie du génome peuvent également être utilisés pour réaliser des KO de gène, c'est-à-dire une invalidation de l'expression d'un gène, voire induire une mort cellulaire selon les gènes ciblés.

Les particules selon l'invention introduites dans les cellules, elles peuvent alors être utilisées pour modifier le génome *in vivo, in vitro* ou *ex vivo.*

L'invention sera mieux comprise au vu des exemples qui suivent donnés à titre illustratif, avec référence aux figures, qui représentent respectivement :
- La figure I est un schéma illustrant la cassette d'expression issue du plasmide d'expression pcDNA-EF1-Cas9-MS2 12X comportant une séquence d'ADN codante pour l'ARN de la nucléase Cas9, dans sa forme sauvage (WT) ou dans sa forme mutée (N) ;
- La figure IIa illustre la stratégie de modification du plasmide d'encapsidation p8.74 porteur des gènes codant les protéines de structure et de fonction (Gag, Pol) pour la construction d'un p8.74ΔZF-MS2-Coat ;
- La figure IIb est un schéma de construction de la cassette d'expression issue du plasmide d'encapsidation p8.74ΔZF-MS2-Coat ayant le second doigt de zinc substitué par la protéine « Coat » du bactériophage MS2, obtenu par la stratégie présentée en Figure IIa ;
- La figure III est un schéma de construction de la cassette d'expression issue du plasmide d'enveloppe pENV ;
- La figure IV est un schéma de construction de la cassette d'expression issue du plasmide d'expression pILV-EF1-Cas9-WPRE comportant un ADN codant pour la séquence d'ARN de la nucléase Cas9, dans sa forme sauvage (WT) ou dans sa forme mutée (N) ;
- La figure Va est un schéma de construction de la cassette d'expression issue du plasmide d'expression pILV-EF1-GFP-WPRE comportant un ADN codant pour l'ARN de la GFP ;
- La figure Vb est un schéma de construction de la cassette d'expression issue du plasmide d'expression pILV-H1-GuideU3-U6-GuideD1-WPRE comportant un ADN codant pour deux ARN non codant ciblant la séquence de la GFP (sgRNA = ARN guide = Guide U3 et Guide D1 dans ce cas) ;
- La figure Vc est un schéma de construction de la cassette d'expression issue du plasmide d'expression pILV-H1/U6-Guide-WPRE comportant un ADN codant pour un ARN non codant ciblant la séquence de la GFP (sgRNA = ARN guide = Guide) ;
- La figure VI est un schéma de construction de la cassette d'expression issue du plasmide d'encapsidation p8.74 porteur des gènes codant les protéines de structure et de fonction (Gag, Pol) ;
- La figure VII est un schéma illustrant la cassette d'expression issue du plasmide d'expression pcDNA-H1-GuideD1-MS2 2X porteur d'une séquence d'ADN codant pour un ARN guide non codant, comprenant le scaffold, ciblant la séquence de la GFP (sgRNA = ARN guide), dans lequel 2 répétitions du motif tige-boucle de l'ARN MS2 (SEQ ID N°3) ont été insérées au sein de la cassette d'expression en aval de l'ARN guide ;
- La figure VIII est un schéma illustrant la cassette d'expression issue du plasmide d'expression pcDNA-H1-GuideD1Chimérique-MS2 2X porteur d'une séquence d'ADN codant pour un ARN guide non codant ciblant la séquence de la GFP (sgRNA = ARN guide), d'une cassette d'expression promoteur-séquence d'intérêt-Term, dans lequel 2 répétitions du motif tige-boucle de l'ARN MS2 (SEQ ID N°3) sont incluses dans la partie « scaffold » du guide chimérique ;
- La figure IX illustre l'efficacité d'une particule MS2 (NC)-RLP 12X selon l'invention à délivrer l'ARN codant pour la nucléase Cas9 Nickase lors de la transduction de cellules HCT119-GFP ;
- La figure X illustre l'impact de la position des motifs répétés MS2 pour l'encapsidation d'ARN guides dans de particules MS2 (NC)-RLP 12X selon l'invention, après transduction des cellules HCT119-GFP Cas9 ;
- La figure XI illustre l'efficacité de production de particules MS2RLP délivrant de l'ARN guide selon qu'elles sont produites par l'utilisation d'une quantité simple ou double du plasmide d'expression portant l'ARN guide pour la transduction des cellules HCT116-GFP-Cas9 ;
- La figure XII illustre l'efficacité d'une particule MS2 (NC)-RLP 12X selon l'invention à délivrer le système CRISPR/Cas9 (ARN guide + ARN codant pour la Cas 9), dans des cellules HCT116-GFP ;
- La figure XIIIa est un schéma illustrant la cassette d'expression issue du plasmide d'expression pcDNA.EF1.PPRV.TALEN 5'.WPRE MS2(12X) comportant une séquence d'ADN codant pour l'ARN de la nucléase TALEN constituée de la nucléase Fokl fusionnée à un domaine de liaison à l'ADN, TALE, reconnaissant le brin 5' de l'ADN ;
- La figure XIIIb est un schéma illustrant la cassette d'expression issue du plasmide d'expression pcDNA.EF1.PPRV.TALEN 3'.WPRE MS2(12X) comportant une séquence d'ADN codant pour l'ARN de la nucléase TALEN constituée de la nucléase Fokl fusionnée à un domaine de liaison à l'ADN, TALE, reconnaissant le brin 3' de l'ADN ;
- La figure XIVa est un schéma illustrant la cassette d'expression issue du plasmide d'expression pILV.EF1.TALEN 5' comportant une séquence d'ADN codant pour l'ARN de la nucléase TALEN constituée de la nucléase Fokl fusionnée à un domaine de liaison à l'ADN, TALE, reconnaissant le brin 5' de l'ADN ;
- La figure XIVb est un schéma illustrant la cassette d'expression issue du plasmide d'expression pILV.EF1.TALEN 3' comportant une séquence d'ADN codant pour l'ARN de la nucléase TALEN constituée de la nucléase Fokl fusionnée à un domaine de liaison à l'ADN, TALE, reconnaissant le brin 3' de l'ADN ;
- La figure XVa est un schéma illustrant la cassette d'expression issue du plasmide d'expression, pcDNA.EF1.PPRV.ZFP 5'.WPRE.MS2 (12X), porteur d'un domaine de reconnaissance à l'ADN de type doigt de zinc chimérique à la nucléase Fokl pour la reconnaissance du brin d'ADN 5' ;
- La figure XVb est un schéma illustrant la cassette d'expression issue du plasmide d'expression pcDNA.EF1.PPRV.ZFP 3'.WPRE.MS2 (12X), porteur d'un domaine de reconnaissance à l'ADN de type doigt de zinc chimérique à la nucléase Fokl pour la reconnaissance du brin d'ADN 3' ;
- La figure XVIa est un schéma de construction de la cassette d'expression issue du plasmide d'expression pILV.EF1.ZFP 5' porteur d'un domaine de reconnaissance à l'ADN de type doigt de zinc chimérique à la nucléase Fokl pour la reconnaissance du brin d'ADN 5';
- La figure XVIb est un schéma de construction de la cassette d'expression issue du plasmide d'expression pILV.EF1.ZFP 3' porteur d'un domaine de reconnaissance à l'ADN de type doigt de zinc chimérique à la nucléase Fokl pour la reconnaissance du brin d'ADN 3';
- La figure XVII présente le schéma de la cassette d'expression issue du plasmide d'expression portant comme séquence d'ARN d'intérêt la Luciférase utilisé pour la production de particules lentivirales PP7(NC)-RLP 12X, comprenant le motif tige-boucle PP7 répété 12 fois, selon l'invention ;
- La figure XVIII présente un schéma de la modification du plasmide d'encapsidation lentiviral p8.74 afin d'insérer un domaine de liaison dans la séquence de l'Intégrase ;
- La figure XIX présente un schéma de la cassette d'expression issue du plasmide d'encapsidation utilisé pour la production de particules lentivirales PP7 (IN)-RLP selon l'invention, obtenu par la modification du plasmide d'encapsidation lentiviral p8.74 présenté Figure VI ;
- La figure XX illustre l'efficacité d'une particule MS2 (NC)-RLP 12X selon l'invention à délivrer le système CRISPR/Cas9 (ARN guide + ARN codant pour la Cas 9), dans des cellules HCT116-GFP ;
- La figure XXI est un schéma de construction de la cassette d'expression issue du plasmide d'expression pcDNA-U6-GuideD1Chimérique-MS2 2X-Term comportant un ARN codant pour un ARN non codant ciblant la séquence de la GFP (sgRNA = ARN guide = Guide D1 dans ce cas), sous dépendance du promoteur U6 ;
- La figure XXII illustre l'efficacité d'une particule MS2RLP à délivrer le système CRISPR/Cas9 (ARN guide + ARN codant pour la Cas9) dans des cellules HCT116-GFP, en fonction du promoteur permettant la transcription de l'ARN guide dans les cellules de production ;
- La figure XXIII est un schéma de construction de la cassette d'expression issue du plasmide d'expression pcDNA-U6-Guide_antiPD1 Chimérique-MS2 2X-Term comportant un ARN codant pour un ARN non codant ciblant le gène PD1 (sgRNA = ARN guide = Guide), sous dépendance du promoteur U6 ;
- La figure XXIV illustre l'efficacité d'une particule MS2RLP à délivrer le système CRISPR/Cas9 (ARN guide + ARN codant pour la Cas9) dans des lymphocytes T primaires activés, pour l'invalidation du gène PD1 ;
- La figure XXV montre l'efficacité d'une particule ILV à délivrer le système CRISPR/Cas9 (ARN guide + ARN codant pour la Cas9) dans des lymphocytes T primaires activés, pour l'invalidation du gène PD1 ;
- La figure XXVI montre l'impact des particules MS2RLP délivrant le système CRISPR/Cas9 pour l'invalidation du gène PD1 (ARN guide + ARN codant pour la Cas9) sur la viabilité des lymphocytes T activés ;
- La figure XXVII illustre l'analyse du phénotype des lymphocytes T activés après transduction par les particules MS2RLP délivrant le système CRISPR/Cas9 pour l'invalidation du gène PD1 (ARN guide + ARN codant pour la Cas9) ;
- La figure XXVIII est un schéma de construction de la cassette d'expression issue du plasmide d'expression pcDNA-U6-Guide_antiCXCR4Chimérique-MS2 2X comportant un ARN codant pour un ARN non codant ciblant le gène CXCR4 (sgRNA = ARN guide = Guide), sous dépendance du promoteur U6 ;
- La figure XXIX montre l'efficacité d'une particule MS2RLP à délivrer le système CRISPR/Cas9 (ARN guide+ARN codant pour la Cas 9) dans des lymphocytes T primaires activés, pour l'invalidation du gène CXCR4;
- La figure XXX montre l'impact des particules MS2RLP délivrant le système CRISPR/Cas9 pour l'invalidation du gène CXCR4 (ARN guide+ARN codant pour la Cas 9) sur la viabilité des lymphocytes T primaires activés ; et
- La figure XXXI illustre l'analyse du phénotype des lymphocytes T activés après transduction par des particules MS2RLP délivrant le système CRISPR/Cas9 pour l'invalidation du gène CXCR4 (ARN guide+ARN codant pour la Cas 9).
- La figure XXXII est un schéma de construction de la cassette d'expression issue du plasmide pILV-H1/U6-GuideantiPD1-WPRE comportant une séquence d'ADN codant pour un ARN non codant ciblant le gène PD1 (sgRNA = ARN guide = Guide) ;
- La figure XXXIII montre l'effet de la dose de particules MS2RLP délivrant le système CRISPR/Cas9_(ARN guide+ARN codant pour la Cas 9) pour l'invalidation du gène de la GFP dans des cellules cibles HCT116 cloneD2 ;
- La figure XXXIV est un schéma de construction de la cassette d'expression issue du plasmide pcDNA-EF1-Cas9-PP7 2X comportant une séquence d'ADN codant pour l'ARN de la nucléase Cas9 ;
- La figure XXXV est un schéma de construction de la cassette d'expression issue du plasmide pcDNA-U6-GuideD1Chimérique-PP7 2X, comportant un ADN codant pour un ARN non codant ciblant la séquence de la GFP (sgRNA = ARN guide), d'une cassette d'expression promoteur-séquence d'intérêt-Term (sgRNA = ARN guide = Guide), sous dépendance du promoteur U6, dans lequel 2 répétitions du motif tige-boucle de l'ARN PP7 (respectivement SEQ ID N°2 et SEQ ID N°4) sont incluses dans la partie « scaffold » du guide chimérique;
- La figure XXXVIa illustre la stratégie de modification du plasmide d'encapsidation p8.74 porteur des gènes codant les protéines de structure et de fonction (Gag, Pol) pour la construction d'un plasmide p8.74ΔZF-PP7-Coat ;
- La figure XXXVIb est un schéma de construction de la cassette d'expression issue du plasmide d'encapsidation p8.74ΔZF-PP7-Coat ayant le second doigt de zinc substitué par la protéine « Coat » du bactériophage PP7, obtenu par la stratégie présentée en Figure XXXVIa ;
- La figure XXXVII illustre l'effet de la dose de particules PP7RLP délivrant le système CRISPR/Cas9 (ARN guide + ARN codant pour la Cas9) pour l'invalidation du gène de la GFP dans des cellules cibles HCT116 clone D2 ;
- La figure XXXVIII est un schéma illustrant la cassette d'expression issue du plasmide d'expression pcDNA.EF1.RapporteurFluorescent.MS2 12X comportant une séquence d'ADN codant pour l'ARN d'un rapporteur fluorescent, suivie de 12 répétitions du motif tige-boucle de l'ARN MS2 (SEQ ID N°1) ;
- La figure XXXIX est un schéma illustrant la cassette d'expression issue du plasmide d'expression pcDNA.EF1.RapporteurFluorescent.PP7 2X comportant une séquence d'ADN codant pour l'ARN d'un rapporteur fluorescent suivie de 2 répétitions du motif tige-boucle de l'ARN PP7 (SEQ ID N°2);
- La figure XXXX est un schéma de construction de la cassette d'expression issue du plasmide d'expression pILV-EF1-ZsGreenI-WPRE comportant un ADN codant pour l'ARN de la ZsGreenl ;
- La figure XXXXI montre l'effet du précurseur GAG-POL sauvage pour la production de particules MS2RLP 12X sur le transfert d'ARN dans des cellules Jurkat à la dose de 2pg p24/cellule ;
- La figure XXXXII montre l'effet du précurseur GAG-POL sauvage pour la production de particules MS2RLP 12X sur le transfert d'ARN dans des cellules Jurkat à la dose de 2pg p24/cellule ;
- La figure XXXXIII a et b illustre l'analyse de la maturation des particules virales MS2RLP par Western blot anti-p24 au bout de 15s (XXXXIIIb) et 1min (XXXXIIIa); et
- La figure XXXXIV montre l'efficacité des particules MS2/PP7 (NC)-RLP 12X 2X pour le transfert d'ARN encapsidés par les séquences d'encapsidation MS2 et PP7 dans des cellules cibles HCT116 ;

### EXEMPLE 1 : Comparaison du transfert de l'ARN codant pour la Cas9 après transduction d'un clone particulier de cellules HCT116 par des ILV ou des particules MS2RLP selon l'invention

### I. Matériels & Méthodes

### 1. Constructions des plasmides

Pour cet Exemple uniquement, la Cas9 utilisée est la Cas9 Nickase (sous une forme mutée). Les Exemples suivants et sauf indication contraire, la Cas9 dans sa forme sauvage (WT) est utilisée, le protocole restant inchangé.

### 1.1 Plasmides pour la production de particules lentivirales MS2-(NC)-RLP 12X selon l'invention

- **Plasmide d'expression d'une séquence d'intérêt:** Le plasmide d'expression est porteur d'une cassette d'expression (Figure I) avec ou non une séquence intronique ou stabilisatrice d'ARN. Afin de véhiculer les ARN dans les particules lentivirales, 12 répétitions du motif tige-boucle de l'ARN MS2 (ctagaaaacatgaggatcacccatgtctgcag, SEQ ID N°1) ont été insérées au sein d'une cassette d'expression en aval de la séquence de l'enzyme Cas9. Le promoteur utilisé est celui d'EF1 (Figure I) mais d'autres promoteurs peuvent être utilisés. La séquence d'intérêt du plasmide est un ADN codant pour l'ARN de la protéine Cas9 (Figure I), dans sa forme sauvage (WT) ou dans sa forme mutée (N), par exemple la Nickase.
- **Plasmide d'encapsidation** : La particule lentivirale a été modifiée pour contenir au sein de la protéine de nucléocapside, en lieu et place du second domaine en doigt de Zn, la séquence de la protéine « Coat » du bactériophage MS2. Le plasmide d'encapsidation p8.74, porteur des gènes codant les protéines de structure et de fonction (Gag, Pol), utilisé pour la production des particules MS2RLP est modifié selon la stratégie illustrée par la Figure IIa : ce plasmide p8.74 est utilisé pour générer, par PCR d'assemblage, un plasmide dépourvu du second doigt de zinc de la protéine de nucléocapside p8.74ΔZF. Le second doigt de zinc est substitué par la protéine « Coat » du bactériophage MS2 par clonage Hpal, pour générer le plasmide p8.74ΔZF-MS2-Coat. On obtient ainsi la construction illustrée en Figure IIb. La séquence codant Pol peut être délétée ou mutée dans certains éléments de fonction comme par exemple la séquence codant la transcriptase inverse (RT) ou l'intégrase (IN) sans altérer la fonction des MS2RLP.
- **Plasmide de l'enveloppe (pENV)** : Ce plasmide est porteur du gène codant une protéine d'enveloppe, qui peut être la VSV-G codant la protéine d'enveloppe du Virus de la stomatite vésiculaire (Figure III).
Ces plasmides sont utilisés pour la production de particules lentivirales MS2-(NC)-RLP 12X selon l'invention. Plus particulièrement, ces plasmides sont utilisés pour la production de particules lentivirales MS2RLP-Cas9 12X.

### 1.2 Plasmides pour la production de vecteurs lentiviraux intégratifs ILV

### 1.2.1. Plasmides pour la production de vecteurs lentiviraux intégratifs contrôles ILVCas9

- **Plasmide d'expression d'une séquence d'intérêt:** Le plasmide d'expression est porteur d'une cassette d'expression (Figure IV). Ce plasmide peut contenir d'autres éléments comme la séquence native WPRE (Woodchuck Hepatitis Virus Posttranscriptional Regulatory Element) ou encore la séquence cPPT/CTS. La pathogénicité virale est éliminée par la substitution de régions du génome viral requises pour la réplication rétrovirale par le transgène. Le promoteur utilisé est celui de l'EF1, mais d'autres promoteurs peuvent être utilisés. La séquence d'intérêt du plasmide est un ADN codant pour l'ARN de la protéine Cas9 (Figure IV), dans sa forme sauvage (WT) ou dans sa forme mutée (N).
- **Plasmide d'encapsidation** : Le plasmide d'encapsidation p8.74, porteur des gènes codant les protéines de structure et de fonction (Gag, Pol) est utilisé pour la production des vecteurs lentiviraux intégratifs (Figure VI).
- **Plasmide de l'enveloppe (pENV)** : Ce plasmide est identique au plasmide d'enveloppe utilisé pour la production de particules lentivirales MS2RLP (Figure III).

### 1.2.2. Plasmides pour la production de vecteurs lentiviraux intégratifs ILV-GFP pour la génération des cellules cibles HCT116-GFP Clone D2

- **Plasmide d'expression d'une séquence d'intérêt:** Le plasmide d'expression est porteur d'une cassette d'expression (Figure Va). Ce plasmide peut contenir d'autres éléments comme la séquence native WPRE (Woodchuck Hepatitis Virus Posttranscriptional Regulatory Element) ou encore la séquence cPPT/CTS. La pathogénicité virale est éliminée par la substitution de régions du génome viral requises pour la réplication rétrovirale par le transgène. Le promoteur utilisé est celui de l'EF1, mais d'autres promoteurs peuvent être utilisés. La séquence d'intérêt est la protéine fluorescente GFP (Figure Va).
- **Plasmide d'encapsidation** : Le plasmide d'encapsidation p8.74, porteur des gènes codant les protéines de structure et de fonction (Gag, Pol) est utilisé pour la production des vecteurs lentiviraux intégratifs (Figure VI).
- **Plasmide de l'enveloppe (pENV)** : Ce plasmide est identique au plasmide d'enveloppe utilisé pour la production de particules lentivirales MS2RLP (Figure III).

### 1.2.3. Plasmides pour la production de vecteurs lentiviraux intégratifs ILV-H1-GuideU3-U6-GuideD1 pour la génération des cellules cibles HTC116-GFP Clone D2-H1-GuideU3-U6-GuideD1

- **Plasmide d'expression d'une séquence d'intérêt:** Le plasmide d'expression est porteur d'une cassette d'expression telle que décrite en Figure Vb. Ce plasmide peut contenir d'autres éléments comme la séquence native WPRE (Woodchuck Hepatitis Virus Posttranscriptional Regulatory Element) ou encore la séquence cPPT/CTS. La pathogénicité virale est éliminée par la substitution de régions du génome viral requises pour la réplication rétrovirale par le transgène. Les promoteurs utilisés sont H1 et U6, mais d'autres promoteurs peuvent être utilisés. Les séquences d'intérêt sont deux ARNs non codants ciblant la séquence de la GFP, ci-après appelés guide U3 et guide D1 (sgRNA = ARN guide) (Figure Vb).
- **Plasmide d'encapsidation** : Le plasmide d'encapsidation p8.74, porteur des gènes codant les protéines de structure et de fonction (Gag, Pol) est utilisé pour la production des vecteurs lentiviraux intégratifs (Figure VI).
- **Plasmide de l'enveloppe (pENV)** : Ce plasmide est identique au plasmide d'enveloppe utilisé pour la production de particules lentivirales MS2RLP (Figure III)

### 2. Productions des lots de particules lentivirales et vecteurs lentiviraux

Après la transfection des plasmides sur des cellules de production, les surnageants sont récoltés et utilisés bruts ou concentrés/purifiés selon l'un des procédés mentionnés ci-après, décrits dans la demande WO 2013/014537.

### 2.1 Production des particules lentivirales et des vecteurs lentiviraux

Les productions sont réalisées en CellSTACK 10 étages (6360 cm², Corning) avec des cellules de production HEK293T (ATCC, CRL-11268), cultivées dans Dulbecco's Modified Eagle's Medium (DMEM, Gibco, Paisley, UK) supplémenté par 1% pénicilline/streptomycine et 1% d'ultraglutamine (PAA) à 37°C en atmosphère humide à 5% CO₂. Pour chaque lot (MS2-(NC)-RLP 12X et ILV), le mélange de transfection est composé des trois plasmides suivants :
- Un des plasmides d'expression décrit ci-avant, selon que l'on forme une particule (MS2-(NC)-RLP 12X) ou un vecteur (ILV),
- p8.74ΔZF Coat (MS2-(NC)-RLP 12X) ou p8.74 (ILV)
- pENV portant l'enveloppe VSV-G.

Plus particulièrement, pour le lot MS2RLP-Cas9 12X, le mélange de transfection est composé des trois plasmides suivants :
- le plasmide d'expression pcDNA-EF1-Cas9-MS2 12X (dont la cassette d'expression est illustrée en Figure I)
- le plasmide p8.74ΔZF-MS2 Coat (dont la cassette d'expression est illustrée en Figure IIb)
- le plasmide pENV portant l'enveloppe VSV-G (dont la cassette d'expression est illustrée en Figure III).

Plus particulièrement, pour les lots ILV-GFP, ILV-H1-GuideU3-U6-GuideD1 ou ILV-Cas9, le mélange de transfection est composé des trois plasmides suivants :
- le plasmide d'expression pILV-EF1-GFP-WPRE (dont la cassette d'expression est illustrée en Figure Va) ou pILV-H1-GuideU3-U6-GuideD1-WPRE (dont la cassette d'expression est illustrée en Figure Vb) ou pILV-EF1-Cas9-WPRE (dont la cassette d'expression est illustrée en Figure IV) respectivement
- le plasmide p8.74 (dont la cassette d'expression est illustrée en Figure VI)
- le plasmide pENV portant l'enveloppe VSV-G (dont la cassette d'expression est illustrée en Figure III).

Les proportions respectives des plasmides sont les suivantes: 40% du plasmide d'expression, 30% du plasmide p8.74 (ou p8.74ΔZF), 30% du plasmide pENV.

24 heures après transfection standard au phosphate de calcium, le surnageant de culture est remplacé par du milieu DMEM frais et non supplémenté. Les cellules de production sont incubées à 37°C / 5% CO2. Après changement du milieu, le surnageant est collecté quatre fois (32h, 48h, 56h et 72h post transfection). Chaque récupération est clarifiée par centrifugation 5min à 3000g avant d'être microfiltrée sur filtre de 0,45µm (Stericup®, Millipore). Toutes les récupérations sont alors mélangées pour composer le surnageant brut.

### 2.2 Concentration et purification des vecteurs lentiviraux et particules lentivirales

Les vecteurs et particules sont concentrés et purifiés selon l'une des deux procédés ci-après :
- Le procédé P1 vise à réaliser une ultrafiltration frontale du surnageant sur des unités centrales de centrifugation.
- Le procédé P2 vise à réaliser une ultrafiltration tangentielle puis une diafiltration du surnageant. Le surnageant brut est concentré et purifié par ultrafiltration tangentielle via l'utilisation de cartouches de fibres creuses en polysulfone. Le surnageant est traité par diafiltration pour 20 diavolumes en mode continu contre du tampon DMEM ou TSSM. Après la diafiltration, le rétentat est récupéré puis concentré à nouveau par ultrafiltration frontale sur des unités centrales de centrifugation.

Pour l'Exemple 1, les surnageants sont récoltés et utilisés concentrés/purifiés selon le procédé P1.

### 3. Titration des lots de particules lentivirales et de vecteurs lentiviraux

### 3.1 Titration des particules fonctionnelles par qPCR.

Les cellules de titration HCT116 (ATCC, CCL-247) sont ensemencées en plaque 96 puits dans 100µL de DMEM supplémenté de 10% SVF, 100 µg/mL Streptomycine, 100 U/mL Pénicilline et 2mM L-Gln (L-Glutamine) puis incubées 24h à 37°C / 5% CO₂. Six dilutions sériées sont réalisées pour chaque vecteur ainsi que pour un étalon interne. Les cellules de titration sont transduites par ces dilutions sériées en présence de Polybrene® 8µg/mL (Sigma) puis incubées trois jours à 37°C / 5% CO₂. Pour chaque série d'échantillons, un puits de cellules non transduites est rajouté en contrôle. Les cellules de titration sont ensuite trypsinées et le titre (Unité de Transduction/mL) est déterminé par qPCR après extraction de l'ADN génomique en utilisant le Nucleospin tissue gDNA extraction kit (Macherey-Nagel). Le titre obtenu (TU/mL) par qPCR est normalisé par l'étalon interne dont le titre a été précédemment déterminé par FACS.

### 3.2 Quantification des particules physiques par test ELISA p24.

La protéine de capside p24 est détectée directement sur le surnageant viral en utilisant et en suivant les recommandations du kit HIV-1 p24 ELISA kit (Perkin Elmer). La protéine p24 capturée est complexée avec un anticorps polyclonal biotinylé, puis détectée par une stréptavidine conjuguée à la péroxydase de raifort (HRP). Le complexe résultant est détecté par spectrophotométrie après incubation avec le substrat ortho-phénylènediamine-HCl (OPD) produisant une coloration jaune qui est directement proportionnelle à la quantité de protéine p24 capturée. L'absorbance de chaque puits est quantifiée au lecteur de plaque Synergy H1 Hybrid (Biotek) et calibrée contre l'absorbance d'une gamme étalon de protéine p24. Le titre viral exprimé en particules physiques par mL est calculé à partir de la concentration de protéine p24 obtenue sachant que 1pg de protéine p24 correspond à 10⁴ particules physiques.

### 4. Génération de cellules cibles et transduction par des particules lentivirales MS2-(NC)-RLP 12X selon l'invention

Cet exemple vise à montrer qu'il est possible de transférer l'ARN codant pour la nucléase Cas9 via des particules MS2RLP non intégratives, et qu'à l'issue de ce transfert d'ARN, pour générer des cassures d'ADN double brins permettant l'invalidation d'un gène cible.

### 4.1 Cellules cibles HCT116-GFP Clone D2

Les cellules cibles HCT116-GFP Clone D2 sont obtenues par transduction de cellules HCT116 (ATCC, CCL 247) à MOI20 en présence de 8µg/mL de Polybrene® avec un vecteur lentiviral intégratif (ILV) exprimant la GFP. La lignée monoclonale cible HCT116-GFP Clone D2 ne contenant qu'une seule copie d'ADN codant pour la GFP a été obtenue par dilution limite de la lignée polyclonale HCT116-GFP (préparé à partir du plasmide d'expression de la Figure Va) et criblage des clones par quantification du nombre de copies intégrées de la séquence GFP par PCR quantitative.

### 4.2 Cellules cibles HCT116-GFP CloneD2-H1-GuideU3-U6-GuideD1 et transduction par des particules lentivirales MS2-(NC)-RLP 12X selon l'invention

Les cellules cibles HCT116-GFP Clone D2 sont ensemencées en plaque 24 puits à 25000 cellules/cm² et incubées 24h à 37°C / 5% CO2. Dans un premier temps les cellules sont transduites par les vecteurs ILV-H1-GuideU3-U6-GuideD1 (préparé à partir du plasmide d'expression de la Figure Vb) à MOI40, en présence de 8µg/mL Polybrene®. Le surnageant de transduction est éliminé 4 heures après et remplacé par du milieu de culture supplémenté frais.

Une semaine après la transduction avec l'ILV-H1-GuideU3-U6-GuideD1, les cellules cibles HCT116-GFP CloneD2-H1-GuideU3-U6-GuideD2 sont transduites par les vecteurs ILV-EF1-Cas9 à MOI40 (préparé à partir du plasmide d'expression de la Figure IV) ou les particules MS2RLP-Cas9 à une dose de 10pg p24/cellule, en présence de 8µg/mL Polybrene®. Un inhibiteur des mécanismes de défense cellulaire, le BX795 (Invivogen), est utilisé à une concentration de 6 µM dans le cas des particules MS2RLP. Le surnageant de transduction est éliminé 4 heures après et remplacé par du milieu de culture supplémenté frais. A J14 post-transduction, les cellules sont récupérées et le pourcentage de cellules exprimant la GFP est quantifié par cytométrie (Macs Quant VYB, Miltenyi Biotec).

### II. Résultats

L'objectif de cette expérience est de comparer l'efficacité de transfert d'ARN codant pour la Cas9 par transduction de cellules cibles HCT116-GFP-CloneD2-H1-GuideU3-U6-GuideD1 avec des vecteurs ILV-Cas9 ou des particules MS2RLP-Cas9 12X. Dans un premier temps, les résultats présentés sur la Figure IX montrent que les cellules HCT116 non transduites (NT) ne sont pas fluorescentes (<0.4% de cellules GFP positives), alors que 99% des cellules cibles HCT116-GFP-CloneD2-H1-GuideU3-U6-GuideD1 sont fluorescentes. Seulement 13% des cellules cibles HCT116-GFP-CloneD2-H1-GuideU3-U6-GuideD1 transduites par le vecteur ILV-Cas9 sont encore fluorescentes. Lorsque les cellules cibles HCT116-GFP-CloneD2-H1-GuideU3-U6-GuideD1 sont transduites par les particules MS2RLP-Cas9 12X, on observe une diminution du nombre de cellules fluorescentes de l'ordre de 57%. Ce qui montre que 57% des cellules ont vu l'invalidation de la séquence GFP dans leur génome. Les particules MS2-(NC)-RLP 12X contenant 12 répétitions de séquences tige-boucles sont donc efficaces pour le transfert de l'ARNm codant pour la nucléase Cas9, et donc pour faire de l'édition de génome.

### EXEMPLE 2 : Comparaison de la position des motifs répétés MS2 pour l'encapsidation d'ARN guides dans des particules MS2 (NC)-RLP 2X après transduction d'un second clone particulier de cellules HCT116

### I. Matériels & Méthodes

### 1. Constructions des plasmides

### 1.1 Plasmides pour la production de particules lentivirales MS2 (NC)-RLP 2X selon l'invention

- **Plasmide d'expression d'une séquence d'intérêt:** Le plasmide d'expression est porteur d'une cassette d'expression telle que décrite en Figures VII ou VIII, avec ou non une séquence intronique ou stabilisatrice d'ARN. Afin de véhiculer les ARN dans les particules lentivirales, 2 répétitions du motif tige-boucle de l'ARN MS2 (ctagaaaacatgaggatcacccatgtctgcag, SEQ ID N°1 pour le guide D1 classique, ggccaacatgaggatcacccatgtctgcagggcc SEQ ID N°3 pour le guide D1 chimérique) ont été insérées au sein d'une cassette d'expression soit en aval de l'ARN guide (guide D1 classique, Figure VII), soit incluses dans la partie du scaffold du guide (guide D1 chimérique, Figure VIII). Le promoteur utilisé est celui de H1 mais d'autres promoteurs peuvent être utilisés comme le promoteur U6. L'utilisation d'un promoteur type ARN pol III dépendant, tel que H1 ou U6, nécessite la présence d'un signal de terminaison de la transcription (Term). La séquence d'intérêt est un ARN non codant ciblant la séquence de la GFP (sgRNA = ARN guide).
- **Plasmide d'encapsidation** : La particule lentivirale a été modifiée pour contenir au sein de la protéine de nucléocapside, en lieu et place du second domaine en doigt de Zn, la séquence de la protéine « Coat » du bactériophage MS2. Le plasmide d'encapsidation p8.74, porteur des gènes codant les protéines de structure et de fonction (Gag, Pol), utilisé pour la production des particules MS2RLP 2X est modifié selon la stratégie illustrée par la Figure IIa : ce plasmide p8.74 est utilisé pour générer, par PCR d'assemblage, un plasmide dépourvu du second doigt de zinc de la protéine de nucléocapside p8.74ΔZF. Le second doigt de zinc est substitué par la protéine « Coat » du bactériophage MS2 par clonage Hpal, pour générer le plasmide p8.74ΔZF-MS2-Coat. On obtient ainsi la construction illustrée en Figure IIb. La séquence codant Pol peut être délétée ou mutée dans certains éléments de fonction comme par exemple la séquence codant la transcriptase inverse (RT) ou l'intégrase (IN) sans altérer la fonction des MS2RLP 2X.
- **Plasmide de l'enveloppe (pENV)** : Ce plasmide est porteur du gène codant une protéine d'enveloppe, qui peut être la VSV-G codant la protéine d'enveloppe du Virus de la stomatite vésiculaire (Figure III).
Ces plasmides sont utilisés pour la production de particules lentivirales MS2-(NC)-RLP 12X selon l'invention. Plus particulièrement, ces plasmides sont utilisés pour la production des particules lentivirales MS2RLP-GuideD1 Classique 2X et MS2RLP-GuideD1 Chimérique 2X.

### 1.2 Plasmides pour la production de vecteurs lentiviraux intégratifs ILV

### 1.2.1. Plasmides pour la production de vecteurs lentiviraux intégratifs contrôles ILV-GuideD1

- **Plasmide d'expression d'une séquence d'intérêt:** Le plasmide d'expression est porteur d'une cassette d'expression telle que décrite en Figure Vc. Ce plasmide peut contenir d'autres éléments comme la séquence native WPRE (Woodchuck Hepatitis Virus Post-transcriptional Regulatory Element) ou encore la séquence cPPT/CTS. La pathogénicité virale est éliminée par la substitution de régions du génome viral requises pour la réplication rétrovirale par le transgène. Les promoteurs utilisés sont H1 et U6, mais d'autres promoteurs peuvent être utilisés. La séquence d'intérêt est un guide (Figure Vc).
- **Plasmide d'encapsidation** : Le plasmide d'encapsidation p8.74, porteur des gènes codant les protéines de structure et de fonction (Gag, Pol) est utilisé pour la production des vecteurs lentiviraux intégratifs (Figure VI).
- **Plasmide de l'enveloppe (pENV)** : Ce plasmide est identique au plasmide d'enveloppe utilisé pour la production de particules lentivirales MS2RLP (Figure III).

### 1.2.2. Plasmides pour la production de vecteurs lentiviraux intégratifs ILV-GFP pour la génération des cellules cibles HCT116-GFP Clone D2

Ces plasmides sont préparés selon un procédé identique à celui de l'Exemple 1.

### 1.2.3 Plasmides pour la production de vecteurs lentiviraux intégratifs ILV-Cas9 pour la préparation des cellules cibles HCT116-GFP Clone D2-EF1-Cas9WT

Ces plasmides sont préparés selon un procédé identique à celui de l'Exemple 1.

### 2. Productions des lots de particules lentivirales et vecteurs lentiviraux

Les particules lentivirales et les vecteurs lentiviraux sont produits comme décrit à l'Exemple 1 et sont concentrés et purifiés selon le procédé P1 tel que décrit à l'Exemple 1.

Plus particulièrement, pour le lot MS2RLP-GuideD1 Classique 2X et MS2RLP-GuideD1 Chimérique 2X, le mélange de transfection est composé des trois plasmides suivants :
- le plasmide d'expression pcDNA-H1-GuideD1-MS2 2X (dont la cassette d'expression est illustrée en Figure VII) ou le plasmide pcDNA-H1-GuideD1Chimérique-MS2 2X (dont la cassette d'expression est illustrée en Figure VIII)
- le plasmide p8.74ΔZF-MS2 Coat (dont la cassette d'expression est illustrée en Figure IIb)
- le plasmide pENV portant l'enveloppe VSV-G (dont la cassette d'expression est illustrée en Figure III).
La proportion de plasmides utilisés est identique à celle de l'Exemple 1.

### 3. Titration des lots de particules lentivirales et de vecteurs lentiviraux

Les particules lentivirales et les vecteurs lentiviraux sont titrés comme décrit à l'Exemple 1.

### 4. Génération de cellules cibles et transduction par des particules lentivirales MS2-(NC)-RLP 2X selon l'invention

Cet exemple vise à déterminer si la position des motifs répétés tiges-boucles MS2 pour l'encapsidation d'ARN guide dans des particules MS2RLP a un impact sur l'efficacité des particules pour l'invalidation d'un gène cible.

### 3.1 Cellules cibles HCT116-GFP CloneD2

Ce clone est préparé selon un procédé identique à celui de l'Exemple 1.

### 3.2 Cellules cibles HCT116-GFP cloneD2-Cas9 et transduction par des particules lentivirales MS2-(NC)-RLP 2X selon l'invention

Les cellules HCT116-GFP Clone D2 sont ensemencées en plaque 24 puits à 25000 cellules/cm² et incubées 24h à 37°C / 5% CO₂. Dans un premier temps les cellules sont transduites par les vecteurs ILV-Cas9, à MOI40, en présence de 8µg/mL Polybrene®. Le surnageant de transduction est éliminé 4 heures après et remplacé par du milieu de culture supplémenté frais. Une semaine après la transduction avec les vecteurs ILV-Cas9, les cellules cibles HCT116-GFP CloneD2 -Cas9 sont transduites par les particules MS2 (NC)-RLP 2X délivrant les ARN guides anti-GFP (classique ou chimérique) à une dose de 10pg p24/cellule, ou par les vecteurs ILV-GuideD1 à MOI40, en présence de 8µg/mL Polybrene®. Un inhibiteur des mécanismes de défense cellulaire, le BX795 (Invivogen), est utilisé à une concentration de 6 µM dans le cas des particules MS2RLP. Le surnageant de transduction est éliminé 4 heures après et remplacé par du milieu de culture supplémenté frais. A J14 post-transduction par les MS2RLP-guides-MS2 2X, les cellules sont récupérées et le pourcentage de cellules exprimant la GFP est quantifié par cytométrie (Macs Quant VYB, Miltenyi Biotec).

### II. Résultats

L'objectif de cette expérience est de comparer la position des motifs répétés MS2 pour l'encapsidation d'ARN guides dans les particules MS2RLP 2X après transduction. Dans un premier temps, les résultats présentés sur la Figure X montrent que les cellules HCT116 non transduites (NT) ne sont pas fluorescentes (<0.16% de cellules GFP positives), alors que plus de 99% des cellules cibles HCT116-GFP CloneD2 sont fluorescentes. A peine 13% des cellules cibles HCT116-GFP CloneD2-Cas9 transduites par les vecteurs ILV-GuideD1 sont encore fluorescentes. Lorsque les cellules cibles HCT116-GFP cloneD2-Cas9 sont transduites par les particules MS2RLP 2X -Guide, on observe une très faible diminution du nombre de cellules fluorescentes de 2% pour les MS2RLP-GuideD1 Classique 2Xtransportant les guides classiques et une diminution plus forte de l'ordre de 10% pour les MS2RLP-GuideD1 Chimérique 2X véhiculant les guides chimériques. La position des motifs répétés MS2 impacte donc sur l'efficacité des particules MS2RLP 2X. Si le pourcentage d'extinction reste modéré, les résultats montrent que les particules MS2RLP 2X permettent le transfert d'ARN guide, et donc de faire de l'édition de génome.

### EXEMPLE 3 : Impact de la quantité simple ou double du plasmide d'expression portant l'ARN guide dans les particules MS2 (NC)-RLP 2X pour la transduction du second clone de cellules HCT116

### I. Matériel & Méthodes

### 1. Constructions des plasmides

### 1.1 Plasmides pour la production de vecteurs lentiviraux intégratifs ILV

### 1.1.1 Plasmides pour la production de vecteurs lentiviraux intégratifs ILV-Cas9 pour la préparation des cellules cibles HCT116-GFP Clone D2-EF1-Cas9

Ces plasmides sont préparés selon un procédé identique à celui de l'Exemple 1.

### 1.1.2 Plasmides pour la production de vecteurs lentiviraux intégratifs ILV-GFP pour la génération des cellules cibles HCT116-GFP Clone D2

Ces plasmides sont préparés selon un procédé identique à celui de l'Exemple 1.

### 1.1.3 Plasmides pour la production de vecteurs lentiviraux intégratifs contrôles ILV pour les guides (ILV-GuideD1)

Ces plasmides sont préparés selon un procédé identique à celui de l'Exemple 2.

### 1.2 Plasmides pour la production de particules lentivirales MS2 (NC)-RLP 2X selon l'invention.

Ces plasmides sont préparés selon un procédé identique à celui de l'Exemple 1. Plus particulièrement, on utilise les plasmides mis en œuvre pour la production de particules lentivirales MS2RLP-GuideD1 Chimérique 2X.

### 2. Productions des lots de particules lentivirales et vecteurs lentiviraux

### 2.1 Production des particules lentivirales et des vecteurs lentiviraux

Les productions sont réalisées en CellSTACK 10 étages (6360 cm², Corning) avec des cellules de production HEK293T (ATCC, CRL-11268), cultivées dans Dulbecco's Modified Eagle's Medium (DMEM, Gibco, Paisley, UK) supplémenté par 1% pénicilline/streptomycine et 1% d'ultraglutamine (PAA) à 37°C en atmosphère humide à 5% CO₂. Les particules MS2RLP 2X sont produites par transfection des trois plasmides suivants :
- Un des plasmides d'expression décrits ci-avant, utilisé en quantité simple ou double selon que l'on forme une particule (MS2-(NC)-RLP 2X) ou en quantité simple pour un vecteur intégratif (ILV),
- p8.74ΔZF Coat (MS2-(NC)-RLP 2X) ou p8.74 (ILV); pENV portant l'enveloppe VSV-G.

Plus particulièrement, les particules lentivirales MS2RLP-GuideD1 Chimérique 2X sont produites par transfection des trois plasmides suivants :
- le plasmide d'expression pcDNA-H1-GuideD1 Chimérique-MS2 2X (dont la cassette d'expression est illustrée en Figure VIII) utilisé en quantité simple ou double
- p8.74ΔZF Coat-MS2 Coat (dont la cassette d'expression est illustrée en Figure IIb)
- pENV portant l'enveloppe VSV-G (dont la cassette d'expression est illustrée en Figure III).

Les proportions des plasmides sont les suivantes : 40% du plasmide d'expression, 30% du plasmide p8.74 ou p8.74ΔZF, 30% du plasmide pENV, 60% du plasmide d'expression, 20% du plasmide p8.74 ou p8.74ΔZF, 20% du plasmide pENV, pour le cas où la production comporte un seul plasmide d'expression, dont la quantité est doublée.

24 heures après transfection standard au phosphate de calcium, le surnageant de culture est remplacé par du milieu DMEM frais et non supplémenté. Les cellules de production sont incubées à 37°C / 5% CO₂. Après changement du milieu, le surnageant est collecté quatre fois (32h, 48h, 56h et 72h post transfection). Chaque récupération est clarifiée par centrifugation 5min à 3000g avant d'être microfiltrée sur filtre de 0,45µm (Stericup®, Millipore). Toutes les récupérations sont alors mélangées pour composer le surnageant brut.

Les vecteurs lentiviraux ILV-Cas9 sont produits comme décrit à l'Exemple 1.

Les vecteurs lentiviraux ILV-GFP sont produits comme décrit à l'Exemple 1.

Les vecteurs lentiviraux ILV-GuideD1 sont produits comme décrit à l'Exemple 2.

### 2.2 Concentration et purification particules lentivirales et des vecteurs lentiviraux.

Les vecteurs et particules sont concentrés et purifiés selon le procédé P1, décrit dans l'exemple1.

### 3. Titration des lots de particules lentivirales et vecteurs lentiviraux

Les particules lentivirales et les vecteurs lentiviraux sont titrés comme décrit dans l'Exemple 1.

### 4. Génération de cellules cibles et transduction par des particules lentivirales MS2-(NC)-RLP 2X selon l'invention

Cet exemple vise à comparer l'efficacité de particules MS2 (NC)-RLP 2X pour l'invalidation d'un gène cible avec des particules produites avec une quantité simple ou double du plasmide d'expression portant l'ARN guide D1 Chimérique, ciblant la séquence codant pour la GFP contenue dans les cellules HCT116-GFP cloneD2-EF1-Cas9 exprimant constitutivement l'enzyme Cas9.

### 4.1 Cellules cibles HCT116-GFP clone D2

Ce clone est préparé selon un procédé identique à celui de l'Exemple 1.

### 4.2 Cellules cibles HCT116-GFP cloneD2- Cas9 et transduction par des particules lentivirales MS2-(NC)-RLP 2X selon l'invention

Les cellules HCT116-GFP Clone D2 sont ensemencées en plaque 24 puits à 25000 cellules/cm2 et incubées 24h à 37°C / 5% CO2. Dans un premier temps les cellules sont transduites par les vecteurs ILV-Cas9 à MOI40, ces vecteurs étant tels que préparés à l'Exemple 2, en présence de 8µg/mL Polybrene®. Le surnageant de transduction est éliminé 4 heures après et remplacé par du milieu de culture supplémenté frais. Une semaine après la transduction avec l'ILV-Cas9, les cellules HCT116-GFP CloneD2- Cas9 sont transduites par les particules MS2RLP délivrant les guides D1 chimériques (produites avec une dose simple ou double de plasmide d'expression) ou par le vecteur ILV-GuideD1, à une dose de 10pg p24/cellule, en présence de 8µg/mL Polybrene®. Un inhibiteur des mécanismes de défense cellulaire, le BX795 (Invivogen), est utilisé à une concentration de 6 µM dans le cas des particules MS2 (NC)-RLP 2X. Le surnageant de transduction est éliminé 4 heures après et remplacé par du milieu de culture supplémenté frais. A J14 post-transduction, les cellules sont récupérées et le pourcentage de cellules exprimant la GFP ainsi que l'intensité de fluorescence sont quantifiés par cytométrie (Macs Quant VYB®, Miltenyi Biotec).

### II. Résultats

L'objectif de cette expérience est de comparer la production de particules MS2RLP délivrant de l'ARN guide produites par utilisation de quantité simple ou double du plasmide d'expression. Dans un premier temps, les résultats présentés sur la Figure XI montrent que les cellules HCT116 non transduites (NT) ne sont pas fluorescentes (<0.2% de cellules GFP positives), alors que plus de 99% des cellules cibles HCT116-GFP CloneD2 sont fluorescentes. Moins de 9% des cellules cibles HCT116-GFP CloneD2- Cas9 transduites par le vecteur ILV-GuideD1 sont encore fluorescentes. Lorsque les cellules cibles HCT116-GFP CloneD2- Cas9 sont transduites par le MS2RLP-GuideD1 Chimérique 2X, on observe une diminution du nombre de cellules fluorescentes de 6% pour les particules MS2RLP 2X transportant les guides chimériques produites en simple dose du plasmide d'expression. Cette diminution est doublée (13% de cellules cibles HCT116-GFP CloneD2- Cas9 négatives) pour les particules MS2RLP 2X véhiculant les guides chimériques produites avec une double dose de plasmide d'expression. Il est vraisemblable que la double dose de plasmide d'expression utilisée pour la production des particules MS2RLP 2X améliore donc le taux d'encapsidation des ARN guideD1, d'où une meilleure efficacité sur l'invalidation de la séquence GFP.

### EXEMPLE 4 : Efficacité d'une particule MS2RLP à délivrer le système CRISPR/Cas9 (ARN guide + ARN codant pour la Cas9) dans des cellules HCT116-GFP

### I. Matériel & Méthodes

### 1. Constructions des plasmides

### 1.1 Plasmides pour la production de particules lentivirales MS2 (NC)-RLP

### 12X 2X selon l'invention

**Plasmide d'expression d'une séquence d'intérêt :** Les plasmides d'expression dont la cassette d'expression est décrite dans l'Exemple 1 (Figure I, pcDNA-EF1-Cas9-MS2 12X) et dans l'Exemple 2 (Figure VIII, pcDNA-H1-GuideD1 Chimerique-MS2 2X) ont été utilisés pour co-encapsider dans la même particule MS2RLP12X-2X à la fois l'ARN codant pour la Cas9 et l'ARN guide ciblant la séquence de la GFP intégrée dans le génome des cellules cibles (HCT116-GFP CloneD2).
- **Plasmide d'encapsidation** : La particule lentivirale a été modifiée pour contenir au sein de la protéine de nucléocapside, en lieu et place du second domaine en doigt de Zn, la séquence de la protéine « Coat » du bactériophage MS2. Le plasmide d'encapsidation p8.74, porteur des gènes codant les protéines de structure et de fonction (Gag, Pol), utilisé pour la production des particules MS2RLP 12X-2X est modifié selon la stratégie illustrée par la Figure IIa : ce plasmide p8.74 est utilisé pour générer, par PCR d'assemblage, un plasmide dépourvu du second doigt de zinc de la protéine de nucléocapside p8.74ΔZF. Le second doigt de zinc est substitué par la protéine « Coat » du bactériophage MS2 par clonage Hpal, pour générer le plasmide p8.74ΔZF-MS2-Coat. On obtient ainsi la construction illustrée en Figure IIb. La séquence codant Pol peut être délétée ou mutée dans certains éléments de fonction comme par exemple la séquence codant la transcriptase inverse (RT) ou l'intégrase (IN) sans altérer la fonction des MS2RLP.
- **Plasmide de l'enveloppe (pENV)** : Ce plasmide est porteur du gène codant une protéine d'enveloppe, qui peut être la VSV-G codant la protéine d'enveloppe du Virus de la stomatite vésiculaire (Figure III).

Ces plasmides sont utilisés pour la production de particules lentivirales MS2(NC)-RLP 12X et 2X (ou MS2RLP 12X 2X) selon l'invention. Plus particulièrement, ces plasmides sont utilisés pour la production de particules lentivirales MS2RLP-Cas9 12X-GuideD1 Chimérique 2X.

### 1.2. Plasmides pour la production de vecteurs lentiviraux intégratifs ILV-GFP pour la génération des cellules cibles HCT116-GFP Clone D2

Ces plasmides sont préparés selon un procédé identique à celui de l'Exemple 1.

### 2. Productions des lots de particules lentivirales et vecteurs lentiviraux

Après la transfection des plasmides sur des cellules de production, les surnageants sont récoltés et utilisés bruts ou concentrés/purifiés selon l'un des procédés P1 ou P2 mentionnés précédemment, décrits dans la demande WO 2013/014537.

### 2.1 Production des particules lentivirales

Les productions sont réalisées en CellSTACK 10 étages (6360 cm², Corning) avec des cellules de production HEK293T (ATCC, CRL-11268), cultivées dans Dulbecco's Modified Eagle's Medium (DMEM, Gibco, Paisley, UK) supplémenté par 1% pénicilline/stréptomycine et 1% d'ultraglutamine (PAA) à 37°C en atmosphère humide à 5% CO₂.

La production des particules MS2 (NC)-RLP 12X-2X est réalisée par transfection des quatre plasmides suivants :
- Les deux plasmides d'expression décrits ci-avant dont le plasmide pcDNA-H1-GuideD1Chimérique-MS2 2X (Figure VIII) est utilisé en quantité double du plasmide pcDNA-EF1-Cas9-MS212X;
- p8.74ΔZF-MS2-Coat ;
- pENV portant l'enveloppe VSV-G.

Plus particulièrement, les particules lentivirales MS2RLP-Cas9 12X-GuideD1 Chimérique 2X sont produites par transfection des quatre plasmides suivants :
- Les deux plasmides d'expression décrits ci-avant dont le plasmide pcDNA-H1-GuideD1Chimérique-MS2 2X (dont la cassette d'expression est illustrée en Figure VIII) est utilisé en quantité double du plasmide pcDNA-EF1-Cas9-MS212X (dont la cassette d'expression est illustrée en Figure I) ;
- p8.74ΔZF-MS2-Coat (dont la cassette d'expression est illustrée en Figure IIb);

- pENV portant l'enveloppe VSV-G (dont la cassette d'expression est illustrée en Figure III).

24 heures après transfection standard au phosphate de calcium, le surnageant de culture est remplacé par du milieu DMEM frais et non supplémenté. Les cellules de production sont incubées à 37°C / 5% CO₂. Après changement du milieu, le surnageant est collecté quatre fois (32h, 48h, 56h et 72h post transfection). Chaque récupération est clarifiée par centrifugation 5min à 3000g avant d'être microfiltrée sur filtre de 0,45µm (Stericup®, Millipore). Toutes les récupérations sont alors mélangées pour composer le surnageant brut.

Cette production comprend donc deux plasmides d'expression, un plasmide d'encapsidation et un plasmide d'enveloppe. La proportion de plasmides utilisés est :
- 33% du plasmide d'expression codant pour le guide,
- 17% du plasmide d'expression codant pour la Cas9 (la quantité plasmide d'expression codant pour le guide est doublée par rapport à celle du plasmide d'expression codant pour la Cas9),
- 25% du plasmide p8.74ΔZF, et
- 25% du plasmide pENV.

Les vecteurs lentiviraux ILV-GFP sont produits comme décrit à l'Exemple 1.

### 2.2 Concentration et purification des particules lentivirales et des vecteurs lentiviraux

Les particules et les vecteurs sont concentrés et purifiés selon le procédé P1 décrit à l'Exemple 1.

### 3. Titration des particules physiques par test ELISA p24

La protéine de capside p24 est détectée directement sur le surnageant viral en utilisant et en suivant les recommandations du kit HIV-1 p24 ELISA kit (Perkin Elmer). La protéine p24 capturée est complexée avec un anticorps polyclonal biotinylé, puis détectée par une stréptavidine conjuguée à la péroxydase de raifort (HRP). Le complexe résultant est détecté par spectrophotométrie après incubation avec le substrat ortho-phenylenediamine-HCl (OPD) produisant une coloration jaune qui est directement proportionnelle à la quantité de protéine p24 capturée. L'absorbance de chaque puits est quantifiée au lecteur de plaque Synergy H1 Hybrid® (Biotek) et calibrée contre l'absorbance d'une gamme étalon de protéine p24. Le titre viral exprimé en particules physiques par mL est calculé à partir de la concentration de protéine p24 obtenue sachant que 1pg de protéine p24 correspond à 10⁴ particules physiques.

Les particules lentivirales et les vecteurs lentiviraux sont titrés comme décrit dans l'Exemple 1.

### 4. Génération de cellules cibles et transduction par des particules lentivirales MS2 (NC)-RLP 12X 2X selon l'invention

Cet exemple vise à montrer qu'il est possible de co-encapsider dans la même particule MS2RLP 12X-2X à la fois l'ARN codant pour la Cas9 et l'ARN guide ciblant la séquence de la GFP, intégrée dans le génome des cellules cibles, puis de transférer ces différents ARN via les particules MS2RLP-Cas9 12X-GuideD1 Chimérique 2X dans les cellules cibles, HCT116-GFP cloneD2. A l'issue de ce transfert d'ARN, le système CRISPR/Cas9 doit être fonctionnel et générer des cassures d'ADN double brin permettant l'invalidation du gène cible, et ainsi, transformer les cellules GFP+ en cellules GFP-, en utilisant qu'un seul et même outil pour le transfert des 2 constituants du système CRISPR/Cas9.

### 4.1 Cellules cibles HCT116-GFP cloneD2

Ce clone est préparé selon un procédé identique à celui de l'Exemple 1.

### 4.2 Transduction des cellules cibles HCT116-GFP CloneD2 par des particules lentivirales MS2 (NC)-RLP 12X 2X selon l'invention

Les cellules HCT116-GFP Clone D2 sont ensemencées en plaque 24 puits à 25000 cellules/cm² et incubées 24h à 37°C / 5% CO₂. Les cellules HCT116-GFP CloneD2 sont transduites par les particules MS2RLP12X-2X délivrant à la fois la Cas9 et le guide D1 chimérique, à une dose de 10pg p24/cellule en présence de 8µg/mL Polybrene®. Un inhibiteur des mécanismes de défense cellulaire, le BX795 (Invivogen), est utilisé à une concentration de 6µM dans le cas des particules MS2RLP 12X-2X. Le surnageant de transduction est éliminé 4 heures après et remplacé par du milieu de culture supplémenté frais. Une seconde transduction a été réalisée dans les mêmes conditions (Figure XX), 6 jours après la première transduction. Enfin, une troisième transduction a été réalisée dans les mêmes conditions 12 jours après la première transduction (Figure XII). 14 jours après la dernière transduction, les cellules sont récupérées et le pourcentage de cellules exprimant la GFP est quantifié par cytométrie (Macs Quant VYB, Miltenyi Biotec).

### II. Résultats

L'objectif de cette expérience est d'étudier la possibilité de transférer simultanément des ARN codant pour la Cas9 et des ARN guides via des particules MS2RLP 12X 2X en mesurant la fonctionnalité du système CRISPR/Cas9 dans des cellules cibles. Dans un premier temps, les résultats présentés sur la Figure XII montrent que les cellules HCT116 non transduites (NT) ne sont pas fluorescentes (<0.2% de cellules GFP+), alors que les cellules cibles HCT116-GFP cloneD2 sont fluorescentes à plus de 96%. Quand les cellules cibles sont transduites avec les particules MS2 (NC)-RLP 12X-2X délivrant le système CRISPR/Cas9 complet, on observe, 14 jours après la troisième transduction des cellules, une diminution du nombre de cellules fluorescentes de 38%. Il y a donc une invalidation du gène de la GFP de l'ordre de 38% avec des particules MS2RLP 12X 2X délivrant à la fois la nucléase Cas9 et les guides anti-GFP. L'extinction du gène cible par le système CRISPR/Cas9 est donc 3 fois plus efficace lorsque les cellules cibles sont transduites par un seul lot de particules MS2RLP ayant été produites par co-transfection de deux espèces de plasmides d'expression exprimant respectivement une nucléase et des ARN guides, que lorsque deux lots de particules transportant respectivement les ARN de la nucléase Cas9 et des ARN guides sont ajoutés au moment de la transduction (Figure XII et Figure XI). L'efficacité du système CRISPR Cas9 est donc plus efficace avec des particules transportant les ARNs multiples codant respectivement pour Cas9 et les ARN guides.

Les résultats présentés sur la Figure XX montrent que les cellules HCT116 non transduites (NT) ne sont pas fluorescentes (<0.2% de cellules GFP+), alors que les cellules cibles HCT116-GFP cloneD2 sont fluorescentes à plus de 99%. Quand les cellules cibles sont transduites avec les particules MS2 (NC)-RLP 12X 2X délivrant le système CRISPR/Cas9 complet, on observe, 14 jours après la seconde transduction des cellules, une diminution du nombre de cellules fluorescentes de 57%. Il y a donc une invalidation du gène de la GFP de l'ordre de 57% avec des particules MS2RLP 12X-2X délivrant les ARN codant à la fois pour la nucléase Cas9 et les guides anti-GFP. Pour rappel, on constate que le pourcentage de cellules exprimant la GFP diminue de 12.8% lorsque la Cas9 est exprimée de façon constitutive et l'ARN guide est apporté par les particules MS2RLP (Figure XI). Le pourcentage de cellules exprimant la GFP est donc cinq fois moins important lorsque les particules MS2RLP délivrent à la fois l'ARN codant pour la Cas9 et l'ARN guide après deux transductions (Figure XX). L'analyse des cellules exprimant encore la GFP après deux transductions versus trois transductions par les particules MS2RLP-Cas9 12X-GuideD1 Chimérique 2X montre que le système CRISPR semble plus efficace après deux transductions (57% d'efficacité, Figure XX) qu'après trois transductions (38% d'efficacité, Figure XII). Cette différence s'expliquerait par le fait que lorsque le système CRISPR est délivré dans la cellule cible (HCT116-GFP CloneD2), il va générer une cassure d'ADN double brin au niveau de la séquence cible, c'est-à-dire la séquence codant pour la protéine GFP, mais également sur d'autres séquences de manière non-spécifique. En effet, le système CRISPR présente un certain taux de coupure non-spécifique, appelé « effet off-target », permettant la génération de cassures d'ADN double brin soumises également au système de réparation de l'ADN (système NHEJ, pour Non Homologous End Joining), comme le sont les cassures d'ADN double brin générées de manière spécifique. Plus le système CRISPR est délivré dans la cellule cible, plus il présentera « d'effet off-target », il y aura de cassures d'ADN double brin générées dans la même cellule cible. Ce système arrive à une limite pour laquelle le système NHEJ devient saturé, et l'ADN n'est plus réparé. Dans ce cas, la non-réparation de l'ADN génomique induit la mort de la cellule transduite. C'est ce qui est observé dans les cellules transduites 3 fois par les particules MS2RLP-Cas9 12X-GuideD1 Chimérique 2X. En effet, les cellules transduites trois fois finissent par mourir dans la flasque de culture. Ainsi, les cellules non transduites, donc non touchées par le système CRISPR permettant l'extinction de la protéine GFP, présentent un avantage prolifératif par rapport aux cellules transduites trois fois, expliquant que le taux de cellules exprimant la GFP soit plus important après trois transductions par les particules MS2RLP-Cas9 12X-GuideD1 Chimérique 2X qu'après deux transductions.

En conclusion, ces particules MS2RLP 12X 2X se démarquent comme étant le meilleur outil pour co-délivrer le système CRISPR/Cas9, avec l'utilisation d'un seul et unique lot de particules, après une ou deux transductions, selon l'efficacité d'édition de génome, et selon le type cellulaire également.

### EXEMPLE 5 : Particules lentivirale selon l'invention pour le système TALEN

L'utilisation du système TALEN dans une stratégie d'édition de génome sous-entend l'utilisation d'un premier TALEN se fixant en amont du site de la coupure d'ADN double brin générée (TALEN 5'), ainsi qu'un second TALEN se fixant en aval du site de la coupure d'ADN double brin générée (TALEN 3').

La Figure XIIIa montre le plasmide d'expression permettant la production de particules MS2RLP 12X exprimant un TALEN se fixant en amont (côté 5') de la coupure d'ADN double brin générée.

La Figure XIIIb montre le plasmide d'expression permettant la production de particules MS2RLP 12X exprimant un TALEN se fixant en aval (côté 3') de la coupure d'ADN double brin générée.

La Figure XIVa montre le plasmide d'expression permettant la production de particules intégratives ILV exprimant un TALEN se fixant en amont (côté 5') de la coupure d'ADN double brin générée.

La Figure XIVb montre le plasmide d'expression permettant la production de particules intégratives ILV exprimant un TALEN se fixant en aval (côté 3') de la coupure d'ADN double brin générée.

### EXEMPLE 6 : Particules lentivirale selon l'invention pour le système Zn Finger Nuclease

L'utilisation du système Zn Finger Nuclease dans une stratégie d'édition de génome sous-entend l'utilisation d'un premier Zn Finger se fixant en amont du site de la coupure d'ADN double brin générée (ZFP 5'), ainsi qu'un second Zn Finger se fixant en aval du site de la coupure d'ADN double brin générée (ZFP 3').

La Figure XVa montre le plasmide d'expression permettant la production de particules MS2RLP 12X exprimant un Zn Finger se fixant en amont (côté 5') de la coupure d'ADN double brin générée.

La Figure XVb montre le plasmide d'expression permettant la production de particules MS2RLP 12X exprimant un Zn Finger se fixant en aval (côté 3') de la coupure d'ADN double brin générée.

La Figure XVIa montre le plasmide d'expression permettant la production de particules intégratives ILV exprimant un Zn Finger se fixant en amont (côté 5') de la coupure d'ADN double brin générée.

La Figure XVIb montre le plasmide d'expression permettant la production de particules intégratives ILV exprimant un Zn Finger se fixant en aval (côté 3') de la coupure d'ADN double brin générée.

### EXEMPLE 7 : Construction de particules lentivirales PP7(IN)-RLP par modification de l'intégrase

### I. Matériel & Méthodes

### 1. Constructions des plasmides

- **Plasmide d'expression d'une séquence d'intérêt** : Le plasmide d'expression est porteur d'une cassette d'expression (voir Figure XVII) avec ou non une séquence intronique ou stabilisatrice d'ARN. Afin de véhiculer les ARNm dans les particules lentivirales, 12 répétions du motif tige-boucle de l'ARN PP7 (ctagaaaggagcagacgatatggcgtcgctccctgcag SEQ ID N°2) ont été insérées au sein d'une cassette d'expression en aval du gène rapporteur (Figure XVII).

Le promoteur utilisé peut être celui du CMV ou EF1 (Figure XVII) mais d'autres promoteurs peuvent être utilisés. La séquence d'intérêt peut être un ADN codant une protéine reportrice comme la Luciferase Firefly native (Figure XVII), une protéine fluorescente verte (ZsGreenl), rouge (mCherry) ou bleue (mtBFP), ou un ADNc codant une protéine, par exemple la protéine CRE. La séquence d'intérêt peut également être celle d'un shRNA, d'un miRNA, d'un sgRNA, d'un LncRNA ou d'un circRNA.
- **Plasmide d'encapsidation** : La particule lentivirale a été modifiée pour contenir au sein de l'intégrase, la séquence de la protéine « Coat » du bactériophage PP7. Le plasmide d'encapsidation p8.74, porteur des gènes codant les protéines de structure et de fonction (Gag, Pol), utilisé pour la production des particules PP7(IN)-RLP est modifié selon la stratégie illustrée par la Figure XVIII : ce plasmide p8.74 est utilisé pour générer, par PCR d'assemblage, un plasmide sur lequel la protéine Coat du phage PP7 est fusionnée au domaine C terminal de l'intégrase. Cette fusion, obtenue par clonage Hpal, permet de générer le plasmide P8.74- POL-PP7 Coat. On obtient ainsi la construction illustrée en Figure XIX. La séquence codant Pol peut être délétée ou mutée dans certains éléments de fonction comme par exemple la séquence codant la transcriptase inverse (RT).
- **Plasmide de l'enveloppe (pENV)** : Ce plasmide est porteur du gène codant une protéine d'enveloppe, qui peut être la VSV-G codant la protéine d'enveloppe du Virus de la stomatite vésiculaire (Figure III).

### 2. Production, concentration/purification et titration des particules lentivirales

Les particules lentivirales sont produites comme décrit à l'Exemple 1, selon le procédé P1.

### 3. Cinétique d'expression de la Luciferase

Les cellules HCT116 (ATCC, CCL-247) sont ensemencées en plaque 96 puits et incubées 24h à 37°C / 5% CO2. La transduction par les particules PP7(IN)-RLP-Luc 12X produites selon le procédé P1 est réalisée à une dose de 2,8 pg p24/cellule, en présence de 8µg/mL Polybrene. Le surnageant de transduction est éliminé 4 heures après et remplacé par du milieu de culture supplémenté frais. A 4h, 8h, 24, 32, et 48h post-transduction, les cellules sont récupérées et l'expression de la Luciferase est analysée à l'aide du kit OneGlo Luciferase assay (Promega) en suivant les recommandations du fournisseur et en utilisant le lecteur de plaque Synergy H1 Hybrid (Biotek). Ce test est réalisé en triplicat. Le contrôle est réalisé par des cellules HCT116 non transduites.

### II. Résultats

Il est possible de véhiculer des ARN dans les particules lentivirales avec PP7-Coat dans l'intégrase. Le maximum d'expression de la Luciferase est atteint à 8h. Après 8h, l'activité de la Luciferase diminue. Les particules PP7(IN)-RLP permettent donc de délivrer des ARN.

### EXEMPLE 8 : Efficacité d'une particule MS2RLP à délivrer le système CRISPR/Cas9 (ARN guide + ARN codant pour la Cas9) dans des cellules HCT116-GFP, en fonction du promoteur permettant la transcription de l'ARN guide dans les cellules de production.

### I. Matériel & Méthodes

### 1. Constructions des plasmides

### 1.1 Plasmides pour la production de particules lentivirales MS2 (NC)-RLP 12X et 2X

- **Plasmide d'expression d'une séquence d'intérêt:** Les plasmides d'expression décrits dans l'Exemple 1 (Figure I, pcDNA-EF1-Cas9-MS2 12X) et sur la Figure XXI, pcDNA-U6-GuideD1 Chimerique-MS2 2X ont été utilisés pour co-encapsider dans la même particule MS2RLP 12X-2X à la fois l'ARN codant pour la Cas9 et l'ARN guide D1 sous le contrôle du promoteur U6, ciblant la séquence de la GFP intégrée dans le génome des cellules cibles (HCT116-GFP CloneD2).
- **Plasmide d'encapsidation** : La particule lentivirale a été modifiée pour contenir au sein de la protéine de nucléocapside, en lieu et place du second domaine en doigt de Zn, la séquence de la protéine « Coat » du bactériophage MS2. Le plasmide d'encapsidation p8.74, porteur des gènes codant les protéines de structures et de fonction (Gag, Pol), utilisé pour la production des particules MS2RLP 12X-2X est modifié selon la stratégie illustrée par la Figure IIa : ce plasmide p8.74 est utilisé pour générer, par PCR d'assemblage, un plasmide dépourvu du second doigt de zinc de la protéine de nucléocapside p8.74ΔZF. Le second doigt de zinc est substitué par la protéine « Coat » du bactériophage MS2 par clonage Hpal, pour générer le plasmide p8.74ΔZF-MS2-Coat. On obtient ainsi la construction illustrée en Figure IIb. La séquence codant Pol peut être délétée ou mutée dans certains éléments de fonction comme par exemple la séquence codant la transcriptase inverse (RT) ou l'intégrase (IN) sans altérer la fonction des MS2RLP.
- **Plasmide de l'enveloppe (pENV)** : Ce plasmide est porteur du gène codant une protéine d'enveloppe, qui peut être la VSV-G codant la protéine d'enveloppe du Virus de la stomatite vésiculaire (Figure III).
Plus particulièrement, ces plasmides sont utilisés pour la production de particules lentivirales MS2RLP-Cas9 12X-GuideD1 Chimérique 2X.

### 1.2. Plasmides pour la production de vecteurs lentiviraux intégratifs ILV-GFP pour la génération des cellules cibles HCT116-GFP Clone D2

Ces plasmides sont préparés selon un procédé identique à celui de l'Exemple 1.

### 2. Productions des lots de particules lentivirales et vecteurs lentiviraux

Après la transfection des plasmides sur des cellules de production, les surnageants sont récoltés et utilisés bruts ou concentrés/purifiés selon l'un des procédés P1 ou P2 mentionnés précédemment, décrits dans la demande WO 2013/014537.

### 2.1 Production des particules lentivirales et des vecteurs lentiviraux

Les productions sont réalisées en CellSTACK 10 étages (6360 cm², Corning) avec des cellules de production HEK293T (ATCC, CRL-11268), cultivées dans Dulbecco's Modified Eagle's Medium (DMEM, Gibco, Paisley, UK) supplémenté par 1% pénicilline/stréptomycine et 1% d'ultraglutamine (PAA) à 37°C en atmosphère humide à 5% CO₂.

La production des particules MS2 (NC)-RLP 12X 2X est réalisée par transfection des quatre plasmides suivants :
- Les deux plasmides d'expression décrits ci-avant dont le plasmide pcCDNA-U6-GuideD1Chimerique-MS2 2X (dont la cassette d'expression est illustrée en Figure XXI) est utilisé en quantité double du plasmide pcDNA-EF1-Cas9-MS2 12X (dont la cassette d'expression est illustrée en Figure I);
- p8.74ΔZF-MS2-Coat ;
- pENV portant l'enveloppe VSV-G.

24 heures après transfection standard au phosphate de calcium, le surnageant de culture est remplacé par du milieu DMEM frais et non supplémenté. Les cellules de production sont incubées à 37°C / 5% CO₂. Après changement du milieu, le surnageant est collecté quatre fois (32h, 48h, 56h et 72h post transfection). Chaque récupération est clarifiée par centrifugation 5min à 3000g avant d'être microfiltrée sur filtre de 0,45µm (Stericup®, Millipore). Toutes les récupérations sont alors mélangées pour composer le surnageant brut.

Les particules lentivirales MS2 (NC)-RLP 12X 2X sont produites comme décrit à l'Exemple 4.

Les vecteurs lentiviraux ILV-GFP sont produits comme décrit à l'Exemple 1.

### 2.2 Concentration et purification des particules lentivirales

Les particules sont concentrées et purifiées selon le procédé P1 décrit à l'Exemple 1.

### 3. Titration des particules physiques par test ELISA p24

La protéine de capside p24 est détectée directement sur le surnageant viral en utilisant et en suivant les recommandations du kit HIV-1 p24 ELISA kit (Perkin Elmer). La protéine p24 capturée est complexée avec un anticorps polyclonal biotinylé, puis détectée par une stréptavidine conjuguée à la péroxydase de raifort (HRP). Le complexe résultant est détecté par spectrophotométrie après incubation avec le substrat ortho-phenylenediamine-HCl (OPD) produisant une coloration jaune qui est directement proportionnelle à la quantité de protéine p24 capturée. L'absorbance de chaque puits est quantifiée au lecteur de plaque Synergy H1 Hybrid® (Biotek) et calibrée contre l'absorbance d'une gamme étalon de protéine p24. Le titre viral exprimé en particules physiques par mL est calculé à partir de la concentration de protéine p24 obtenue sachant que 1pg de protéine p24 correspond à 10⁴ particules physiques.

Les particules lentivirales et les vecteurs lentiviraux sont titrés comme décrit dans l'Exemple 1.

### 4. Génération de cellules cibles et transduction par des particules lentivirales MS2 (NC)-RLP 12X 2X selon l'invention

Cet exemple vise à montrer qu'il est possible de co-encapsider dans la même particule MS2RLP 12X-2X à la fois l'ARN codant pour la Cas9 et l'ARN guideD1 ciblant la séquence de la GFP intégrée dans le génome des cellules cibles, puis de transférer ces différents ARN via les particules MS2 (NC)-RLP 12X-2X dans les cellules cibles, HCT116-GFP cloneD2. A l'issue de ce transfert d'ARN, le système CRISPR/Cas9 doit être fonctionnel et générer des cassures d'ADN double brin permettant l'invalidation du gène cible, et ainsi, transformer les cellules GFP+ en cellules GFP-, en utilisant qu'un seul et même outil pour le transfert des 2 constituants du système CRISPR/Cas9.

### 4.1 Cellules cibles HCT116-GFP cloneD2

Ce clone est préparé selon un procédé identique à celui de l'Exemple 1.

### 4.2 Transduction des cellules cibles HCT116-GFP CloneD2 par des particules lentivirales MS2 (NC)-RLP 12X 2X selon l'invention

Les cellules HCT116-GFP Clone D2 sont ensemencées en plaque 24 puits à 25000 cellules/cm² et incubées 24h à 37°C / 5% CO₂. Les cellules HCT116-GFP CloneD2 sont transduites par les particules MS2RLP 12X 2X délivrant à la fois la Cas9 et le guide D1, une dose de 10pg p24/cellule, en présence de 8µg/mL Polybrene®. Un inhibiteur des mécanismes de défense cellulaire, le BX795 (Invivogen), est utilisé à une concentration de 6 µM dans le cas des particules MS2RLP 12X 2X. Le surnageant de transduction est éliminé 4 heures après et remplacé par du milieu de culture supplémenté frais. A J14 post-transduction, les cellules sont récupérées et le pourcentage de cellules exprimant la GFP est quantifié par cytométrie (Macs Quant VYB, Miltenyi Biotec).

### II. Résultats

L'objectif de cette expérience est d'évaluer l'impact du promoteur permettant l'expression de l'ARN guide pour la génération de particules MS2RLP délivrant simultanément des ARN codant pour la Cas9 et des ARN guides.

Dans cet exemple, deux promoteurs sont testés : H1 et U6.

Dans un premier temps, les résultats présentés sur la Figure XXII montrent que les cellules HCT116 non transduites (NT) ne sont pas fluorescentes (<0.1% de cellules GFP+), alors que les cellules cibles HCT116-GFP cloneD2 sont fluorescentes à plus de 99%. Quand les cellules cibles sont transduites avec les particules MS2 (NC)-RLP 12X 2X délivrant le système CRISPR/Cas9 complet, on observe, 14 jours après la seconde transduction des cellules, une diminution du nombre de cellules fluorescentes de 46,2% dans le cas des particules générées à partir du plasmide portant le promoteur H1, et 81,9% dans le cas des particules générées à partir du plasmide portant le promoteur U6. On observe donc la plus forte invalidation du gène de la GFP de l'ordre de 81,9% avec des particules MS2RLP 12X 2X générées à partir du plasmide portant le promoteur U6 après deux transductions. Le pourcentage de cellules exprimant la GFP est donc presque 2 fois moins important lorsque les particules sont générées à partir du plasmide portant le promoteur U6 que lorsque les particules sont générées à partir du plasmide portant le promoteur H1. En conclusion, la nature du promoteur utilisé pour générer les ARN guides à encapsider dans les particules MS2RLP a un impact sur l'efficacité de la particule MS2RLP délivrant le système CRISPR/Cas9.

### EXEMPLE 9 : Efficacité d'une particule MS2RLP à délivrer le système CRISPR/Cas9 (ARN guide + ARN codant pour la Cas9) dans des lymphocytes T primaires activés, pour l'invalidation du gène PD1.

### I. Matériel & Méthodes

### 1. Constructions des plasmides

### 1.1 Plasmides pour la production de particules lentivirales MS2 (NC)-RLP 12X 2X selon l'invention

- **Plasmide d'expression d'une séquence d'intérêt:** Le plasmide d'expression décrit dans l'Exemple 1 (dont la cassette d'expression est illustrée en Figure I, pcDNA-EF1-Cas9-MS2 12X) et le plasmide d'expression, dont la cassette d'expression est illustrée en Figure XXIII, pcDNA-U6-Guide_antiPD1Chimérique-MS2 2X ont été utilisés pour co-encapsider dans la même particule MS2RLP 12X-2X à la fois l'ARN codant pour la Cas9 et l'ARN guide sous le contrôle du promoteur U6, ciblant la séquence du gène PD1 intégré dans le génome des lymphocytes T primaires.
- **Plasmide d'encapsidation** : La particule lentivirale a été modifiée pour contenir au sein de la protéine de nucléocapside, en lieu et place du second domaine en doigt de Zn, la séquence de la protéine « Coat » du bactériophage MS2. Le plasmide d'encapsidation p8.74, porteur des gènes codant les protéines de structure et de fonction (Gag, Pol), utilisé pour la production des particules MS2RLP 12X-2X est modifié selon la stratégie illustrée par la Figure IIa : ce plasmide p8.74 est utilisé pour générer, par PCR d'assemblage, un plasmide dépourvu du second doigt de zinc de la protéine de nucléocapside p8.74ΔZF. Le second doigt de zinc est substitué par la protéine « Coat » du bactériophage MS2 par clonage Hpal, pour générer le plasmide p8.74ΔZF-MS2-Coat. On obtient ainsi la construction illustrée en Figure IIb. La séquence codant Pol peut être délétée ou mutée dans certains éléments de fonction comme par exemple la séquence codant la transcriptase inverse (RT) ou l'intégrase (IN) sans altérer la fonction des MS2RLP.
- **Plasmide de l'enveloppe (pENV)** : Ce plasmide est porteur du gène codant une protéine d'enveloppe, qui peut être la VSV-G codant la protéine d'enveloppe du Virus de la stomatite vésiculaire (Figure III).
Ces plasmides sont utilisés pour la production de particules lentivirales MS2 (NC)-RLP 12X 2X selon l'invention. Plus particulièrement, ces plasmides sont utilisés pour la production de particules lentivirales MS2RLP-Cas9 12X-GuideantiPD1Chimérique 2X.

### 1.2 Plasmides pour la production de particules lentivirales contrôles MS2-(NC)-RLP 12X selon l'invention

Ces plasmides sont préparés selon un procédé identique à celui de l'Exemple 1.Plus particulièrement, ces plasmides sont utilisés pour la production de particules lentivirales MS2RLP-Cas9 12X, tel que décrits au point 1.1 de l'Exemple 1.

### 1.3 Plasmides pour la production de vecteurs lentiviraux intégratifs contrôles ILVCas9+guideantiPD1

- **Plasmide d'expression d'une séquence d'intérêt:** Les plasmides d'expression utilisés sont porteurs d'une cassette d'expression telle que décrite en Figure IV et d'une cassette d'expression telle que décrite en Figure XXXII. Ces plasmides peuvent contenir d'autres éléments comme la séquence native WPRE (Woodchuck Hepatitis Virus Posttranscriptional Regulatory Element) ou encore la séquence cPPT/CTS. La pathogénicité virale est éliminée par la substitution de régions du génome viral requises pour la réplication rétrovirale par le transgène. Pour le premier plasmide, le promoteur utilisé est celui de l'EF1, mais d'autres promoteurs peuvent être utilisés. La séquence d'intérêt du plasmide est un ADN codant pour l'ARN de la protéine Cas9 (Figure IV), dans sa forme sauvage (WT) ou dans sa forme mutée (N).

Pour le second plasmide, les promoteurs utilisés sont U6 ou H1, mais d'autres promoteurs peuvent être utilisés. La séquence d'intérêt est un guide antiPD1 (Figure XXXII).
- **Plasmide d'encapsidation** : Le plasmide d'encapsidation p8.74, porteur des gènes codant les protéines de structure et de fonction (Gag, Pol) est utilisé pour la production des vecteurs lentiviraux intégratifs (Figure VI).
- **Plasmide de l'enveloppe (pENV)** : Ce plasmide est identique au plasmide d'enveloppe utilisé pour la production de particules lentivirales MS2RLP (Figure III).

### 2. Productions des lots de particules lentivirales et vecteurs lentiviraux

Après la transfection des plasmides sur des cellules, les surnageants sont récoltés et utilisés bruts ou concentrés/purifiés selon l'un des procédés P1 ou P2 mentionnés précédemment, décrits dans la demande WO 2013/014537.

### 2.1 Production des particules lentivirales et des vecteurs lentiviraux

Les productions sont réalisées en CellSTACK 10 étages (6360 cm², Corning) avec des cellules de production HEK293T (ATCC, CRL-11268), cultivées dans Dulbecco's Modified Eagle's Medium (DMEM, Gibco, Paisley, UK) supplémenté par 1% pénicilline/stréptomycine et 1% d'ultraglutamine (PAA) à 37°C en atmosphère humide à 5% CO₂.

La production des particules MS2 (NC)-RLP 12X 2X est réalisée par transfection des quatre plasmides suivants :
- Les deux plasmides d'expression décrits ci-avant dont le plasmide pcDNA-U6-Guide_antiPD1Chimérique-MS2 2X (dont la cassette d'expression est illustrée en Figure XXIII) est utilisé en quantité double du plasmide pcDNA-EF1-Cas9-MS2 12X (dont la cassette d'expression est illustrée en Figure I);
- p8.74ΔZF-MS2-Coat ;
- pENV portant l'enveloppe VSV-G.

Les particules MS2 (NC)-RLP 12X 2X sont produites selon le procédé décrit en Exemple 4.

Plus particulièrement, ces plasmides sont utilisés pour produire les particules lentivirales MS2RLP-Cas9 12X-GuideantiPD1 Chimérique 2X.

24 heures après transfection standard au phosphate de calcium, le surnageant de culture est remplacé par du milieu DMEM frais et non supplémenté. Les cellules de production sont incubées à 37°C / 5% CO₂. Après changement du milieu, le surnageant est collecté quatre fois (32h, 48h, 56h et 72h post transfection). Chaque récupération est clarifiée par centrifugation 5min à 3000g avant d'être microfiltrée sur filtre de 0,45µm (Stericup®, Millipore). Toutes les récupérations sont alors mélangées pour composer le surnageant brut.

Les particules lentivirales contrôles MS2 (NC)-RLP 12X sont produites comme décrit à l'Exemple 4.

La production, la purification et la titration des vecteurs lentiviraux ILV contrôles, exprimant le système CRISPR/Cas9 (ARN guide+Cas9), sont réalisées dans les mêmes conditions que dans l'Exemple 1, selon le procédé P2.

### 2.2 Concentration et purification des particules lentivirales

Les particules sont concentrées et purifiées selon le procédé P2 décrit à l'Exemple 1.

### 3. Titration des particules physiques par test ELISA p24

La protéine de capside p24 est détectée directement sur le surnageant viral en utilisant et en suivant les recommandations du kit HIV-1 p24 ELISA kit (Perkin Elmer). La protéine p24 capturée est complexée avec un anticorps polyclonal biotinylé, puis détectée par une stréptavidine conjuguée à la péroxydase de raifort (HRP). Le complexe résultant est détecté par spectrophotométrie après incubation avec le substrat ortho-phenylenediamine-HCl (OPD) produisant une coloration jaune qui est directement proportionnelle à la quantité de protéine p24 capturée. L'absorbance de chaque puits est quantifiée au lecteur de plaque Synergy H1 Hybrid® (Biotek) et calibrée contre l'absorbance d'une gamme étalon de protéine p24. Le titre viral exprimé en particules physiques par mL est calculé à partir de la concentration de protéine p24 obtenue sachant que 1pg de protéine p24 correspond à 10⁴ particules physiques.

Les particules lentivirales et les vecteurs lentiviraux sont titrés comme décrit dans l'Exemple 1.

### 4. Préparation des cellules cibles et transduction par des particules lentivirales MS2 (NC)-RLP 12X 2X selon l'invention

Cet exemple vise à montrer qu'il est possible de co-encapsider dans la même particule MS2RLP 12X 2X à la fois l'ARN codant pour la Cas9 et l'ARN guide ciblant la séquence du gène PD1, puis de transférer ces différents ARN via les particules MS2 (NC)-RLP 12X 2X dans les cellules cibles, les lymphocytes T primaires activés. A l'issue de ce transfert d'ARN, le système CRISPR/Cas9 doit être fonctionnel et générer des cassures d'ADN double brin permettant l'invalidation du gène cible PD1 en utilisant qu'un seul et même outil pour le transfert des 2 constituants du système CRISPR/Cas9.

### 4.1 Préparation des cellules cibles lymphocytes T primaires activés

Les cellules cibles, les lymphocytes T, sont préparées à partir de prélèvement de sang périphérique. Les cellules mononucléées du sang périphérique sont isolées par centrifugation sur gradient de Ficoll, puis mis en adhérence pendant 2 heures à 37°C /5% CO₂ en flasque T75. Les cellules en suspension sont récupérées, et les lymphocytes T sont purifiés par sélection négative en utilisant des billes magnétiques (Pan T cell isolation kit, Miltenyi Biotec). Les lymphocytes T purifiés sont activés pendant 24 heures (Dynabeads® Human T-Activator CD3/CD28, ThermoFisher) à 37°C /5% CO₂.

### 4.2 Transduction des cellules cibles lymphocytes T primaires activés

Les cellules cibles lymphocytes T primaires activés sont ensemencées en plaque 96 puits à 1000 000 cellules/mL et transduits par les particules MS2RLP 12X 2X délivrant à la fois la Cas9 et le guide, ou par les particules MS2RLP 12X délivrant seulement la Cas9 (contrôle négatif), en présence de 8µg/mL Polybrene®, à différentes doses (0.1 ; 0.5 ; 1 ou 5pg p24/cellule), ou par les particules ILV délivrant à la fois la Cas9 et le guide (contrôle positif), à différentes doses (MOI 5, 10, 25, 50). Un inhibiteur des mécanismes de défense cellulaire, le BX795 (Invivogen), est utilisé à une concentration de 6 µM dans le cas des particules MS2RLP 12X 2X et MS2RLP 12X.
Le surnageant de transduction est éliminé 4 heures après et remplacé par du milieu de culture supplémenté frais. 48 heures plus tard, une seconde transduction est réalisée dans les mêmes conditions que la première. Quatre jours après la seconde transduction, les cellules sont récupérées et le pourcentage de cellules exprimant PD1 est quantifié par cytométrie (Macs Quant VYB, Miltenyi Biotec) après immunomarquage des cellules par l'anticorps antiPD1 (Miltenyi Biotec).

La viabilité est analysée par ajout du Viobility™ Fixable Dyes (Miltenyi Biotec) juste avant l'analyse des cellules au FACS, et le phénotypage des lymphocytes est réalisé par immunomarquage des cellules avec les anticorps anti-TCR et antiCD25 (Miltenyi Biotec).

### II. Résultats

L'objectif de cette expérience est d'évaluer l'efficacité du système CRISPR/Cas9 délivré par les particules MS2RLP sur l'invalidation d'un gène endogène de cellules primaires, par exemple le gène PD1 dans des lymphocytes T primaires activés (Fig. XXIV).

Le gène PD1 fait l'objet d'une attention particulière dans le contexte tumoral car l'extinction de l'expression de PD1 permet une reconnaissance des cellules tumorales par le système immunitaire.

Quand les cellules sont transduites avec les particules MS2 (NC)-RLP 12X 2X délivrant le système CRISPR/Cas9 complet, on observe, quatre jours après la seconde transduction, une diminution du nombre de cellules exprimant la protéine PD1 de 31% dans le cas de la première dose (0.1pg p24/cellule), de 60% dans le cas de la deuxième dose (0.5pg p24/cellule), de 75% dans le cas de la troisième dose (1pg p24/cellule) et de 86% dans le cas de la quatrième dose (5pg p24/cellule). En parallèle, MS2RLP exprimant Cas9 seul est utilisé en contrôle négatif de l'invalidation du gène PD1. Les résultats montrent que le pourcentage de cellules exprimant la protéine PD1 est stable, quelque soit la dose de particules MS2RLP 12X 2X délivrant le système CRISPR/Cas9 utilisé.

La Figure XXV montre l'efficacité de la particule ILV à délivrer le système CRISPR/Cas9 pour l'invalidation du gène PD1 dans des conditions comparables à celles utilisées pour délivrer le système CRISPR/Cas9 avec l'utilisation des particules MS2RLP 12X 2X. A la MOI la plus forte utilisée (MOI 50), 42% des cellules expriment le gène PD1 alors qu'à la dose la plus forte de MS2RLP (5 pg p24/cellule), il ne reste que 14% de cellules exprimant le gène PD1. La particule MS2RLP est donc le meilleur outil pour délivrer le système CRISPR/Cas9 et permettre une invalidation efficace d'une cible endogène.

Les Figures XXIV et XXV ont été obtenues à partir de transduction de lymphocytes T humains activés, qui sont des cellules primaires, donc sensibles et délicates à toute manipulation. La Figure XXVI illustre la mesure de la viabilité des lymphocytes T, après la première et la seconde transduction avec les particules MS2RLP 12X-2X délivrant le système CRISPR/Cas9. La Figure XXVI permet de déterminer la dose optimale de particules MS2RLP 12X 2X délivrant le système CRISPR/Cas9, correspondant à 1 pg p24/cellule, afin d'obtenir une invalidation efficace du gène PD1 (Figure XXIV, 75% d'invalidation du gène PD1) sans affecter la viabilité des lymphocytes T activés.

La Figure XXVII illustre l'analyse du phénotype des lymphocytes T, après la seconde transduction avec les particules MS2RLP 12X-2X délivrant le système CRISPR/Cas9, par mesure de l'expression du TCR et du marqueur d'activation CD25. Les résultats montrent que le pourcentage de cellules exprimant le TCR et le CD25 est stable, quel que soit la dose de particules MS2RLP utilisée.

En conclusion, les Figures XXIV, XXVI et XXVII montrent que les particules MS2RLP sont le meilleur outil pour délivrer le système CRISPR/Cas9 (ARN guide + ARN codant pour la Cas9) tout en préservant la viabilité et le phénotype des lymphocytes T transduits.

### EXEMPLE 10 : Efficacité d'une particule MS2RLP à délivrer le système CRISPR/Cas9 (ARN guide + ARN codant pour la Cas9) dans des lymphocytes T primaires activés, pour l'invalidation du gène CXCR4.

### I. Matériel & Méthodes

### 1. Constructions des plasmides

### 1.1 Plasmides pour la production de particules lentivirales MS2 (NC)-RLP 12X 2X selon l'invention

**Plasmide d'expression d'une séquence d'intérêt** : Les plasmides d'expression décrits dans l'Exemple 1 (dont la cassette d'expression est illustrée Figure I, pcDNA-EF1-Cas9-MS2 12X) et sur la Figure XXVIII, pcDNA-U6-Guide_antiCXCR4Chimérique-MS2 2X, ont été utilisés pour co-encapsider dans la même particule MS2RLP 12X 2X à la fois l'ARN codant pour la Cas9 et l'ARN guide sous le contrôle du promoteur U6, ciblant la séquence du gène CXCR4 intégré dans le génome des lymphocytes T primaires.
- **Plasmide d'encapsidation** : La particule lentivirale a été modifiée pour contenir au sein de la protéine de nucléocapside, en lieu et place du second domaine en doigt de Zn, la séquence de la protéine « Coat » du bactériophage MS2. Le plasmide d'encapsidation p8.74, porteur des gènes codant les protéines de structure et de fonction (Gag, Pol), utilisé pour la production des particules MS2RLP 12X-2X est modifié selon la stratégie illustrée par la Figure IIa : ce plasmide p8.74 est utilisé pour générer, par PCR d'assemblage, un plasmide dépourvu du second doigt de zinc de la protéine de nucléocapside p8.74ΔZF. Le second doigt de zinc est substitué par la protéine « Coat » du bactériophage MS2 par clonage Hpal, pour générer le plasmide p8.74ΔZF-MS2-Coat. On obtient ainsi la construction illustrée en Figure IIb. La séquence codant Pol peut être délétée ou mutée dans certains éléments de fonction comme par exemple la séquence codant la transcriptase inverse (RT) ou l'intégrase (IN) sans altérer la fonction des MS2RLP.
- **Plasmide de l'enveloppe (pENV)** : Ce plasmide est porteur du gène codant une protéine d'enveloppe, qui peut être la VSV-G codant la protéine d'enveloppe du Virus de la stomatite vésiculaire (Figure III).
Ces plasmides sont utilisés pour la production de particules lentivirales MS2 (NC)-RLP 12X 2X selon l'invention. Plus particulièrement, ces plasmides sont utilisés pour la production de particules lentivirales MS2RLP-Cas9 12X-GuideantiCXCR4Chimérique 2X.

### 1.2 Plasmides pour la production de particules lentivirales contrôles MS2-(NC)-RLP 12X selon l'invention

Ces plasmides sont préparés selon un procédé identique à celui de l'Exemple 1. Plus particulièrement, ces plasmides sont utilisés pour la production de particules lentivirales MS2RLP-Cas9 12X, tel que décrits au point 1.1 de l'Exemple 1.

### 2. Productions des lots de particules lentivirales

Après la transfection des plasmides sur des cellules de production, les surnageants sont récoltés et utilisés bruts ou concentrés/purifiés selon l'un des procédés P1 ou P2 mentionnés précédemment, décrits dans la demande WO 2013/014537.

### 2.1 Production des particules lentivirales

Les productions sont réalisées en CellSTACK 10 étages (6360 cm², Corning) avec des cellules de production HEK293T (ATCC, CRL-11268), cultivées dans Dulbecco's Modified Eagle's Medium (DMEM, Gibco, Paisley, UK) supplémenté par 1% pénicilline/stréptomycine et 1% d'ultraglutamine (PAA) à 37°C en atmosphère humide à 5% CO₂.

La production des particules MS2 (NC)-RLP 12X-2X est réalisée par transfection des quatre plasmides suivants :
- Les deux plasmides d'expression décrits ci-avant dont le plasmide pcDNA-U6-Guide_antiCXCR4Chimérique-MS2 2X (dont la cassette d'expression est illustrée en Figure XXVIII) est utilisé en quantité double du plasmide pcDNA-EF1-Cas9-MS2 12X (dont la cassette d'expression est illustrée en Figure I);
- p8.74ΔZF-MS2-Coat ;
- pENV portant l'enveloppe VSV-G.

La proportion de plasmides utilisés est identique à celle de l'Exemple 4.

Plus particulièrement, ces plasmides sont utilisés pour produire les particules lentivirales MS2RLP-Cas9 12X-GuideantiCXCR4Chimérique 2X.

24 heures après transfection standard au phosphate de calcium, le surnageant de culture est remplacé par du milieu DMEM frais et non supplémenté. Les cellules de production sont incubées à 37°C / 5% CO₂. Après changement du milieu, le surnageant est collecté quatre fois (32h, 48h, 56h et 72h post transfection). Chaque récupération est clarifiée par centrifugation 5min à 3000g avant d'être microfiltrée sur filtre de 0,45µm (Stericup®, Millipore). Toutes les récupérations sont alors mélangées pour composer le surnageant brut.

Les particules lentivirales contrôles MS2-(NC)RLP 12X sont produites comme décrit à l'Exemple 1.

### 2.2 Concentration et purification des particules lentivirales

Les particules sont concentrées et purifiées selon le procédé P2 décrit à l'Exemple 1.

### 3. Titration des particules physiques par test ELISA p24

La protéine de capside p24 est détectée directement sur le surnageant viral en utilisant et en suivant les recommandations du kit HIV-1 p24 ELISA kit (Perkin Elmer). La protéine p24 capturée est complexée avec un anticorps polyclonal biotinylé, puis détectée par une stréptavidine conjuguée à la péroxydase de raifort (HRP). Le complexe résultant est détecté par spectrophotométrie après incubation avec le substrat ortho-phenylenediamine-HCl (OPD) produisant une coloration jaune qui est directement proportionnelle à la quantité de protéine p24 capturée. L'absorbance de chaque puits est quantifiée au lecteur de plaque Synergy H1 Hybrid® (Biotek) et calibrée contre l'absorbance d'une gamme étalon de protéine p24. Le titre viral exprimé en particules physiques par mL est calculé à partir de la concentration de protéine p24 obtenue sachant que 1pg de protéine p24 correspond à 10⁴ particules physiques.

### 4. Préparation des cellules cibles et transduction par des particules lentivirales MS2 (NC)-RLP 12X 2X selon l'invention

Cet exemple vise à montrer qu'il est possible de co-encapsider dans la même particule MS2RLP 12X-2X à la fois l'ARN codant pour la Cas9 et l'ARN guide ciblant la séquence du gène CXCR4, puis de transférer ces différents ARN via les particules MS2 (NC)-RLP 12X-2X dans les cellules cibles lymphocytes T primaires activés. A l'issue de ce transfert d'ARN, le système CRISPR/Cas9 doit être fonctionnel et générer des cassures d'ADN double brin permettant l'invalidation du gène cible CXCR4 en utilisant qu'un seul et même outil pour le transfert des 2 constituants du système CRISPR/Cas9.

### 4.1 Préparation des cellules cibles lymphocytes T primaires activés

Les cellules cibles lymphocytes T sont préparées à partir de prélèvement de sang périphérique. Les cellules mononucléées du sang périphérique sont isolées par centrifugation sur gradient de Ficoll, puis mis en adhérence pendant 2 heures à 37°C /5% CO₂ en flasque T75. Les cellules en suspension sont récupérées, et les lymphocytes T sont purifiés par sélection négative en utilisant des billes magnétiques (Pan T cell isolation kit, Miltenyi Biotec). Les lymphocytes T purifiés sont activés pendant 24 heures (Dynabeads® Human T-Activator CD3/CD28, ThermoFisher) à 37°C /5% CO₂.

### 4.2 Transduction des cellules cibles lymphocytes T primaires activés

Les cellules cibles lymphocytes T primaires activées sont ensemencées en plaque 96 puits à 1000 000 cellules/mL et transduits par les particules MS2RLP 12X 2X délivrant à la fois la Cas9 et le guide, ou par les particules MS2RLP 12X délivrant seulement la Cas9 (contrôle négatif), en présence de 8µg/mL Polybrene®, à différentes doses (0.1 ; 0.5 ; 1 ou 5pg p24/cellule). Un inhibiteur des mécanismes de défense cellulaire, le BX795 (Invivogen), est utilisé à une concentration de 6 µM dans le cas des particules MS2RLP 12X 2X et MS2RLP 12X.

Le surnageant de transduction est éliminé 4 heures après et remplacé par du milieu de culture supplémenté frais. 48 heures plus tard, une seconde transduction est réalisée dans les mêmes conditions que la première. Quatre jours après la seconde transduction, les cellules sont récupérées et le pourcentage de cellules exprimant CXCR4 est quantifié par cytométrie (Macs Quant VYB, Miltenyi Biotec) après immunomarquage des cellules par l'anticorps antiCXCR4 (Miltenyi Biotec).

La viabilité est analysée par ajout du Viobility™ Fixable Dyes (Miltenyi Biotec) juste avant l'analyse des cellules au FACS, et le phénotypage des lymphocytes est réalisé par immunomarquage des cellules avec les anticorps anti-TCR et antiCD25 (Miltenyi Biotec).

### II. Résultats

L'objectif de cette expérience est d'évaluer l'efficacité du système CRISPR/Cas9 délivré par les particules MS2RLP sur l'invalidation d'un gène endogène de cellules primaires, par exemple le gène CXCR4 dans des lymphocytes T primaires activés (Fig. XXIX).

Le gène CXCR4 est une cible d'intérêt car l'extinction de son expression dans les lymphocytes T CD4+ permet le blocage de l'infection par le virus du HIV-1.

Quand les cellules sont transduites avec les particules MS2 (NC)-RLP 12X 2X délivrant le système CRISPR/Cas9 complet, on observe, quatre jours après la seconde transduction, une diminution du nombre de cellules exprimant la protéine CXCR4 de 92% dans le cas de la première dose (0.1 pg p24/cellule), de 97% dans le cas de la deuxième dose (0.5pg p24/cellule), de 98% dans le cas de la troisième dose (1pg p24/cellule) et de 99% dans le cas de la quatrième dose (5pg p24/cellule).

En parallèle, MS2RLP exprimant Cas9 seul est utilisé en contrôle négatif de l'invalidation du gène CXCR4. Les résultats montrent que le pourcentage de cellules exprimant la protéine CXCR4 est stable, quelque soit la dose de particules MS2RLP 12X délivrant la protéine Cas9 seule.

Les Figures XXX et XXXI ont été obtenues à partir de transduction de lymphocytes T humains activés, qui sont des cellules primaires, donc sensibles et délicates à toute manipulation. La Figure XXX illustre la mesure de la viabilité des lymphocytes T, après la première et la seconde transduction avec les particules MS2RLP 12X-2X délivrant le système CRISPR/Cas9. On observe que la viabilité des lymphocytes T n'est pas altérée par la ou les transductions avec les particules MS2RLP 12X 2X, quelque soit la dose utilisée.

La dose optimale de particules MS2RLP 12X 2X délivrant le système CRISPR/Cas9, correspond à 5 pg p24/cellule, et permet d'obtenir une invalidation efficace du gène CXCR4 (Figure XXIX, 99% d'invalidation du gène CXCR4) sans affecter la viabilité des lymphocytes T activés.

La Figure XXXI illustre l'analyse du phénotype des lymphocytes T, après la seconde transduction avec les particules MS2RLP 12X 2X délivrant le système CRISPR/Cas9 pour l'invalidation du gène CXCR4, par mesure de l'expression du TCR et du marqueur d'activation CD25. Les résultats montrent que le pourcentage de cellules exprimant le TCR et le CD25 est stable, quelque soit la dose de particules MS2RLP utilisée.

En conclusion, les Figures XXIX, XXX et XXXI montrent que les particules MS2RLP sont le meilleur outil pour délivrer le système CRISPR/Cas9 (ARN guide + ARN codant pour la Cas9) tout en préservant la viabilité et le phénotype des lymphocytes T transduits.

### EXEMPLE 11 : Effet dose d'une particule MS2RLP délivrant le système CRISPR/Cas9 (ARN guide + ARN codant pour la Cas9) dans des cellules HCT116-GFP

### I. Matériel & Méthodes

### 1. Constructions des plasmides

### 1.1 Plasmides pour la production de particules lentivirales MS2 (NC)-RLP 12X 2X selon l'invention

Ces plasmides sont préparés selon un procédé identique à celui de l'Exemple 8. Plus particulièrement, ces plasmides sont utilisés pour la production de particules lentivirales MS2RLP-Cas9 12X-GuideD1 Chimérique 2X.

### 1.2 Plasmides pour la production de vecteurs lentiviraux intégratifs ILV-GFP pour la génération des cellules cibles HCT116-GFP Clone D2

Ces plasmides sont préparés selon un procédé identique à celui de l'Exemple 1.

### 2. Productions des lots de particules lentivirales et vecteurs lentiviraux

Après la transfection des plasmides sur des cellules de production, les surnageants sont récoltés et utilisés bruts ou concentrés/purifiés selon l'un des procédés P1 ou P2 mentionnés précédemment et tels que décrits dans la demande WO 2013/014537.

### 2.1 Production des particules lentivirales et des vecteurs lentiviraux

Les particules lentivirales MS2RLP 12X 2X sont produites selon le procédé décrit à l'Exemple 4.
Les vecteurs lentiviraux ILV-GFP sont produits comme décrit à l'Exemple 1.

### 2.2 Concentration et purification des particules lentivirales et des vecteurs lentiviraux

Les particules lentivirales et les vecteurs lentiviraux sont concentrés et purifiés selon le procédé P1 décrit à l'Exemple 1.

### 3. Titration des lots de particules lentivirales et de vecteurs lentiviraux

Les particules lentivirales et les vecteurs lentiviraux sont titrés selon le procédé décrit à l'Exemple 1.

### 4. Génération de cellules cibles et transduction par des particules lentivirales MS2 (NC)-RLP 12X 2X selon l'invention

Cet exemple vise à montrer qu'une particule MS2RLP 12X 2X délivrant le système CRISPR Cas9 dans des cellules cibles possède un effet dose sur l'efficacité d'édition du génome. A l'issue de ce transfert d'ARN, le système CRISPR/Cas9 doit être fonctionnel et générer des cassures d'ADN double brin permettant l'invalidation du gène cible, et ainsi, transformer les cellules GFP+ en cellules GFP-, en utilisant un seul et même outil pour le transfert des 2 constituants du système CRISPR/Cas9.

### 4.1 Cellules cibles HCT116-GFP cloneD2

Ce clone est préparé selon un procédé identique à celui de l'Exemple 1.

### 4.2 Transduction des cellules cibles HCT116-GFP CloneD2 par les particules lentivirales MS2 (NC)-RLP 12X 2X selon l'invention

Les cellules cibles HCT116-GFP CloneD2 sont ensemencées en plaque 24 puits à 25000 cellules/cm² et incubées 24h à 37°C / 5% CO₂. Les cellules cibles HCT116-GFP CloneD2 sont transduites par les particules MS2RLP-Cas9 12X-GuideD1 Chimérique 2X, délivrant à la fois la Cas9 et le guide D1 Chimérique, à différentes doses (0,5 ; 1 ; 5 ou 10pg p24/cellule) en présence de 8µg/mL Polybrene®. Un inhibiteur des mécanismes de défense cellulaire, le BX795 (Invivogen), est utilisé à une concentration de 6 µM dans le cas des particules MS2RLP 12X 2X. Le surnageant de transduction est éliminé 5 heures après et remplacé par du milieu de culture supplémenté frais. Une seconde transduction a été réalisée dans les mêmes conditions 6 jours après la première transduction (Figure XXXIII). 7 jours après la dernière transduction, les cellules sont récupérées et le pourcentage de cellules exprimant la GFP est quantifié par cytométrie (Macs Quant VYB, Miltenyi Biotec).

### II. Résultats

L'objectif de cette expérience est d'étudier la capacité des particules MS2RLP 12X 2X selon l'invention à induire un effet dose sur l'édition du génome, en transférant simultanément des ARN codant pour la Cas9 et des ARN guides à des concentrations différentes et en mesurant l'extinction de la GFP dans des HCT116-GFP CloneD2, avec une première transduction (Figure XXXIII, % de cellules positives T1) et une seconde transduction (Figure XXXIII, % de cellules positives T2).

Dans un premier temps, les résultats présentés sur la Figure XXXIII montrent que les cellules cibles HCT116-GFP cloneD2 sont fluorescentes à 100% alors que les cellules HCT116 non transduites (NT) ne sont pas fluorescentes. Quand les cellules sont transduites avec les particules MS2RLP 12X 2X délivrant le système CRISPR/Cas9 complet, on observe, 7 jours après la deuxième transduction des cellules (Figure XXXIII, % de cellules positives T2), une diminution du nombre de cellules fluorescentes suivant un effet dose. On observe une plus forte extinction de la GFP après la seconde transduction qu'après la première transduction quel que soit la dose de pg p24/cellule (0,5, 1, 5 ou10).

Après la seconde transduction, il y a une invalidation du gène de la GFP de l'ordre de 33% pour une dose de 0,5 pg p24/cellule, une invalidation du gène de la GFP de l'ordre de 39% pour 1 pg p24/cellule, une invalidation du gène de la GFP de l'ordre de 58% pour 5 pg p24/ cellule et enfin une invalidation du gène de la GFP de l'ordre de 65% à 10 pg p24/cellule. Une deuxième transduction permet donc d'augmenter l'efficacité de l'invalidation de la GFP par rapport à la première transduction. Plus la dose de particules MS2RLP 12X 2X est élevée plus l'invalidation de la GFP est importante.

### EXEMPLE 12 : Efficacité d'une particule PP7RLP à délivrer le système CRISPR/Cas9 (ARN guide + ARN codant pour la Cas9) dans des cellules HCT116-GFP

### I. Matériel & Méthodes

### 1. Constructions des plasmides

### 1.1. Plasmides pour la production de particules lentivirales PP7 (NC)-RLP 2X selon l'invention

Dans cet Exemple, les particules PP7 (NC)-RLP 2X seront appelées PP7 (NC)-RLP 2X 2X car elles comportent deux séries de motifs tige-boucle de PP7 différentes (1^{ere} série de motifs : SEQ ID N°2 suivie de SEQ ID N°4 et 2^{nde} série de motifs : SEQ ID N°5 suivie de SEQ ID N°6).
- **Plasmides d'expression d'une séquence d'intérêt** : Le premier plasmide d'expression est porteur d'une cassette d'expression telle que décrite en Figure XXXIV (pcDNA-EF1-Cas9-PP7 2X), avec ou non une séquence intronique ou stabilisatrice d'ARN. Afin de véhiculer les ARNm dans les particules lentivirales, 2 répétions du motif tige-boucle de l'ARN PP7 (ctagaaaggagcagacgatatggcgtcgctccctgcag SEQ ID N°2 et ctagaaaccagcagagcatatgggctcgctggctgcag SEQ ID N°4) ont été insérées au sein d'une cassette d'expression en aval de la séquence de l'enzyme Cas9 (Figure XXXIV). Le promoteur utilisé est celui d'EF1 (Figure XXXIV) mais d'autres promoteurs peuvent être utilisés. La séquence d'intérêt du plasmide est un ADN codant pour l'ARN de la protéine Cas9 (Figure XXXIV), dans sa forme sauvage (WT) ou dans sa forme mutée (N).

Le second plasmide d'expression est porteur d'une cassette d'expression telle que décrite en Figure XXXV (pcDNA-U6-GuideD1Chimerique-PP7 2X), avec ou non une séquence intronique ou stabilisatrice d'ARN. Afin de véhiculer les ARN guides dans les particules lentivirales, 2 répétitions du motif tige-boucle de l'ARN PP7 (ggagcagacgatatggcgtcgctcc SEQ ID N°5 et ccagcagagcatatgggctcgctgg SEQ ID N°6) ont été insérées au sein d'une cassette d'expression dans la partie du scaffold du guide (guide chimérique, Figure XXXV). Le promoteur utilisé est U6 mais d'autres promoteurs peuvent être utilisés, comme le promoteur H1. L'utilisation d'un promoteur type ARN pol III dépendant, tel que H1 ou U6, nécessite la présence d'un signal de terminaison de la transcription (Term). La séquence d'intérêt est un ARN non codant ciblant la séquence de la GFP (sgRNA = ARN guide).

Ces plasmides d'expression ont été utilisés pour co-encapsider dans la même particule PP7RLP 2X à la fois l'ARN codant pour la Cas9 et l'ARN guide ciblant la séquence de la GFP intégrée dans le génome des cellules cibles (HCT116-GFP CloneD2).
- **Plasmide d'encapsidation** : La particule lentivirale a été modifiée pour contenir au sein de la protéine de nucléocapside, en lieu et place du second domaine en doigt de Zn, la séquence de la protéine « Coat » du bactériophage PP7. Le plasmide d'encapsidation p8.74, porteur des gènes codant les protéines de structures et de fonction (Gag, Pol), utilisé pour la production des particules PP7RLP 2X est modifié selon la stratégie illustrée par la Figure XXXVIa : ce plasmide p8.74 est utilisé pour générer, par PCR d'assemblage, un plasmide dépourvu du second doigt de zinc de la protéine de nucléocapside p8.74ΔZF. Le second doigt de zinc est substitué par la protéine « Coat » du bactériophage PP7 par clonage Hpal, pour générer le plasmide p8.74ΔZF-PP7-Coat. On obtient ainsi la construction illustrée en Figure XXXVIb. La séquence codant Pol peut être délétée ou mutée dans certains éléments de fonction comme par exemple la séquence codant la transcriptase inverse (RT) ou l'intégrase (IN) sans altérer la fonction des PP7RLP 2X 2X.
- **Plasmide de l'enveloppe (pENV)** : Ce plasmide est porteur du gène codant une protéine d'enveloppe, qui peut être la VSV-G codant la protéine d'enveloppe du Virus de la stomatite vésiculaire (Figure III).

Plus particulièrement, ces plasmides sont utilisés pour la production de particules lentivirales PP7RLP Cas9 2X-GuideD1 Chimérique 2X.

### 1.2. Plasmides pour la production de vecteurs lentiviraux intégratifs ILV-GFP pour la génération des cellules cibles HCT116-GFP Clone D2

Ces plasmides sont préparés selon un procédé identique à celui de l'Exemple 1.

### 2. Productions des lots de particules lentivirales et vecteurs lentiviraux

Après la transfection des plasmides sur des cellules de production, les surnageants sont récoltés et utilisés bruts ou concentrés/purifiés selon l'un des procédés P1 ou P2 mentionnés précédemment et tels que décrits dans la demande WO 2013/014537.

### 2.1 Production des particules lentivirales et des vecteurs lentiviraux

Les productions sont réalisées en CellSTACK 10 étages (6360 cm², Corning) avec des cellules de production HEK293T (ATCC, CRL-11268), cultivées dans Dulbecco's Modified Eagle's Medium (DMEM, Gibco, Paisley, UK) supplémenté par 1% pénicilline/stréptomycine et 1% d'ultraglutamine (PAA) à 37°C en atmosphère humide à 5% CO₂.

La production des particules PP7 (NC)-RLP 2X 2X, préférentiellement PP7RLP-Cas9 2X-GuideD1 Chimérique 2X, est réalisée par transfection des quatre plasmides suivants :
- Les deux plasmides d'expression décrits ci-avant, dont le plasmide pcDNA-U6-GuideD1Chimerique-PP7 2X (dont la cassette d'expression est illustrée en Figure XXXV) est utilisé en quantité double du plasmide pcDNA-EF1-Cas9-PP7 2X (dont la cassette d'expression est illustrée en Figure XXXIV);
- p8.74ΔZF-PP7-Coat (dont la cassette d'expression est illustrée en Figure XXXVIb) ;
- pENV portant l'enveloppe VSV-G (dont la cassette d'expression est illustrée en Figure III).

Les particules PP7 (NC)-RLP 2X 2X sont produites selon le procédé décrit en Exemple 4. 24 heures après transfection standard au phosphate de calcium, le surnageant de culture est remplacé par du milieu DMEM frais et non supplémenté. Les cellules de production sont incubées à 37°C / 5% CO₂. Après changement du milieu, le surnageant est collecté quatre fois (32h, 48h, 56h et 72h post transfection). Chaque récupération est clarifiée par centrifugation 5min à 3000g avant d'être microfiltrée sur filtre de 0,45µm (Stericup®, Millipore). Toutes les récupérations sont alors mélangées pour composer le surnageant brut.

Les vecteurs lentiviraux ILV-GFP sont produits comme décrit à l'Exemple 1.

### 2.2 Concentration et purification des particules lentivirales et de vecteurs lentiviraux

Les particules lentivirales et les vecteurs lentiviraux sont concentrés et purifiés selon le procédé P1 décrit à l'Exemple 1.

### 3. Titration des lots de particules lentivirales et de vecteurs lentiviraux

Les particules lentivirales et les vecteurs lentiviraux sont titrés comme décrit dans l'Exemple 1.

### 4. Génération de cellules cibles et transduction par des particules lentivirales PP7 (NC)-RLP 2X 2X selon l'invention

Cet exemple vise à montrer qu'une particule PP7RLP délivrant le système CRISPR Cas9 à des cellules cibles possède un effet dose sur l'efficacité d'édition du génome. A l'issue de ce transfert d'ARN, le système CRISPR/Cas9 doit être fonctionnel et générer des cassures d'ADN double brin permettant l'invalidation du gène cible, et ainsi, transformer les cellules GFP+ en cellules GFP-, en utilisant qu'un seul et même outil pour le transfert des 2 constituants du système CRISPR/Cas9.

### 4.1 Cellules cibles HCT116-GFP cloneD2

Ce clone est préparé selon un procédé identique à celui de l'Exemple 1.

### 4.2 Transduction des cellules cibles HCT116-GFP CloneD2 par des particules lentivirales PP7 (NC)-RLP 2X 2X selon l'invention

Les cellules cibles HCT116-GFP CloneD2 sont ensemencées en plaque 24 puits à 25000 cellules/cm² et incubées 24h à 37°C / 5% CO₂. Les cellules cibles HCT116-GFP CloneD2 sont transduites par les particules PP7RLP 2X 2X délivrant à la fois la Cas9 et le guide D1 Chimérique à différentes doses (0.5 ; 1 ; 5 ou 10pg p24/cellule) en présence de 8µg/mL Polybrene®. Un inhibiteur des mécanismes de défense cellulaire, le BX795 (Invivogen), est utilisé à une concentration de 6 µM dans le cas des particules PP7RLP 2X 2X. Le surnageant de transduction est éliminé 17 heures après et remplacé par du milieu de culture supplémenté frais. Une seconde transduction a été réalisée dans les mêmes conditions 6 jours après la première transduction (Figure XXXVII). 7 jours après la dernière transduction, les cellules cibles sont récupérées et le pourcentage de cellules exprimant la GFP est quantifié par cytométrie (Macs Quant VYB, Miltenyi Biotec).

### II. Résultats

L'objectif de cette expérience est d'étudier la capacité des particules PP7RLP à induire un effet dose sur l'édition du génome, en transférant simultanément un ARN codant pour la Cas9 et un ARN guide ciblant la séquence codante pour la protéine GFP à des concentrations différentes et en mesurant l'extinction de la GFP dans des HCT116-GFP CloneD2 par cytométrie, avec une première transduction (Figure XXXVII, % de cellules positives T1) et une seconde transduction (Figure XXXVII, % de cellules positives T2).

Dans un premier temps, les résultats présentés sur la Figure XXXVII montrent que les cellules HCT116 non transduites (NT) ne sont pas fluorescentes, alors que les cellules cibles HCT116-GFP cloneD2 sont fluorescentes à 100%. Quand les cellules HCT116-GFP cloneD2 sont transduites avec les particules PP7RLP 2X délivrant le système CRISPR/Cas9 complet, on observe, 7 jours après la première et la seconde transduction des cellules cibles (Figure XXXVII, % de cellules positives T2), une diminution du nombre de cellules fluorescentes suivant un effet dose. On observe, comme pour les particules MS2RLP, une plus forte extinction de la GFP après la seconde transduction qu'après la première transduction quel que soit le nombre de pg p24/cellule (0.5, 1, 5 ou10).

Après la seconde transduction, il y a une invalidation du gène de la GFP de l'ordre de 33% pour une dose de 0.5 pg p24/cellule, de l'ordre de 54% pour 1 pg p24/cellule, de l'ordre de 85% pour 5 pg p24/ cellule et enfin de l'ordre de 75% à 10 pg p24/cellule. Une deuxième transduction permet donc d'augmenter l'efficacité de l'invalidation de la GFP par rapport à la première transduction. Plus la dose de particules PP7RLP est élevée plus l'invalidation de la GFP est importante, atteignant une efficacité maximale d'extinction à la dose 5 pg p24/cellule. Par ailleurs, les particules PP7RLP présentent un « effet off-target » tel que décrit en Exemple 4, entre les doses de particules PP7RLP 5 pg p24/ cellule et 10 pg p24/ cellule. La dose et le nombre de transduction utilisés sont donc des facteurs importants à prendre en compte pour l'efficacité des particules PP7RLP à délivrer le système CRISPR/Cas9 complet.

Les particules PP7RLP permettent d'obtenir un pourcentage plus important d'extinction de la GFP dans les cellules cibles HCT116-GFP cloneD2 que les particules MS2RLP (respectivement Figure XXXVII et Figure XXII). A la dose optimale de chaque particule, on observe une invalidation du gène de la GFP de l'ordre de 85% pour 5 pg p24/ cellule de particules PP7RLP vs. 82% pour 10 pg p24/ cellule de particules MS2RLP, soit une dose deux fois plus importante que pour les particules PP7RLP. L'efficacité des particules PP7RLP permet d'optimiser le transfert d'ARN CRISPR/Cas9, en particulier dans des cellules primaires délicates, pouvant être sensibles à plusieurs transductions. En conclusion, ces particules PP7RLP se démarquent comme étant le meilleur outil pour co-délivrer le système CRISPR/Cas9, avec l'utilisation d'un seul type de particules.

### EXEMPLE 13 : Impact du précurseur GAG-POL sauvage pour la production des particules MS2RLP-ZsGreen1 12X en vue de l'optimisation des particules MS2RLP-CRISPR/Cas9 efficaces (ARN guide + ARN codant pour la Cas9)

### I. Matériel & Méthodes

### 1. Constructions des plasmides

### 1.1 Plasmide pour la production d'une particule lentivirale MS2RLP 12X selon l'invention

- **Plasmide d'expression d'une séquence d'intérêt:** Le plasmide d'expression est porteur d'une cassette d'expression telle que décrite en Figure XXXVIII) avec ou non une séquence intronique ou stabilisatrice d'ARN. Afin de véhiculer les ARN dans les particules lentivirales, 12 répétitions du motif tige-boucle de l'ARN MS2 (ctagaaaacatgaggatcacccatgtctgcag, SEQ ID N°1) ont été insérées au sein d'une cassette d'expression en aval de la séquence de la protéine ZsGreenl. Le promoteur utilisé est celui d'EF1 mais d'autres promoteurs peuvent être utilisés. La séquence d'intérêt du plasmide est un ADN codant pour l'ARN de la protéine ZsGreenl.
- **Plasmides d'encapsidation** : Le premier plasmide d'encapsidation est le plasmide p8.74ΔZF dont la cassette d'expression est décrite en Figure IIb, obtenu par modification du plasmide d'encapsidation p8.74 pour contenir au sein de la protéine de nucléocapside, en lieu et place du second domaine en doigt de Zn, la séquence de la protéine « Coat » du bactériophage MS2, selon la stratégie illustrée par la Figure IIa et tel que décrit en Exemple 1.

Le second plasmide d'encapsidation est le plasmide p8.74, porteur des gènes codant les protéines de structures et de fonction sauvages (Gag, Pol), tel que présenté en Figure VI.
- **Plasmide de l'enveloppe (pENV)** : Ce plasmide est porteur du gène codant une protéine d'enveloppe, qui peut être la VSVG codant la protéine d'enveloppe du Virus de la stomatite vésiculaire (Figure III).

Plus particulièrement, ces plasmides sont utilisés pour la production de particules lentivirales MS2RLP-ZsGreenI12X.

### 1.2 Plasmide pour la production de vecteurs lentiviraux intégratifs contrôles ILV-ZsGreenI

- **Plasmide d'expression d'une séquence d'intérêt:** Le plasmide d'expression est porteur d'une cassette d'expression telle que décrit dans la Figure XXXXI. Ce plasmide peut contenir d'autres éléments comme la séquence native WPRE (Woodchuck Hepatitis Virus Posttranscriptional Regulatory Element) ou encore la séquence cPPT/CTS. La pathogénicité virale est éliminée par la substitution de régions du génome viral requises pour la réplication rétrovirale par le transgène. Le promoteur utilisé est celui de l'EF1, mais d'autres promoteurs peuvent être utilisés. La séquence d'intérêt du plasmide est un ADN codant pour l'ARN de la protéine ZsGreenl.
- **Plasmide d'encapsidation** : Le plasmide d'encapsidation p8.74, porteur des gènes codant les protéines de structures et de fonction (Gag, Pol) est utilisé pour la production des vecteurs lentiviraux intégratifs (Figure VI).
- **Plasmide de l'enveloppe (pENV)** : Ce plasmide est identique au plasmide d'enveloppe utilisé pour la production de particules lentivirales MS2RLP (Figure III).

### 2. Productions des lots de particules lentivirales et vecteurs lentiviraux

### 2.1 Production des particules lentivirales

Les productions sont réalisées en CellSTACK 10 étages (6360 cm², Corning) avec des cellules de production HEK293T (ATCC, CRL-11268), cultivées dans Dulbecco's Modified Eagle's Medium (DMEM, Gibco, Paisley, UK) supplémenté par 1% pénicilline/streptomycine et 1% d'ultraglutamine (PAA) à 37°C en atmosphère humide à 5% CO₂.

La production des particules MS2RLP 12X est réalisée par transfection des quatre plasmides suivants :
- Le plasmide d'expression décrit ci-avant, dont la cassette d'expression est illustrée en Figure XXXVIII ;
- p8.74ΔZF-MS2-Coat (dont la cassette d'expression est illustrée en Figure IIb) et le plasmide p8.74 (dont la cassette d'expression est illustrée en Figure VI), en utilisant quatre ratios différents des deux plasmides d'encapsidation, respectivement [100%
- 0%] ; [90% - 10%] ; [80% - 20%] et [50% - 50%] ;
- pENV portant l'enveloppe VSV-G (dont la cassette d'expression est illustrée en Figure III).

Les particules lentivirales MS2RLP-ZsGreenl 12X sont produites comme décrit à l'Exemple 1, c'est-à-dire avec les proportions respectives des plasmides suivantes: 40% du plasmide d'expression, 30% du plasmide p8.74 (ou p8.74ΔZF), 30% du plasmide pENV (ratio [100% - 0%]).

Plus particulièrement, les proportions respectives des plasmides sont les suivantes:
- ratio [90% - 10%] : 40% du plasmide d'expression, 27% du plasmide p8.74ΔZF, 3% du plasmide p8.74, 30% du plasmide pENV ;
- ratio [80% - 20%] : 40% du plasmide d'expression, 24% du plasmide p8.74ΔZF, 6% du plasmide p8.74, 30% du plasmide pENV
- ratio [50% - 50%] : 40% du plasmide d'expression, 15% du plasmide p8.74ΔZF, 15% du plasmide p8.74, 30% du plasmide pENV.

Dans le cas de la production d'une particule MS2 (NC)-RLP 12X 2X pour le transfert du système CRISPR/Cas9 complet, les particules sont produites le procédé tel que décrit à l'Exemple 8. Plus particulièrement, les proportions respectives des plasmides sont les suivantes : 33% du plasmide d'expression codant pour le guide, 17% du plasmide d'expression codant pour la Cas9 (la quantité plasmide d'expression codant pour le guide est doublée par rapport à celle du plasmide d'expression codant pour la Cas9), 25% du plasmide p8.74ΔZF, 25% du plasmide pENV (ratio [100% - 0%]),

Plus particulièrement, les proportions respectives des plasmides sont les suivantes:
- ratio [90% - 10%] : 40% du plasmide d'expression, 22,5% du plasmide p8.74ΔZF, 2,5% du plasmide p8.74, 30% du plasmide pENV ;
- ratio [80% - 20%] : 40% du plasmide d'expression, 20% du plasmide p8.74ΔZF, 5% du plasmide p8.74, 30% du plasmide pENV
- ratio [50% - 50%] 40% du plasmide d'expression, 12,5% du plasmide p8.74ΔZF, 12,5% du plasmide p8.74, 30% du plasmide pENV

Plus particulièrement, ces plasmides sont utilisés pour produire les particules lentivirales MS2RLP-ZsGreenI 12X.

Les vecteurs lentiviraux intégratifs ILV-ZsGreenI contenant la cassette d'expression EF1-ZsGreenI sont produits en contrôle.

Pour les lots ILV-ZsGreenI, le mélange de transfection est composé des trois plasmides suivants :
- le plasmide d'expression dont la cassette d'expression est illustrée en Figure XXXX
- le plasmide p8.74 (dont la cassette d'expression est illustrée en Figure VI)
- le plasmide pENV portant l'enveloppe VSV-G (dont la cassette d'expression est illustrée en Figure III).

24 heures après transfection standard au phosphate de calcium, le surnageant de culture est remplacé par du milieu DMEM frais et non supplémenté. Les cellules de production sont incubées à 37°C / 5% CO₂. Après changement du milieu, le surnageant est collecté quatre fois (32h, 48h, 56h et 72h post transfection). Chaque récupération est clarifiée par centrifugation 5min à 3000g avant d'être microfiltrée sur filtre de 0,45µm (Stericup®, Millipore). Toutes les récupérations sont alors mélangées pour composer le surnageant brut.

### 2.2 Concentration et purification des particules lentivirales

Les particules lentivirales et les vecteurs lentiviraux sont concentrés et purifiés selon le procédé P1 décrit à l'Exemple 1.

### 3. Titration des lots de particules lentivirales et de vecteurs lentiviraux

Les particules lentivirales et les vecteurs lentiviraux sont titrés comme décrit dans l'Exemple 1.

### 4. Préparation des cellules cibles et transduction par des particules lentivirales MS2RLP 12X selon l'invention

Des cellules cibles Jurkat (ATCC TIB-152) sont ensemencées à 200000 cellules/mL en plaque 96 puits, transduites par les particules MS2RLP 12X selon deux doses (2 et 10 pg p24/cellule), ou par des vecteurs lentiviraux contrôles ILV-ZsGreenI à MOI40, en présence de 4µg/mL Polybrene® puis incubées à 37°C / 5% CO₂. Un inhibiteur des mécanismes de défense cellulaire, le BX795 (Invivogen), est utilisé à une concentration de 6 µM dans le cas des particules MS2RLP 12X. Le surnageant de transduction est éliminé 5 heures après et remplacé par du milieu de culture supplémenté frais. 24h post-transduction, les cellules cibles sont récupérées et le pourcentage de cellules exprimant la ZsGreenl est quantifié par cytométrie (Macs Quant VYB, Miltenyi Biotec).

### 5. Analyse de la maturation des particules virales MS2RLP par Western blot anti-p24

48h après la transfection des cellules de production (HEK293T) avec les plasmides de production des particules MS2RLP 12X, les surnageants de cultures sont récupérés puis concentrés selon le procédé P1, tel que décrit à l'Exemple 1, et titrés par quantification de la protéine p24, tel que décrit à l'Exemple 1. L'équivalent de 15ng de p24 sont chargés pour chaque condition de ratio des plasmides p8.74ΔZF-MS2-Coat/p8.74 sur un gel dénaturant SDS-PAGE 4/12% puis migrés 1h à 200V en tampon MOPS1X. Après transfert sur membrane de nylon, les protéines sont hybridées avec un anticorps anti-p24 [clone 39/5.4A, Abcam). Le western blot est révélé à l'aide du kit Pierce™ Fast Western Blot Kit, ECL Substrate (Pierce). La visualisation des bandes est réalisée par chimioluminescence sur film d'autoradiographie.

### II. Résultats

Cet exemple vise à montrer qu'il est possible d'améliorer la fonctionnalité des particules MS2RLP par l'amélioration de la maturation du précurseur GAG lors de la production des particules, démontré par la preuve de concept sur la production de particules MS2RLP 12X. Le plasmide p8.74ΔZF-MS2 permet l'expression d'un précurseur GAG comportant la protéine Coat du bactériophage à la place du second doigt de Zinc de la protéine Nucléocapside. Cette protéine Coat est susceptible de perturber la maturation du précurseur GAG, lorsqu'il est clivé en trois protéines : la protéine de Matrice, la protéine de Capside et la protéine de Nucléocapside. Ces trois protéines sont indispensables à la structure des particules virales.

L'apport du précurseur GAG sauvage, par le plasmide p8.74 en plus du plasmide d'encapsidation p8.74ΔZF-MS2-Coat lors de la production des particules MS2RLP-ZsGreen1 12X pourrait permettre d'augmenter la maturation du précurseur GAG lorsqu'il est exprimé grâce au plasmide p8.74ΔZF-MS2, et ainsi, augmenter la fonctionnalité des particules MS2RLP.

L'objectif de cette expérience est d'évaluer l'impact du plasmide p8.74 lorsqu'il est co-transfecté, dans les cellules de production des particules MS2RLP 12X, en même temps que le plasmide d'encapsidation p8.74ΔZF-MS2-Coat permettant d'améliorer la maturation du précurseur GAG, et ainsi rendre les particules finales plus fonctionnelles pour la transduction de cellules cibles.

Dans cet exemple, quatre ratios de plasmides d'encapsidation p8.74ΔZF-MS2-Coat/p8.74 sont testés: 100/0; 90/10; 80/20 et 50/50. Un vecteur intégratif ILV exprimant la ZsGreenl est utilisé comme contrôle. Les cellules sont transduites selon deux quantités de p24/ml : 2 pg p24/cellule (Figure XXXXI) et 10 pg p24/cellule (Figure XXXXII).

Dans un premier temps, les résultats présentés sur la Figure XXXXI montrent que pour les cellules non transduites, le pourcentage de cellules fluorescentes est très proche de 0, alors que les cellules transduites par les particules MS2RLP-ZsGreenl 12X sont fluorescentes à plus de 99% quelque soit le ratio de plasmides d'encapsidation utilisé. Il est important de noter que l'intensité de fluorescence de la ZsGreenl augmente en fonction de l'augmentation de la quantité du plasmide p8.74. Les cellules transduites par les particules MS2RLP-ZsGreenl 12X produites avec 50% du plasmide d'encapsidation p8.74ΔZF-MS2-Coat et 50% du plasmide p8.74 présentent une intensité de fluorescence de 7,76 alors que celle des cellules transduites par les particules MS2RLP-ZsGreenl 12X produites uniquement avec le plasmide d'encapsidation p8.74ΔZF-MS2-Coat est de 3,5.

La Figure XXXXII montre le même résultat en termes de pourcentage de cellules transduites. Concernant l'intensité de fluorescence, plus la quantité de plasmide p8.74 augmente, et plus l'intensité de fluorescence augmente, comme montré en Figure XXXXI. Les cellules transduites par les particules MS2RLP-ZsGreenl 12X produites avec 50% du plasmide d'encapsidation p8.74ΔZF-MS2-Coat et 50% du plasmide p8.74 présentent une intensité de fluorescence de 60,67 alors que celle des cellules transduites par les particules MS2RLP-ZsGreenl 12X produites uniquement avec le plasmide d'encapsidation p8.74ΔZF-MS2-Coat est de 11,48.

Cela signifie qu'aux doses de 2pg et 10pg p24/cellule, la fluorescence obtenue est respectivement deux et cinq fois plus importante lorsque les particules sont produites avec 50% du plasmide d'encapsidation p8.74ΔZF-MS2-Coat et 50% du plasmide p8.74 que lorsqu'elles sont produites uniquement avec le plasmide p8.74ΔZF-MS2-Coat.

L'utilisation du plasmide p8.74 lors de la production de particules MS2RLP, en co-transfection avec le plasmide p8.74ΔZF-MS2-Coat, a donc apporté un gain sur l'amélioration de la fonctionnalité des particules MS2RLP-ZsGreenl 12X, en vue d'une optimisation des particules MS2RLP.

La Figure XXXXIII montre la maturation des particules MS2RLP-ZsGreenl 12X sur le plan biochimique, par recherche de la protéine p24 dans le surnageant de production contenant les particules. Elle correspond à une analyse des surnageants viraux par western blot anti-p24, selon deux temps d'exposition du film d'autoradiographie, une minute (Figure XXXXIIIa) et quinze secondes (Figure XXXXIIIb).

Dans le cas d'une particule lentivirale intégrative dérivée de HIV, produite uniquement avec le plasmide p8.74 en tant que plasmide d'encapsidation, la protéine p24 est détectable à quatre niveaux :
- dans le précurseur protéique p160 (GAG-POL)
- dans le précurseur protéique p55 (GAG),
- à l'état de protéine mature (p24)
- dans d'autres précurseurs protéiques intermédiaires en cours de maturation (entre p160 et p55, et entre p55 et p24).

Si la maturation se fait normalement, la protéine p24 doit être détectée en quantité plus importante à l'état mature (p24) qu'à l'état de précurseurs protéiques (p160, p55) ou de précurseurs protéiques intermédiaires. En effet, la maturation des particules virales se fait après la libération de la particule par la cellule de production. Autrement dit, lors de leur production, les particules bourgeonnent à la surface de la cellule de production, puis sont libérées de la cellule à l'état de particules immatures. Ce n'est qu'après la libération des particules dans le surnageant, que les précurseurs protéiques GAG-POL et GAG maturent en protéines définitives.

Dans le cas d'une particule MS2RLP 12X dérivée de HIV, produite uniquement avec le plasmide p8.74ΔZF-MS2-Coat en tant que plasmide d'encapsidation, la protéine p24 est détectable à quatre niveaux :
- dans le précurseur protéique p172 (GAGΔZF-MS2-Coat-POL)
- dans le précurseur protéique p67 (GAGΔZF-MS2-Coat),
- à l'état de protéine mature (p24)
- dans d'autres précurseurs protéiques intermédiaires en cours de maturation (entre p172 et p67, et entre p67 et p24).

Dans cette particule MS2RLP-ZsGreenl 12X, chaque précurseur est plus lourd de 12kDa, ce qui correspond à la taille de la protéine Coat du bactériophage MS2 insérée dans le second doigt de zinc de la protéine Nucléocapside.

La figure XXXXIII montre, sur la piste ILV, les différentes formes de précurseurs protéiques, p160 (GAG-POL), p55 (GAG) ainsi que la protéine p24 parfaitement mature. Il y a une majorité de protéine p24 mature, comparée aux formes précurseurs protéiques. Sur la piste 100/0, correspondant à des particules MS2RLP-ZsGreenl 12X produites uniquement avec le plasmide p8.74ΔZF-MS2-Coat en tant que plasmide d'encapsidation, la proportion de précurseurs/protéine p24 mature augmente par rapport à l'ILV, montrant que l'insertion de la protéine Coat du bactériophage MS2 diminue la maturation des particules virales. Sur les pistes 90/10, 80/20 et 50/50, la proportion de précurseurs protéiques p172 et p67 diminue au profit respectivement des précurseurs protéiques p160 et p55, pour arriver à un même niveau d'expression pour la piste 50/50. L'ajout du plasmide p8.74 au plasmide p8.74ΔZF-MS2-Coat, en tant que plasmide d'encapsidation pour la production de particules a pour conséquence l'augmentation de la proportion de la protéine p24 mature (Figure XXXXIIIb, 15 secondes d'exposition). Ce résultat montre que pour favoriser la maturation des précurseurs protéiques dans les particules MS2RLP, il faut rajouter au moins 10% de plasmide p8.74 en plus du plasmide p8.74ΔZF-MS2-Coat en tant que plasmide d'encapsidation pour la production de particules.

En conclusion, la production des particules MS2RLP utilisant au moins 10% de plasmide p8.74 en plus du plasmide p8.74ΔZF-MS2-Coat en tant que plasmide d'encapsidation permet non seulement une augmentation de la maturation des précurseurs en protéines matures, mais en plus permet d'augmenter la fonctionnalité des particules après transduction de cellules cibles.

### EXEMPLE 14 : Recrutement d'ARNs non viraux dirigé par deux séquences d'encapsidation différentes (MS2 et PP7) en vue de l'optimisation du transfert du système CRISPR par une particule RLP (modulation des types d'ARN encapsidés)

### I. Matériels & Méthodes

### 1. Constructions des plasmides

### 1.1 Plasmides pour la production de particules lentivirales MS2/PP7(NC)-RLP 12X 2X selon l'invention

- **Plasmides d'expression d'une séquence d'intérêt** : Le premier plasmide d'expression est porteur d'une cassette d'expression telle que décrite en Figure XXXVIII, avec ou non une séquence intronique ou stabilisatrice d'ARN. Afin de véhiculer les ARN dans les particules lentivirales, 12 répétitions du motif tige-boucle de l'ARN MS2 (ctagaaaacatgaggatcacccatgtctgcag, SEQ ID N°1) ont été insérées au sein d'une cassette d'expression en aval de l'ARN codant une protéine reportice comme la luciférase firefly native, une protéine fluorescente verte (ZsGreenl), rouge (mCherry) telle que décrite dans la Figure XXXVIII, ou un ADNc codant une protéine, par exemple une nucléase, comme la protéine Cas9 dans sa forme sauvage (WT) ou dans sa forme mutée (N). Le promoteur utilisé peut être celui du CMV ou EF1 (Figure XXXVIII) mais d'autres promoteurs peuvent être utilisés.

Le second plasmide d'expression est porteur d'une cassette d'expression telle que décrite en Figure XXXIX, avec ou non une séquence intronique ou stabilisatrice d'ARN. Afin de véhiculer les ARNm dans les particules lentivirales, 2 répétions du motif tige-boucle de l'ARN PP7 (ctagaaaggagcagacgatatggcgtcgctccctgcag SEQ ID N°2 et ctagaaaccagcagagcatatgggctcgctggctgcag SEQ ID N°4) ont été insérées au sein d'une cassette d'expression en aval de l'ARN codant une protéine reportice comme la luciférase firefly native, une protéine fluorescente verte (ZsGreenl), rouge (mCherry) telle que décrite dans la Figure XXXIX, ou un ADNc codant une protéine, par exemple une nucléase, comme la protéine Cas9 dans sa forme sauvage (WT) ou dans sa forme mutée (N). Le promoteur utilisé peut être celui du CMV ou EF1 (Figure XXXIX) mais d'autres promoteurs peuvent être utilisés.
- **Plasmides d'encapsidation** : La particule lentivirale a été modifiée pour contenir au sein de la protéine de nucléocapside, en lieu et place du second domaine en doigt de Zn, la séquence de la protéine « Coat » du bactériophage MS2 ou PP7. Le plasmide d'encapsidation p8.74, porteur des gènes codant les protéines de structures et de fonction (Gag, Pol), utilisé pour la production des particules MS2RLP 12X est modifié selon la stratégie illustrée par la Figure IIa : ce plasmide p8.74 est utilisé pour générer, par PCR d'assemblage, un plasmide dépourvu du second doigt de zinc de la protéine de nucléocapside p8.74ΔZF. Le second doigt de zinc est substitué par la protéine « Coat » du bactériophage MS2 par clonage Hpal, pour générer le plasmide p8.74ΔZF-MS2-Coat. On obtient ainsi la construction illustrée en Figure IIb. La séquence codant Pol peut être délétée ou mutée dans certains éléments de fonction comme par exemple la séquence codant la transcriptase inverse (RT) ou l'intégrase (IN) sans altérer la fonction des MS2RLP 12X.

Le plasmide d'encapsidation p8.74, porteur des gènes codant les protéines de structures et de fonction (Gag, Pol), utilisé pour la production des particules PP7RLP 2X est modifié selon la stratégie illustrée par la Figure XXXVIa : ce plasmide p8.74 est utilisé pour générer, par PCR d'assemblage, un plasmide dépourvu du second doigt de zinc de la protéine de nucléocapside p8.74ΔZF. Le second doigt de zinc est substitué par la protéine « Coat » du bactériophage PP7 par clonage Hpal, pour générer le plasmide p8.74ΔZF-PP7-Coat. On obtient ainsi la construction illustrée en Figure XXXVIb. La séquence codant Pol peut être délétée ou mutée dans certains éléments de fonction comme par exemple la séquence codant la transcriptase inverse (RT) ou l'intégrase (IN) sans altérer la fonction des PP7RLP 2X.
- **Plasmide de l'enveloppe (pENV)** : Ce plasmide est porteur du gène codant une protéine d'enveloppe, qui peut être la VSV-G codant la protéine d'enveloppe du Virus de la stomatite vésiculaire (Figure III).

Plus particulièrement, ces plasmides sont utilisés pour la production de particules lentivirales MS2/PP7-RLP-mCherry 12X- ZsGreenl 2X.

### 1.2 Plasmides pour la production de particules lentivirales contrôles MS2(NC)-RLP 12X selon l'invention

- **Plasmide d'expression d'une séquence d'intérêt:** Le plasmide d'expression est porteur d'une cassette d'expression telle que décrite en Figure XXXVIII avec ou non une séquence intronique ou stabilisatrice d'ARN. Afin de véhiculer les ARN dans les particules lentivirales, 12 répétitions du motif tige-boucle de l'ARN MS2 (ctagaaaacatgaggatcacccatgtctgcag, SEQ ID N°1) ont été insérées au sein d'une cassette d'expression en aval de l'ARN une protéine reportice comme la luciférase firefly native, une protéine fluorescente verte (ZsGreenl), rouge (mCherry) telle que décrite dans la Figure XXXVIII, ou un ADNc codant une protéine, par exemple une nucléase, comme la protéine Cas9 dans sa forme sauvage (WT) ou dans sa forme mutée (N). Le promoteur utilisé peut être celui du CMV ou EF1 (Figure XXXVIII) mais d'autres promoteurs peuvent être utilisés.
- **Plasmide d'encapsidation** : La particule lentivirale a été modifiée pour contenir au sein de la protéine de nucléocapside, en lieu et place du second domaine en doigt de Zn, la séquence de la protéine « Coat » du bactériophage MS2. Le plasmide d'encapsidation p8.74, porteur des gènes codant les protéines de structures et de fonction (Gag, Pol), utilisé pour la production des particules MS2RLP 12X est modifié selon la stratégie illustrée par la Figure IIa : ce plasmide p8.74 est utilisé pour générer, par PCR d'assemblage, un plasmide dépourvu du second doigt de zinc de la protéine de nucléocapside p8.74ΔZF. Le second doigt de zinc est substitué par la protéine « Coat » du bactériophage MS2 par clonage Hpal, pour générer le plasmide p8.74ΔZF-MS2-Coat. On obtient ainsi la construction illustrée en Figure IIb. La séquence codant Pol peut être délétée ou mutée dans certains éléments de fonction comme par exemple la séquence codant la transcriptase inverse (RT) ou l'intégrase (IN) sans altérer la fonction des MS2RLP 12X.
- **Plasmide de l'enveloppe (pENV) :** Ce plasmide est porteur du gène codant une protéine d'enveloppe, qui peut être la VSV-G codant la protéine d'enveloppe du Virus de la stomatite vésiculaire (Figure III).

Plus particulièrement, ces plasmides sont utilisés pour la production de particules lentivirales MS2RLP-mCherry 12X.

### 1.3 Plasmides pour la production de particules lentivirales contrôles PP7(NC)-RLP 2X selon l'invention

- **Plasmide d'expression d'une séquence d'intérêt** : Le plasmide d'expression est porteur d'une cassette d'expression telle que décrite en Figure XXXIX, avec ou non une séquence intronique ou stabilisatrice d'ARN. Afin de véhiculer les ARNm dans les particules lentivirales, 2 répétions du motif tige-boucle de l'ARN PP7 (ctagaaaggagcagacgatatggcgtcgctccctgcag SEQ ID N°2 et ctagaaaccagcagagcatatgggctcgctggctgcag SEQ ID N°4) ont été insérées au sein d'une cassette d'expression en aval de l'ARN codant une protéine reportice comme la luciférase firefly native, une protéine fluorescente verte (ZsGreenl), rouge (mCherry) telle que décrite dans la Figure XXXIX, ou un ADNc codant une protéine, par exemple une nucléase, comme la protéine Cas9 dans sa forme sauvage (WT) ou dans sa forme mutée (N). Le promoteur utilisé peut être celui du CMV ou EF1 (Figure XXXIX) mais d'autres promoteurs peuvent être utilisés.
- **Plasmide d'encapsidation** : La particule lentivirale a été modifiée pour contenir au sein de la protéine de nucléocapside, en lieu et place du second domaine en doigt de Zn, la séquence de la protéine « Coat » du bactériophage PP7. Le plasmide d'encapsidation p8.74, porteur des gènes codant les protéines de structures et de fonction (Gag, Pol), utilisé pour la production des particules PP7RLP 2X est modifié selon la stratégie illustrée par la Figure XXXVIa : ce plasmide p8.74 est utilisé pour générer, par PCR d'assemblage, un plasmide dépourvu du second doigt de zinc de la protéine de nucléocapside p8.74ΔZF. Le second doigt de zinc est substitué par la protéine « Coat » du bactériophage PP7 par clonage Hpal, pour générer le plasmide p8.74ΔZF-PP7-Coat. On obtient ainsi la construction illustrée en Figure XXXVIb. La séquence codant Pol peut être délétée ou mutée dans certains éléments de fonction comme par exemple la séquence codant la transcriptase inverse (RT) ou l'intégrase (IN) sans altérer la fonction des PP7RLP 2X.
- **Plasmide de l'enveloppe (pENV)** : Ce plasmide est porteur du gène codant une protéine d'enveloppe, qui peut être la VSV-G codant la protéine d'enveloppe du Virus de la stomatite vésiculaire (Figure III).

Plus particulièrement, ces plasmides sont utilisés pour la production de particules lentivirales PP7RLP-ZsGreenl 2X.

### 2. Productions des lots de particules lentivirales

Après la transfection des plasmides sur des cellules de production, les surnageants sont récoltés et utilisés bruts ou concentrés/purifiés selon l'un des procédés P1 ou P2 mentionnés précédemment et tels que décrits dans la demande WO 2013/014537.

### 2.1 Production des particules lentivirales

Les productions sont réalisées en CellSTACK 10 étages (6360 cm², Corning) avec des cellules de production HEK293T (ATCC, CRL-11268), cultivées dans Dulbecco's Modified Eagle's Medium (DMEM, Gibco, Paisley, UK) supplémenté par 1% pénicilline/stréptomycine et 1% d'ultraglutamine (PAA) à 37°C en atmosphère humide à 5% CO₂.

La production des particules lentivirales MS2/PP7(NC)-RLP 12X 2X, préférentiellement MS2/PP7-RLP-mCherry 12X- ZsGreenl 2X, est réalisée par transfection des cinq plasmides suivants :
- Les deux plasmides d'expression décrits ci-avant dont le plasmide pcDNA.EF1.mCherry.MS2 12X (dont la cassette d'expression est illustrée en Figure XXXVIII) (50%) et le plasmide pcDNA.EF1.ZsGreenI.PP7 2X (dont la cassette d'expression est illustrée en Figure XXXIX) (50%) utilisés en quantité simple ;
- p8.74ΔZF-PP7-Coat (50%) dont la cassette d'expression est illustrée en Figure XXXVIb et p8.74ΔZF-MS2-Coat (50%) dont la cassette d'expression est illustrée en Figure IIb;
- pENV portant l'enveloppe VSV-G dont la cassette d'expression est illustrée en Figure III.

Les particules lentivirales MS2/PP7 (NC)-RLP 12X 2X sont produites comme décrit à l'Exemple 1, c'est-à-dire avec les proportions respectives des plasmides suivantes: 40% du plasmide d'expression, 30% du plasmide p8.74 (ou p8.74ΔZF), 30% du plasmide pENV. Plus particulièrement, les proportions respectives des plasmides sont les suivantes: 20% du plasmide d'expression pcDNA.EF1.mCherry.MS2 12X, 20% du plasmide d'expression pcDNA.EF1.ZsGreenI.PP7 2X, 30% du plasmide p8.74ΔZF, 30% du plasmide pENV.

Dans le cas de la production d'une particule MS2/PP7 (NC)-RLP 12X 2X pour le transfert du système CRISPR/Cas9 complet, les particules sont produites le procédé tel que décrit à l'Exemple 8. Plus particulièrement, les proportions respectives des plasmides sont les suivantes : 33% du plasmide d'expression codant pour le guide, 17% du plasmide d'expression codant pour la Cas9 (la quantité plasmide d'expression codant pour le guide est doublée par rapport à celle du plasmide d'expression codant pour la Cas9), 25% du plasmide p8.74ΔZF et 25% du plasmide pENV.

La production des particules lentivirales contrôles MS2 (NC)-RLP 12X, préférentiellement MS2-RLP-mCherry 12X, est réalisée par transfection des trois plasmides suivants :
- Le plasmide d'expression décrit ci-avant pcDNA.EF1.mCherry.MS2 12X (dont la cassette d'expression est illustrée en XXXVIII) ;
- p8.74ΔZF-MS2-Coat dont la cassette d'expression est illustrée en Figure IIb ;
- pENV portant l'enveloppe VSV-G dont la cassette d'expression est illustrée en Figure III.

Les particules lentivirales MS2 (NC)-RLP 12X sont produites comme décrit à l'Exemple 1.

La production des particules lentivirales contrôles PP7 (NC)-RLP 2X, préférentiellement PP7-RLP-ZsGreenl 2X, est réalisée par transfection des trois plasmides suivants :
- Le plasmide d'expression décrit ci-avant pcDNA.EF1.ZsGreenI.PP7 2X (dont la cassette d'expression est illustrée en Figure XXXIX);
- p8.74ΔZF-PP7-Coat dont la cassette d'expression est illustrée en Figure XXXVIb ;
- pENV portant l'enveloppe VSV-G dont la cassette d'expression est illustrée en Figure III.

Les particules lentivirales PP7(NC)-RLP 2X sont produites comme décrit à l'Exemple 1.

24 heures après transfection standard au phosphate de calcium, le surnageant de culture est remplacé par du milieu DMEM frais et non supplémenté. Les cellules de production sont incubées à 37°C / 5% CO₂. Après changement du milieu, le surnageant est collecté quatre fois (32h, 48h, 56h et 72h post transfection). Chaque récupération est clarifiée par centrifugation 5min à 3000g avant d'être microfiltrée sur filtre de 0,45µm (Stericup®, Millipore). Toutes les récupérations sont alors mélangées pour composer le surnageant brut.

### 2.2 Concentration et purification des particules lentivirales

Les particules lentivirales sont concentrées et purifiées selon le procédé P1 décrit à l'Exemple 1.

### 3. Titration des lots de particules lentivirales

Les particules lentivirales sont titrées comme décrit dans l'Exemple 1.

### 4. Transduction par des particules lentivirales MS2/PP7(NC)-RLP 12X 2X selon l'invention

Cet exemple est réalisé en utilisant des particules MS2/PP7(NC)-RLP 12X 2X permettant le transfert de plusieurs types d'ARNs et donc l'expression de plusieurs protéines différentes (ZsGreenl + mCherry).

Des cellules cibles HCT116 (ATCC, CCL-247) sont ensemencées en plaque 24 puits et incubées 24h à 37°C / 5% CO2 et ont été transduites par les particules MS2/PP7(NC)-RLP 12X 2X à une dose de 10pg p24/cellule.

Les contrôles réalisés sont les suivants :
- Transduction avec les particules lentivirales MS2RLP-mCherry 12X et PP7RLP-ZsGreenl 2X, à une dose de 10pg p24/cellule chacune ;
- Transduction avec les particules lentivirales MS2RLP-mCherry 12X seul, à une dose de 10pg p24/cellule ;
- Transduction avec les particules lentivirales PP7RLP-ZsGreenl 2X seul, à une dose de 10pg p24/cellule.

La transduction par les particules lentivirales est réalisée en présence de 8µg/mL de Polybrene®. Un inhibiteur des mécanismes de défense cellulaire, le BX795 (Invivogen), est utilisé à une concentration de 6 µM dans le cas des particules MS2/PP7(NC)-RLP 12X 2X, MS2RLP-mCherry 12X et PP7RLP-ZsGreenl 2X. Les cellules cibles sont récupérées à 48h post-transduction et le pourcentage de cellules exprimant la ZsGreenl et la mCherry est quantifié par cytométrie (Macs Quant VYB, Miltenyi Biotec).

### II. Résultats

La Figure XXXXIV illustre l'efficacité des particules MS2/PP7(NC)-RLP 12X 2X pour le transfert d'ARN encapsidés par les séquences d'encapsidation provenant des bactériophages MS2 et PP7 dans des cellules cibles HCT116. La Figure montre que la proportion de cellules bifluorescentes est de 97% après transduction des particules MS2/PP7(NC)-RLP 12X 2X, à une dose de 10pg p24/cellule, et de 98% après transduction des particules MS2RLP-mCherry 12X et PP7RLP-ZsGreenl 2X, à 20pg p24/cellule. On observe donc le même ordre d'efficacité de transduction avec les particules MS2/PP7(NC)-RLP 12X 2X, pour une dose deux fois moins importante de MS2/PP7(NC)-RLP 12X 2X.

Les cellules cibles transduites par les particules MS2RLP-mCherry 12X seules ou PP7RLP-ZsGreenl 2X seules présentent un pourcentage d'efficacité de transduction similaire au pourcentage obtenu après transduction des cellules cibles par les particules MS2/PP7(NC)-RLP 12X 2X pour une seule protéine fluorescente. Les résultats montrent donc que les particules RLP sont capables de transporter et de transférer au moins 2 types d'ARN encapsidés par deux systèmes différents (PP7 et MS2) en une seule transduction des cellules cibles.

La mise en évidence de la capacité de transfert de différents types d'ARN dirigés par deux séquences d'encapsidation différentes, MS2 et PP7, en une seule transduction d'un même lot de RLP représente un gain significatif sur la modulation des types d'ARN encapsidés en particulier pour le transfert efficace du système CRISPR/Cas9. En remplaçant les ARN codant pour les rapporteurs fluorescents par des ARN codant pour la nucléase Cas9 et pour un ARN guide non codant, cette modulation pourrait permettre de diversifier les applications d'édition du génome avec le système CRISPR/Cas9.

## Revendications

1. Particule rétrovirale comportant une protéine issue de la polyprotéine Gag, une protéine d'enveloppe, optionnellement une intégrase et au moins deux ARN non viraux encapsidés, les ARN non viraux encapsidés comportant chacun une séquence d'ARN d'intérêt liée à une séquence d'encapsidation, chaque séquence d'encapsidation étant reconnue par un domaine de liaison hétérologue introduit dans la protéine issue de la polyprotéine Gag et/ou dans l'intégrase, dans laquelle les au moins deux ARN non viraux encapsidés se distinguent par leur séquence d'intérêt :
- l'une au moins desdites séquences d'intérêt des ARN non viraux encapsidés comportant une partie codant pour une nucléase choisie parmi le groupe constitué par les nucléases associées au système CRISPR, et
- la séquence d'intérêt de l'autre ARN non viral encapsidé correspondant à au moins un élément de reconnaissance d'un guide d'ARN.

2. Particule selon la revendication 1, dans laquelle la nucléase est Cas9.

3. Particule rétrovirale selon la revendication 1 ou 2, qui comporte en outre au moins un troisième ARN non viral encapsidé ayant une séquence d'intérêt correspondant à un second élément de reconnaissance d'un guide d'ARN ou à un guide d'ARN supplémentaire.

4. Particule rétrovirale selon l'une quelconque des revendications 1 à 3, comportant une protéine de nucléocapside issue de la polyprotéine Gag, une protéine d'enveloppe, optionnellement une intégrase et au moins deux ARN non viraux encapsidés, les ARN non viraux encapsidés comportant chacun une séquence d'ARN d'intérêt liée à une séquence d'encapsidation, chaque séquence d'encapsidation étant reconnue par un domaine de liaison hétérologue introduit dans la protéine de nucléocapside et/ou dans l'intégrase.

5. Particule rétrovirale selon la revendication 4, dans laquelle le domaine de liaison hétérologue est introduit dans la protéine de nucléocapside, un second domaine de liaison hétérologue pouvant être introduit dans la nucléocapside et/ou dans l'intégrase.

6. Particule rétrovirale selon l'une quelconque des revendications 1 à 5, dans laquelle au moins une séquence d'encapsidation est le motif tige boucle du bactériophage MS2 répété 12 fois, ce motif étant reconnu par la protéine Coat du bactériophage MS2 introduite dans la protéine de nucléocapside.

7. Particule rétrovirale selon la revendication 6, dans laquelle l'ARN non viral encapsidé comportant comme séquence d'encapsidation le motif tige boucle du bactériophage MS2 répété 12 fois est un ARN codant pour Cas 9, et un deuxième ARN non viral encapsidé est un ARN correspondant à au moins un élément de reconnaissance d'un guide d'ARN ou codant pour un guide, ledit ARN non viral encapsidé comportant comme séquence d'encapsidation le motif tige boucle du bactériophage MS2 répété 2 fois.

8. Particule rétrovirale selon l'une quelconque des revendications 1 à 7, dans laquelle au moins une séquence d'encapsidation est le motif tige boucle du bactériophage PP7 répété 2 fois, ce motif étant reconnu par la protéine Coat du bactériophage PP7 introduite dans la protéine de nucléocapside.

9. Particule rétrovirale selon l'une quelconque des revendications 1 à 4, dans laquelle le domaine de liaison hétérologue est introduit dans l'intégrase, un second domaine de liaison hétérologue pouvant être introduit dans la nucléocapside et/ou dans l'intégrase.

10. Particule rétrovirale selon l'une quelconque des revendications 1 à 9, laquelle est une particule lentivirale.

11. Composition comportant des particules selon l'une quelconque des revendications 1 à 10.

12. Kit de production de particules selon l'une quelconque des revendications 1 à 10, comportant :
(i) un plasmide d'expression comportant au moins deux séquences d'ARN non virales différentes, chaque séquence d'ARN comportant une séquence d'intérêt pour laquelle en amont, en aval ou au sein de cette séquence est insérée une séquence d'encapsidation, ou alternativement, un premier et un second plasmide d'expression comportant chacun une séquence d'intérêt en amont ou en aval de laquelle est insérée une séquence d'encapsidation, au moins une des séquences d'intérêt codant pour une nucléase choisie parmi le groupe constitué par les nucléases associées au système CRISPR, l'autre séquence d'intérêt correspondant à au moins un élément de reconnaissance d'un guide d'ARN,
(ii) un plasmide d'encapsidation codant pour une protéine issue de la polyprotéine Gag et/ou une intégrase, chimérique, comportant un domaine de liaison hétérologue permettant de reconnaître une séquence d'encapsidation, et,
(iii) un plasmide d'enveloppe codant pour une protéine d'enveloppe.

13. Kit de production selon la revendication 12, comportant en outre un second plasmide d'encapsidation codant pour :
- une protéine issue de la polyprotéine Gag sauvage, lorsque le premier plasmide d'encapsidation code pour une protéine issue de la polyprotéine Gag chimérique, et/ou
- une intégrase sauvage, lorsque le premier plasmide d'encapsidation code pour une intégrase chimérique.

14. Procédé de fabrication des particules selon l'une quelconque des revendications 1 à 10, comportant une étape de co-transfection de cellules avec :
(i) un plasmide d'expression comportant au moins deux séquences d'ARN non virales différentes, chaque séquence d'ARN comportant une séquence d'intérêt pour laquelle en amont, en aval ou au sein de cette séquence est insérée une séquence d'encapsidation, ou alternativement, un premier et un second plasmide d'expression comportant chacun une séquence d'intérêt en amont ou en aval de laquelle est insérée une séquence d'encapsidation, au moins une des séquences d'intérêt codant pour une nucléase choisie parmi le groupe constitué par les nucléases associées au système CRISPR, l'autre séquence d'intérêt correspondant à au moins un élément de reconnaissance d'un guide d'ARN,
(ii) un plasmide d'encapsidation codant pour une protéine issue de la polyprotéine Gag et/ou une intégrase chimérique comportant un domaine de liaison hétérologue permettant de reconnaître une séquence d'encapsidation, et,
(iii) un plasmide d'enveloppe codant pour une protéine d'enveloppe. et la récupération du surnageant des cellules transfectées comportant les particules.

15. Procédé de fabrication selon la revendication 14, dans lequel l'étape de co-transfection est réalisée en outre avec un second plasmide d'encapsidation codant pour :
- une protéine issue de la polyprotéine Gag sauvage, lorsque le premier plasmide d'encapsidation code pour une protéine issue de la polyprotéine Gag chimérique, et/ou
- une intégrase sauvage, lorsque le premier plasmide d'encapsidation code pour une intégrase chimérique.

16. Utilisation d'une particule selon l'une quelconque des revendications 1 à 10, ou d'une composition selon la revendication 11, pour transduire des cellules *in vitro* ou *ex vivo.*

17. Utilisation selon la revendication 16, pour générer chez les cellules transduites une cassure du double brin d'ADN, une mutation ponctuelle, une délétion de séquence dont invalidation de gène, une insertion de séquence ou remplacement de gène.

## Patentansprüche

1. Retrovirales Teilchen, umfassend ein Protein, das aus dem Gag-Polyprotein stammt, ein Hüllprotein, optional eine Integrase und mindestens zwei eingekapselte nicht virale RNA, wobei die eingekapselten nicht viralen RNA jeweils eine Sequenz der untersuchten RNA umfassen, die mit einer Einkapselungssequenz verbunden ist, wobei jede Einkapselungssequenz von einer heterologen Bindungsdomäne erkannt wird, eingeführt in das Protein, das aus dem Gag-Polyprotein stammt, und/oder in die Integrase, wobei sich die mindestens zwei eingekapselten nicht viralen RNA durch ihre untersuchte Sequenz unterscheiden:
- wobei mindestens eine der untersuchten Sequenzen der eingekapselten nicht viralen RNA einen Teil umfasst, der eine Nuklease codiert, ausgewählt aus der Gruppe, bestehend aus den Nukleasen, die mit dem CRISPR-System assoziiert sind, und
- wobei die untersuchte Sequenz der anderen eingekapselten nicht viralen RNA mindestens einem Aufklärungselement einer RNA-Führung entspricht.

2. Teilchen nach Anspruch 1, wobei die Nuklease Cas9 ist.

3. Retrovirales Teilchen nach Anspruch 1 oder 2, das außerdem mindestens eine dritte eingekapselte nicht virale RNA mit einer untersuchten Sequenz umfasst, die einem zweiten Aufklärungselement einer RNA-Führung oder einer zusätzlichen RNA-Führung entspricht.

4. Retrovirales Teilchen nach einem der Ansprüche 1 bis 3, umfassend ein Nukleocapsidprotein, das aus dem Gag-Polyprotein stammt, ein Hüllprotein, optional eine Integrase und mindestens zwei eingekapselte nicht virale RNA, wobei die eingekapselten nicht viralen RNA jeweils eine Sequenz der untersuchten RNA umfassen, die mit einer Einkapselungssequenz verbunden ist, wobei jede Einkapselungssequenz von einer heterologen Bindungsdomäne erkannt wird, eingeführt in das Nukleocapsidprotein und/oder in die Integrase.

5. Retrovirales Teilchen nach Anspruch 4, wobei die heterologe Bindedomäne in das Nukleocapsidprotein eingeführt ist, wobei eine zweite heterologe Bindedomäne in das Nukleocapsid und/oder in die Integrase eingeführt werden kann.

6. Retrovirales Teilchen nach einem der Ansprüche 1 bis 5, wobei mindestens eine Einkapselungssequenz das Stamm-Schleifen-Motiv des Bakteriophagen MS2, wiederholt 12 Mal, ist, wobei dieses Motiv vom Coat-Protein des Bakteriophagen MS2, eingeführt in das Nukleocapsidprotein, erkannt wird.

7. Retrovirales Teilchen nach Anspruch 6, wobei die eingekapselte nicht virale RNA, die als Einkapselungssequenz das Stamm-Schleifen-Motiv des Bakteriophagen MS2, wiederholt 12 Mal, umfasst, eine RNA ist, die Cas 9 codiert, und eine zweite eingekapselte nicht virale RNA eine RNA ist, die mindestens einem Aufklärungselement einer RNA-Führung entspricht oder eine Führung codiert, wobei die eingekapselte nicht virale RNA als Einkapselungssequenz das Stamm-Schleifen-Motiv des Bakteriophagen MS2, wiederholt 2 Mal, umfasst.

8. Retrovirales Teilchen nach einem der Ansprüche 1 bis 7, wobei mindestens eine Einkapselungssequenz das Stamm-Schleifen-Motiv des Bakteriophagen PP7, wiederholt 2 Mal, ist, wobei dieses Motiv vom Coat-Protein des Bakteriophagen PP7, eingeführt in das Nukleocapsidprotein, erkannt wird.

9. Retrovirales Teilchen nach einem der Ansprüche 1 bis 4, wobei die heterologe Bindedomäne in die Integrase eingeführt ist, wobei eine zweite heterologe Bindedomäne in das Nukleocapsid und/oder in die Integrase eingeführt werden kann.

10. Retrovirales Teilchen nach einem der Ansprüche 1 bis 9, welches ein lentivirales Teilchen ist.

11. Zusammensetzung, umfassend Teilchen nach einem der Ansprüche 1 bis 10.

12. Kit zur Herstellung von Teilchen nach einem der Ansprüche 1 bis 10, umfassend:
(i) ein Expressionsplasmid, umfassend mindestens zwei verschiedene nicht-virale RNA-Sequenzen, wobei jede RNA-Sequenz eine untersuchte Sequenz umfasst, für die vorgelagert, nachgelagert oder innerhalb dieser Sequenz eine Einkapselungssequenz eingeführt ist, oder, alternativ, ein erstes und ein zweites Expressionsplasmid, umfassend jeweils eine untersuchte Sequenz, vorgelagert oder nachgelagert von der eine Einkapselungssequenz eingeführt ist, wobei mindestens eine der untersuchten Sequenzen eine Nuklease codiert, ausgewählt aus der Gruppe, bestehend aus den Nukleasen, die mit dem CRISPR-System assoziiert sind, wobei die andere untersuchte Sequenz mindestens einem Aufklärungselement einer RNA-Führung entspricht,
(ii) ein Einkapselungsplasmid, das ein Protein codiert, das aus dem Gag-Polyprotein stammt, und/oder eine schimärische Integrase, umfassend eine heterologe Bindedomäne, die ermöglicht, eine Einkapselungssequenz zu erkennen, und
(iii) ein Hüllplasmid, das ein Hüllprotein codiert.

13. Kit zur Herstellung nach Anspruch 12, umfassend außerdem ein zweites Einkapselungsplasmid, das codiert:
- ein Protein, das aus dem Wildtyp-Gag-Polyprotein stammt, wenn das erste Einkapselungsplasmid ein Protein codiert, das aus dem schimärischen Gag-Polyprotein stammt, und/oder
- eine Wildtyp-Integrase, wenn das erste Einkapselungsplasmid eine schimärische Integrase codiert.

14. Verfahren zur Herstellung der Teilchen nach einem der Ansprüche 1 bis 10, umfassend einen Schritt des Cotransfizierens von Zellen mit:
(i) einem Expressionsplasmid, umfassend mindestens zwei verschiedene nicht-virale RNA-Sequenzen, wobei jede RNA-Sequenz eine untersuchte Sequenz umfasst, für die vorgelagert, nachgelagert oder innerhalb dieser Sequenz eine Einkapselungssequenz eingeführt ist, oder, alternativ, ein erstes und ein zweites Expressionsplasmid, umfassend jeweils eine untersuchte Sequenz, vorgelagert oder nachgelagert von der eine Einkapselungssequenz eingeführt ist, wobei mindestens eine der untersuchten Sequenzen eine Nuklease codiert, ausgewählt aus der Gruppe, bestehend aus den Nukleasen, die mit dem CRISPR-System assoziiert sind, wobei die andere untersuchte Sequenz mindestens einem Aufklärungselement einer RNA-Führung entspricht,
(ii) einem Einkapselungsplasmid, das ein Protein codiert, das aus dem Gag-Polyprotein stammt, und/oder eine schimärische Integrase, umfassend eine heterologe Bindedomäne, die ermöglicht, eine Einkapselungssequenz zu erkennen, und
(iii) einem Hüllplasmid, das ein Hüllprotein codiert und Wiedergewinnung des Überstands der transfizierten Zellen, die die Teilchen umfassen.

15. Verfahren zur Herstellung nach Anspruch 14, wobei der Schritt des Cotransfizierens außerdem mit einem zweiten Einkapselungsplasmid durchgeführt wird, das codiert:
- ein Protein, das aus dem Wildtyp-Gag-Polyprotein stammt, wenn das erste Einkapselungsplasmid ein Protein codiert, das aus dem schimärischen Gag-Polyprotein stammt, und/oder
- eine Wildtyp-Integrase, wenn das erste Einkapselungsplasmid eine schimärische Integrase codiert.

16. Verwendung eines Teilchens nach einem der Ansprüche 1 bis 10 oder einer Zusammensetzung nach Anspruch 11, um Zellen in vitro oder ex vivo zu transduzieren.

17. Verwendung nach Anspruch 16, um bei den transduzierten Zellen einen Bruch des doppelten RNA-Strangs, eine punktuelle Mutation, eine Deletion der Sequenz, somit Invalidierung des Gens, eine Einführung der Sequenz oder einen Ersatz des Gens zu erzeugen.

## Claims

1. Retroviral particle comprising a protein derived from the Gag polyprotein, an envelope protein, optionally an integrase and at least two encapsidated non-viral RNAs, the encapsidated non-viral RNAs each comprising an RNA sequence of interest bound to an encapsidation sequence, each encapsidation sequence being recognized by a heterologous binding domain introduced into the protein derived from the Gag polyprotein and/or into the integrase, in which the at least two encapsidated non-viral RNAs are distinguished by their sequence of interest:
- at least one of said sequences of interest of the encapsidated non-viral RNAs comprising a part coding a nuclease selected from the group constituted by the nucleases associated with the CRISPR system, and
- the sequence of interest of the other encapsidated non-viral RNA corresponding to at least one recognition element of a guide RNA.

2. Particle according to claim 1, in which the nuclease is Cas9.

3. Retroviral particle according to claim 1 or 2, which further comprises at least one third encapsidated non-viral RNA having a sequence of interest corresponding to a second recognition element of a guide RNA or to an additional guide RNA.

4. Retroviral particle according to any one of claims 1 to 3, comprising a nucleocapsid protein derived from the Gag polyprotein, an envelope protein, optionally an integrase and at least two encapsidated non-viral RNAs, the encapsidated non-viral RNAs each comprising an RNA sequence of interest bound to an encapsidation sequence, each encapsidation sequence being recognized by a heterologous binding domain introduced into the nucleocapsid protein and/or into the integrase.

5. Retroviral particle according to claim 4, in which the heterologous binding domain is introduced into the nucleocapsid protein, and a second heterologous binding domain may be introduced into the nucleocapsid and/or into the integrase.

6. Retroviral particle according to any one of claims 1 to 5, in which at least one encapsidation sequence is the stem-loop motif of the MS2 bacteriophage repeated 12 times, this motif being recognized by the Coat protein of the MS2 bacteriophage introduced into the nucleocapsid protein.

7. Retroviral particle according to claim 6, in which the encapsidated non-viral RNA comprising as encapsidation sequence the stem-loop motif of the MS2 bacteriophage repeated 12 times is an RNA coding Cas9, and a second encapsidated non-viral RNA is an RNA corresponding to at least one recognition element of a guide RNA or coding a guide, said encapsidated non-viral RNA comprising as encapsidation sequence the stem-loop motif of the MS2 bacteriophage repeated 2 times.

8. Retroviral particle according to any one of claims 1 to 7, in which at least one encapsidation sequence is the stem-loop motif of the PP7 bacteriophage repeated 2 times, this motif being recognized by the Coat protein of the PP7 bacteriophage introduced into the nucleocapsid protein.

9. Retroviral particle according to any one of claims 1 to 4, in which the heterologous binding domain is introduced into the integrase, and a second heterologous binding domain may be introduced into the nucleocapsid and/or into the integrase.

10. Retroviral particle according to any one of claims 1 to 9, which is a lentiviral particle.

11. Composition comprising particles according to any one of claims 1 to 10.

12. Kit for producing particles according to any one of claims 1 to 10, comprising:
(i) an expression plasmid comprising at least two different non-viral RNA sequences, each RNA sequence comprising a sequence of interest for which an encapsidation sequence is inserted upstream of, downstream of or within this sequence, or alternatively, a first and a second expression plasmid each comprising a sequence of interest for which an encapsidation sequence is inserted upstream or downstream of this sequence, at least one of the sequences of interest coding a nuclease selected from the group constituted by the nucleases associated with the CRISPR system, the other sequence of interest corresponding to at least one recognition element of a guide RNA,
(ii) an encapsidation plasmid coding a protein derived from the Gag polyprotein and/or a chimeric integrase, comprising a heterologous binding domain allowing recognition of an encapsidation sequence, and,
(iii) an envelope plasmid coding an envelope protein.

13. Production kit according to claim 12, further comprising a second encapsidation plasmid coding:
- a protein derived from the wild-type Gag polyprotein, when the first encapsidation plasmid codes a protein derived from the chimeric Gag polyprotein, and/or
- a wild-type integrase, when the first encapsidation plasmid codes a chimeric integrase.

14. Manufacturing method for the particles according to any one of claims 1 to 10, comprising a step of co-transfection of cells with:
(i) an expression plasmid comprising at least two different non-viral RNA sequences, each RNA sequence comprising a sequence of interest for which an encapsidation sequence is inserted upstream of, downstream of or within this sequence, or alternatively, a first and a second expression plasmid each comprising a sequence of interest for which an encapsidation sequence is inserted upstream or downstream of this sequence, at least one of the sequences of interest coding a nuclease selected from the group constituted by the nucleases associated with the CRISPR system, the other sequence of interest corresponding to at least one recognition element of a guide RNA,
(ii) an encapsidation plasmid coding a protein derived from the Gag polyprotein and/or a chimeric integrase comprising a heterologous binding domain allowing recognition of an encapsidation sequence, and,
(iii) an envelope plasmid coding an envelope protein;
and recovery of the supernatant from the transfected cells comprising the particles.

15. Manufacturing method according to claim 14, in which the step of co-transfection is moreover carried out with a second encapsidation plasmid coding:
- a protein derived from the wild-type Gag polyprotein, when the first encapsidation plasmid codes a protein derived from the chimeric Gag polyprotein, and/or
- a wild-type integrase, when the first encapsidation plasmid codes a chimeric integrase.

16. Use of a particle according to any one of claims 1 to 10, or of a composition according to claim 11, for the transduction of cells *in vitro* or ex *vivo.*

17. Use according to claim 16, for generating, in the transduced cells, a DNA double-strand break, a point mutation, a sequence deletion including gene knock-out, a sequence insertion or gene replacement.
